# EUROPEAN PATENT APPLICATION

(11) **EP 3 542 789 A2**
(43) Date of publication of application: **25.09.2019**
(21) Application number: 19165057.1
(22) Date of filing: 31.08.2011
(51) Int. Cl.: A61K 9/127, A61K 39/12, A61K 39/155, A61K 48/00, A61K 38/00, A61K 9/00

(54) **LIPIDS SUITABLE FOR LIPOSOMAL DELIVERY OF PROTEIN-CODING RNA**

(30) Priority: 31.08.2010 US 37883310 P
(62) Divisional of application: 11763813.0
(71) Applicant: GlaxoSmithKline Biologicals SA, 1330 Rixensart (BE)
(72) Inventor: GEALL, Andrew, Emeryville, CA California 94662-8097 (US)
(74) Representative: Sanderson, Andrew John

(57) **Abstract**

RNA is encapsulated within a liposome for *in vivo* delivery. The RNA encodes a polypeptide of interest, such as an immunogen for immunisation purposes. The liposome includes at least one compound selected from the group consisting of compounds of formula (I) and formula (XI).

## Description

This application claims the benefit of U.S. provisional application number 61/378,833, which was filed August 31, 2010), the complete contents of which are hereby incorporated herein by reference for all purposes.

### TECHNICAL FIELD

This invention is in the field of non-viral delivery of RNA to animals.

### BACKGROUND ART

The delivery of nucleic acids for *in vivo* expression of encoded proteins is useful for both gene therapy and immunisation. Various approaches to successful delivery have been tested, including the use of DNA or RNA, of viral or non-viral delivery vehicles (or even no delivery vehicle, in a "naked" vaccine), of replicating or non-replicating vectors, or of viral or non-viral vectors.

There remains a need for further and improved ways of delivering nucleic acids to animals for *in vivo* expression of their encoded proteins.

### DISCLOSURE OF THE INVENTION

According to the invention, RNA is delivered encapsulated within a liposome. The RNA encodes a polypeptide of interest. The liposome includes at least one compound selected from the group consisting of compounds of formula (I) and formula (XI). These liposomes can efficiently deliver RNA for *in vivo* expression. The invention is particularly useful for immunisation, in which the encoded polypeptide is an immunogen.

Thus the invention provides a liposome within which RNA encoding a polypeptide of interest is encapsulated, wherein the liposome includes at least one compound selected from the group consisting of compounds of formula (I) and formula (XI).

Formula (I) is: 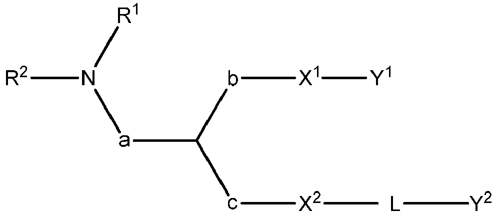 wherein:
R¹ and R² together with the nitrogen atom to which they are attached form an optionally substituted C₃₋₂₀-heterocycloalkyl, C₃₋₂₀-heterocycloalkenyl, C₃₋₂₀-heterocycloalkynyl or C₅₋₂₀-heteroaryl group;
a is absent or optionally substituted C₁₋₄ alkylene;
b is absent or optionally substituted C₁₋₄ alkylene;
c is absent or optionally substituted C₁₋₄ alkylene;
X¹ is O or S;
X² is O or S;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is absent or is -(L^{a})_{d}-(L^{b})ₑ-(L^{c})_{f}-, wherein
   L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   L^{b} is optionally substituted C₆₋₁₄arylene or C₅₋₁₃heteroarylene;
   L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   d is 0 or 1;
   e is 0 or 1; and
   f is 0 or 1; and
Y² is an optionally substituted steroid.

Formula (XI) is:

R^{a}-(AA)_{z}-R^{b}

wherein
R^{a} is a N-terminal alkylamide;
z is an integer from 2 to 10;
each AA is an amino acid, provided that at least one histidine and at least one cationic amino acid are present;
R^{b} is -H or -NH₂.

The invention also provides a process for preparing a RNA-containing liposome, comprising a step of mixing RNA with a compound selected from the group consisting of compounds of formula (I) and formula (XI), under conditions such that the compounds form a liposome in which the RNA is encapsulated. The RNA and the compound may be mixed in the presence of other compounds which also become incorporated into the liposome *e.g.* further lipids.

### The liposome

The invention utilises liposomes within which polypeptide-encoding RNA is encapsulated. Thus the RNA is (as in a natural virus) separated from any external medium. Encapsulation within the liposome has been found to protect RNA from RNase digestion. The liposomes can include some external RNA (*e.g.* on their surface), but at least half of the RNA (and ideally all of it) is encapsulated in the liposome's core. Encapsulation within liposomes is distinct from, for instance, the lipid/RNA complexes disclosed in reference 1, where RNA is mixed with pre-formed liposomes.

Various amphiphilic lipids can form bilayers in an aqueous environment to encapsulate a RNA-containing aqueous core as a liposome. These lipids can have an anionic, cationic or zwitterionic hydrophilic head group. Formation of liposomes from anionic phospholipids dates back to the 1960s, and cationic liposome-forming lipids have been studied since the 1990s. Some phospholipids are anionic whereas other are zwitterionic and others are cationic. Suitable classes of phospholipid include, but are not limited to, phosphatidylethanolamines, phosphatidylcholines, phosphatidylserines, and phosphatidyl-glycerols, and some useful phospholipids are listed in Table 1. Useful cationic lipids in the prior art include, but are not limited to, dioleoyl trimethylammonium propane (DOTAP), 1,2-distearyloxy-N,N-dimethyl-3-aminopropane (DSDMA), 1,2-dioleyloxy-N,Ndimethyl-3-aminopropane (DODMA), 1,2-dilinoleyloxy-N,N-dimethyl-3-aminopropane (DlinDMA), 1,2-dilinolenyloxy-N,N-dimethyl-3-aminopropane (DLenDMA). Zwitterionic lipids include, but are not limited to, acyl zwitterionic lipids and ether zwitterionic lipids. Examples of useful zwitterionic lipids are DPPC, DOPC, DSPC, dodecylphosphocholine, 1,2-dioleoyl-sn-glycero-3-phosphatidylethanolamine (DOPE), and 1,2-diphytanoyl-sn-glycero-3-phosphoethanolamine (DPyPE). The lipids in the liposomes of the invention can be saturated or unsaturated. The use of at least one unsaturated lipid for preparing liposomes is preferred. If an unsaturated lipid has two tails, both tails can be unsaturated, or it can have one saturated tail and one unsaturated tail. A lipid can include a steroid group in one tail *e.g.* as in RV05.

Liposomes of the invention can be formed from a single lipid or, more usually, from a mixture of lipids. A mixture may comprise (i) a mixture of cationic lipids (ii) a mixture of anionic lipids and cationic lipids (iii) a mixture of zwitterionic lipids and cationic lipids or (vii) a mixture of anionic lipids, cationic lipids and zwitterionic lipids. Similarly, a mixture may comprise both saturated and unsaturated lipids. Where a mixture of lipids is used, not all of the component lipids in the mixture need to be amphiphilic *e.g.* one or more amphiphilic lipids can be mixed with cholesterol.

Liposomes of the invention comprise at least one compound of formula (I) and/or at least one compound of formula (XI). Preferred liposomes of the invention include a cationic lipid of formula (I). As shown in the examples, such liposomes are particularly useful for *in vivo* delivery of RNA for protein expression. Other preferred liposomes of the invention include a lipopeptide of formula (XI). A liposome can include both a liposome of formula (I) and a lipopeptide of formula (XI), but usually includes only one of these two classes of cationic compound.

Where a liposome of the invention is formed from a mixture of lipids, it is preferred that the proportion of those lipids which have formula (I) or (XI) should be between 20-80% of the total amount of lipids e.g. between 30-70%, or between 40-60%. For instance, useful liposomes are shown below in which 40% or 60% of the total lipid is a lipid of formula (I). The remainder can be made of *e.g.* cholesterol (*e.g.* 35-50% cholesterol) and/or DMG (optionally PEGylated) and/or DSPC. Such mixtures are used below. These percentage values are mole percentages.

A liposome may include an amphiphilic lipid whose hydrophilic portion is PEGylated (*i.e.* modified by covalent attachment of a polyethylene glycol). This modification can increase stability and prevent non-specific adsorption of the liposomes. For instance, lipids can be conjugated to PEG using techniques such as those disclosed in reference 2 and 3. Useful PEGylated lipids include PEG-DMG and the lipids of reference 8's formula (XI). PEG provides the liposomes with a coat which can confer favourable pharmacokinetic characteristics. Various lengths of PEG can be used *e.g.* between 0.5-8kDa.

Useful mixtures of lipids, for forming liposomes of the invention, comprise: a cationic lipid of formula (I); cholesterol; and a PEGylated lipid, such as PEG-DMG *i.e.* PEG-conjugated 1,2-dimyristoyl-sn-glycero-3-phosphoethanolamine-N-[methoxy(polyethylene glycol). This mixture may also include a neutral zwitterionic lipid, such as DSPC (1,2-Diastearoyl-sn-glycero-3-phosphocholine) or DPyPE. These (and other) mixtures are used in the examples below.

Liposomes are usually divided into three groups: multilamellar vesicles (MLV); small unilamellar vesicles (SUV); and large unilamellar vesicles (LUV). MLVs have multiple bilayers in each vesicle, forming several separate aqueous compartments. SUVs and LUVs have a single bilayer encapsulating an aqueous core; SUVs typically have a diameter ≤50nm, and LUVs have a diameter >50nm. Liposomes of the invention are ideally LUVs with a diameter in the range of 60-180nm, and preferably in the range of 80-160nm.

A liposome of the invention can be part of a composition comprising a plurality of liposomes, and the liposomes within the plurality can have a range of diameters. For a composition comprising a population of liposomes with different diameters: (i) at least 80% by number of the liposomes should have diameters in the range of 60-180nm, and preferably in the range of 80-160nm, and/or (ii) the average diameter (by intensity *e.g.* Z-average) of the population is ideally in the range of 60-180nm, and preferably in the range of 80-160nm. The diameters within the plurality should ideally have a polydispersity index <0.2. The liposome/RNA complexes of reference 1 are expected to have a diameter in the range of 600-800nm and to have a high polydispersity. Diameters in a population can be measured using dynamic light scattering.

Techniques for preparing suitable liposomes are well known in the art *e.g.* see references 4 to 6. One useful method is described in reference 7 and involves mixing (i) an ethanolic solution of the lipids (ii) an aqueous solution of the nucleic acid and (iii) buffer, followed by mixing, equilibration, dilution and purification. Preferred liposomes of the invention are obtainable by this mixing process.

To obtain liposomes with the desired diameter(s), mixing can be performed using a process in which two feed streams of aqueous RNA solution are combined in a single mixing zone with one stream of an ethanolic lipid solution, all at the same flow rate *e.g.* in a microfluidic channel as described below.

### Formula (I)

Cationic lipids of formula (I) are as follows: 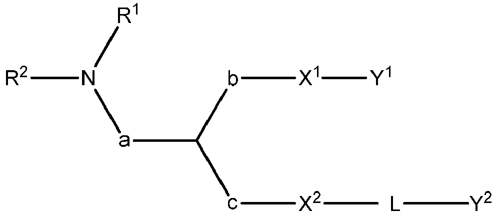 wherein:
R¹ and R² together with the nitrogen atom to which they are attached form an optionally substituted C₃₋₂₀-heterocycloalkyl, C₃₋₂₀-heterocycloalkenyl, C₃₋₂₀-heterocycloalkynyl or C₅₋₂₀-heteroaryl group;
a is absent or optionally substituted C₁₋₄ alkylene;
b is absent or optionally substituted C₁₋₄ alkylene;
c is absent or optionally substituted C₁₋₄ alkylene;
X¹ is O or S;
X² is O or S;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is absent or is -(L^{a})_{d}-(L^{b})ₑ-(L^{c})_{f}-, wherein
   L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   L^{b} is optionally substituted C₆₋₁₄arylene or C₅₋₁₃heteroarylene;
   L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   d is 0 or 1;
   e is 0 or 1; and
   f is 0 or 1; and
Y² is an optionally substituted steroid.

Thus R¹ and R², together with the nitrogen atom to which they are attached, form a cyclic "headgroup" with a tertiary amine. These compounds are described in more detail in reference 8, the complete contents of which are incorporated herein by reference.

In some embodiments the compounds of formula (I) have formula (II): 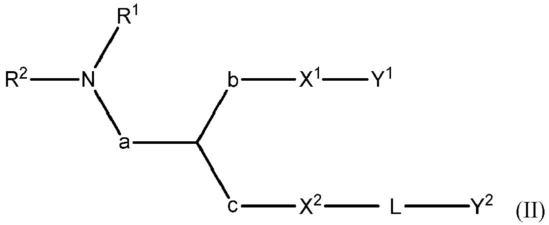 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is absent or optionally substituted C₁₋₄ alkylene;
b is absent or optionally substituted C₁₋₄ alkylene;
c is absent or optionally substituted C₁₋₄ alkylene;
X¹ is O or S;
X² is O or S;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is -(L^{a})_{d}-(L^{b})ₑ-(L^{c})_{f}-, wherein
   L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   L^{b} is optionally substituted C₆₋₁₄arylene or C₅₋₁₃heteroarylene;
   L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   d is 0 or 1;
   e is 0 or 1; and
   f is 0 or 1;
provided that L comprises one or more heteroatoms, and
Y² is an optionally substituted steroid.

In some embodiments the compounds of formula (I) have formula (III): 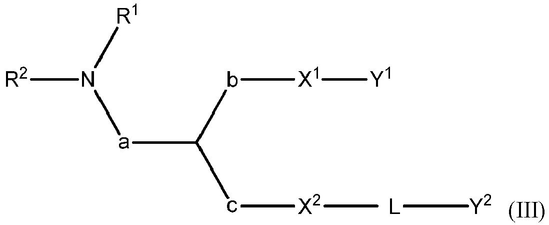 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is methylene;
b is methylene;
c is absent;
X¹ is O or S;
X² is O or S;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is -(L^{a})_{d}-(L^{b})ₑ-(L^{c})_{f}-, wherein
   L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   L^{b} is optionally substituted C₆₋₁₄arylene or C₅₋₁₃heteroarylene;
   L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   d is 0 or 1;
   e is 0 or 1; and
   f is 0 or 1; and
Y² is an optionally substituted steroid.

In some embodiments the compounds of formula (I) have formula (IV): 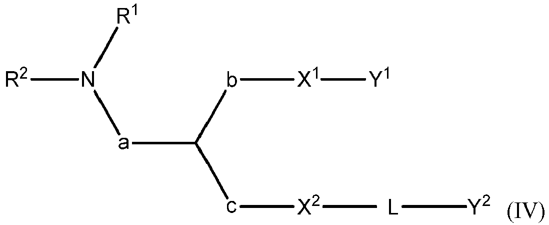 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is methylene;
b is methylene;
c is absent;
X¹ is O or S;
X² is O or S;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is -(L^{a})_{d}-(L^{b})ₑ-(L^{c})_{f}-, wherein
   L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   L^{b} is optionally substituted C₆₋₁₄arylene or C₅₋₁₃heteroarylene;
   L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   d is 0 or 1;
   e is 0 or 1; and
   f is 0 or 1;
provided that L comprises one or more heteroatoms, and
Y² is an optionally substituted steroid.

In some embodiments the compounds of formula (I) have formula (V): 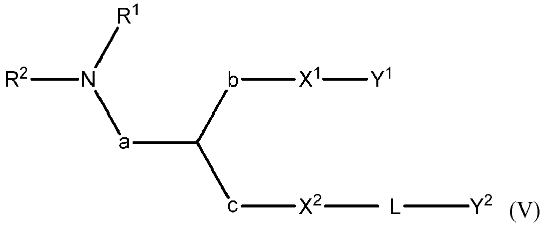 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is methylene;
b is methylene;
c is absent;
X¹ is O;
X² is O;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is -(L^{a})_{d}-(L^{b})ₑ-(L^{c})_{f}-, wherein
   L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   L^{b} is optionally substituted C₆₋₁₄arylene or C₅₋₁₃heteroarylene;
   L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   d is 0 or 1;
   e is 0 or 1; and
   f is 0 or 1;
provided that L comprises one or more heteroatoms, and
Y² is an optionally substituted steroid.

In some embodiments the compounds of formula (I) have formula (VI): 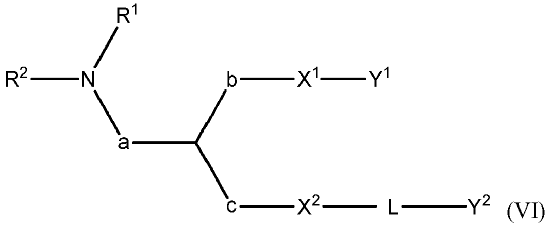 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is methylene;
b is methylene;
c is absent;
X¹ is O;
X² is O;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is -L^{c}-, wherein L^{c} is optionally substituted C₁₋₁₅heteroalkylene, C₁₋₁₅heteroalkenylene or C₁₋₁₅heteroalkynylene; and
Y² is an optionally substituted steroid.

In some embodiments the compounds of formula (I) have formula (VII): 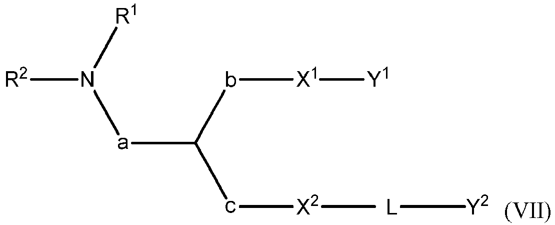 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is methylene;
b is methylene;
c is absent;
X¹ is O;
X² is O;
Y¹ is an optionally substituted C₁₆₋₂₂ alkenyl group;
L is -L^{c}-, wherein L^{c} is optionally substituted C₁₋₁₅heteroalkylene, C₁₋₁₅heteroalkenylene or C₁₋₁₅heteroalkynylene; and
Y² is an optionally substituted steroid.

In some embodiments the compounds of formula (I) have formula (VIII): 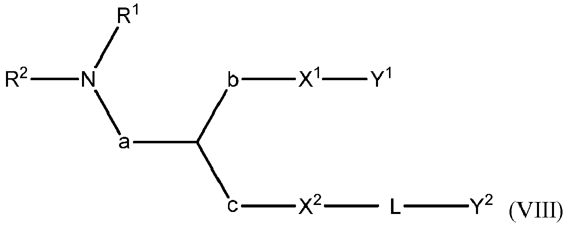 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is methylene;
b is methylene;
c is absent;
X¹ is O;
X² is O;
Y¹ is an optionally substituted C₁₆₋₂₂ alkenyl group;
L is -L^{c}-, wherein L^{c} is optionally substituted C₁₋₁₅heteroalkylene, C₁₋₁₅heteroalkenylene or C₁₋₁₅heteroalkynylene; and
Y² is cholesterol connected through the hydroxy group at the 3-position of the A steroid ring, the hydrogen atom of said hydroxy group being absent.

In some embodiments the compounds of formula (I) have formula (IX): 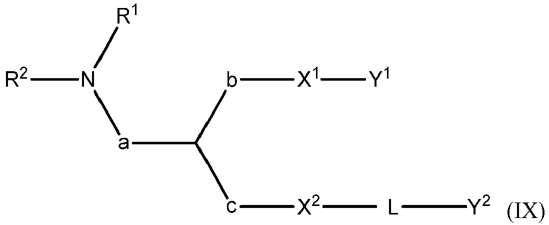 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is methylene;
b is methylene;
c is absent;
X¹ is O or S;
X² is O or S;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is -(L^{a})_{d}-(L^{b})ₑ-(L^{c})_{f}-, wherein
   L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   L^{b} is optionally substituted C₆₋₁₄arylene or C₅₋₁₃heteroarylene;
   L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   d is 0 or 1;
   e is 0 or 1; and
   f is 0 or 1;
provided that L comprises one or more heteroatoms,
Y² is an optionally substituted steroid; and
the pKa of the compound is from about 5.9 to about 7.

In some embodiments the compounds of formula (I) have formula (X): 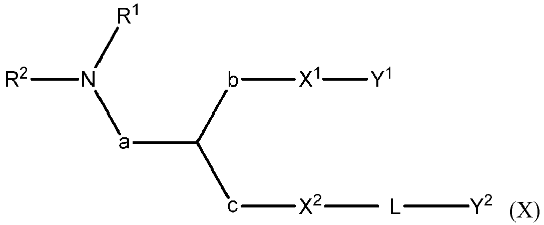 wherein:
R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl or C₅₋₂₀-heteroaryl group;
a is methylene;
b is methylene;
c is absent;
X¹ is O or S;
X² is O or S;
Y¹ is optionally substituted C₁₀₋₃₀alkenyl, C₁₀₋₃₀alkynyl, C₁₀₋₃₀heteroalkenyl or C₁₀₋₃₀heteroalkynyl;
L is -(L^{a})_{d}-(L^{b})ₑ-(L^{c})_{f}-, wherein
   L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   L^{b} is optionally substituted C₆₋₁₄arylene or C₅₋₁₃heteroarylene;
   L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
   d is 0 or 1;
   e is 0 or 1; and
   f is 0 or 1;
provided that L comprises one or more heteroatoms,
Y² is an optionally substituted steroid; and
the pKa of the compound is from about 4.5 to about 6.2.

### a, b and c

In one embodiment, a is optionally substituted C₁₋₂ alkylene. In a further embodiment, a is optionally substituted C₁ alkylene.

In one embodiment, b is optionally substituted C₀₋₂ alkylene. In a further embodiment, b is optionally substituted C₁ alkylene.

In one embodiment, c is absent or is optionally substituted C₁ alkylene. In a further embodiment, c is absent.

In one embodiment, a, b and c are, if present, unsubstituted.

### The headgroup

In one embodiment, R¹ and R² together with the nitrogen atom to which they are attached form an optionally substituted C₃-₂₀-heterocycloalkyl, C₃₋₂₀-heterocycloalkenyl, C₃₋₂₀-heterocycloalkynyl group, Cs-heteroaryl or C₆-heteroaryl group. In one embodiment, R¹ and R² together with the nitrogen atom to which they are attached form an optionally substituted C₃-₂₀-heterocycloalkyl, C₃₋₂₀-heterocycloalkenyl or C₃₋₂₀-heterocycloalkynyl group. In a further embodiment, R¹ and R² together with the nitrogen atom to which they are attached form an optionally substituted C₃₋₂₀-heterocycloalkyl group.

In one embodiment, R¹ and R² together with the nitrogen atom to which they are attached form an optionally substituted C₅₋₁₆ group. In a further embodiment, R¹ and R² together with the nitrogen atom to which they are attached form an optionally substituted C₅₋₁₂ group. In a further embodiment, R¹ and R² together with the nitrogen atom to which they are attached form an optionally substituted C₅ group, C₆ group or C₇ group. In a further embodiment, R¹ and R² together with the nitrogen atom to which they are attached form an optionally substituted C₅ group or C₆ group.

In one of the preferred embodiments of this invention, R¹ and R² together with the nitrogen atom to which they are attached forms a species which comprises at least one oxygen atom.

In one embodiment, R¹ and R² together with the nitrogen atom to which they are attached are selected from H¹ to H⁴⁸ as provided in Table 1.

**Table 1 - Moieties named H¹ to H⁴⁸**

| | Structure | | Structure | | Structure |
|---|---|---|---|---|---|
| H¹ | 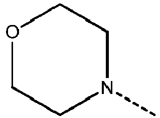 | H¹⁷ | 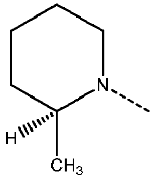 | H³³ | 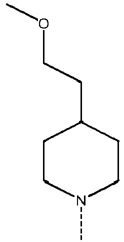 |
| H² | 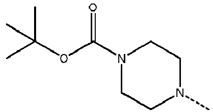 | H¹⁸ | 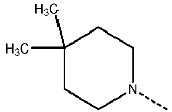 | H³⁴ | 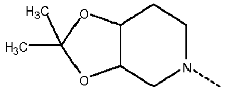 |
| H³ | 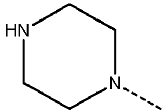 | H¹⁹ | 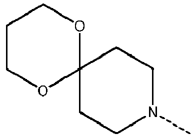 | H³⁵ | 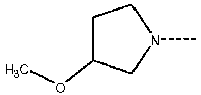 |
| H⁴ | 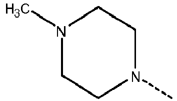 | H²⁰ | 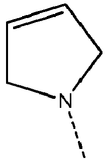 | H³⁶ | 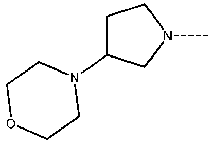 |
| H⁵ | 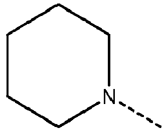 | H²¹ | 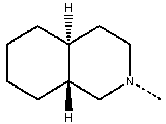 | H³⁷ | 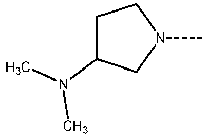 |
| H⁶ | 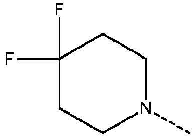 | H²² | 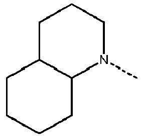 | H³⁸ | 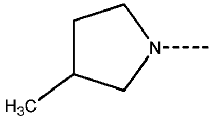 |
| H⁷ | 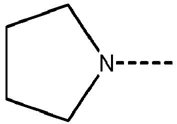 | H²³ | 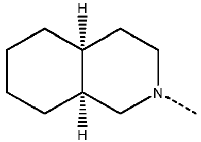 | H³⁹ | 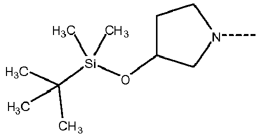 |
| H⁸ | 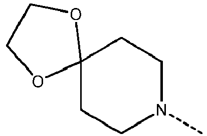 | H²⁴ | 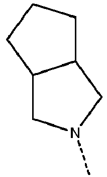 | H⁴⁰ | 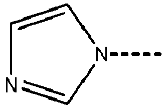 |
| H⁹ | 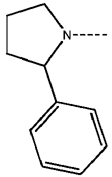 | H²⁵ | 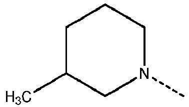 | H⁴¹ | 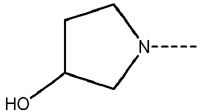 |
| H¹⁰ | 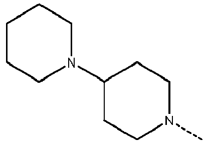 | H²⁶ | 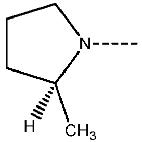 | H⁴² | 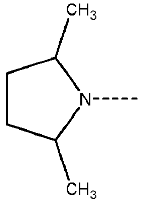 |
| H¹¹ | 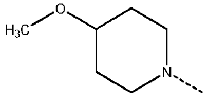 | H²⁷ | 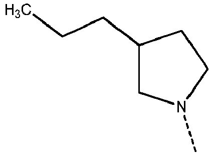 | H⁴³ | 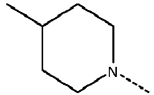 |
| H¹² | 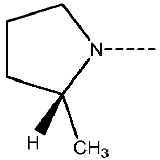 | H²⁸ | 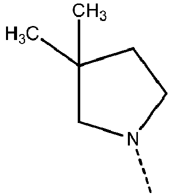 | H⁴⁴ | 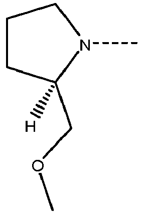 |
| H¹³ | 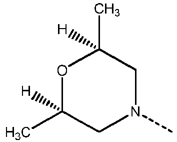 | H²⁹ | 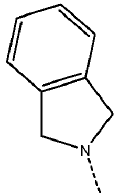 | H⁴⁵ | 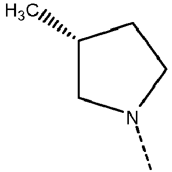 |
| H¹⁴ | 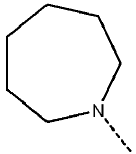 | H³⁰ | 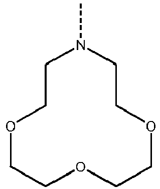 | H⁴⁶ | 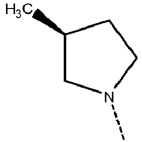 |
| H¹⁵ | 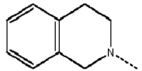 | H³¹ | 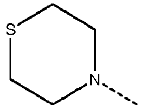 | H⁴⁷ | 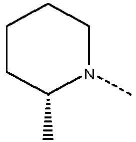 |
| H¹⁶ | 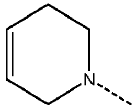 | H³² | 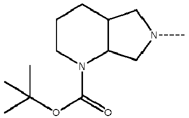 | H⁴⁸ | 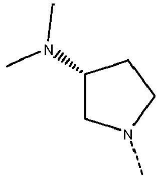 |

### X¹ and X²

In one embodiment, X¹ is O. In another embodiment, X² is O. In a further embodiment, both X¹ and X² are O.

### Linker

In a preferred embodiment, L comprises at least one heteroatom. This means that the chain which provides a direct link between X² and Y² has at least one heteroatom or, in other words, that any heteroatom in a substituent on L does not count for these purposes. In a further embodiment, L comprises at least one O atom.

In one embodiment, L comprises at least two heteroatoms. In a further embodiment, L comprises at least two O atoms.

In one embodiment, L^{c} is optionally substituted C₁₋₁₅alkylene or C₁₋₁₅heteroalkylene. In one embodiment, L^{c} is optionally substituted C₁₋₁₅alkylene or C₁₋₁₅heteroalkylene and d and e are both 0.

In one embodiment, L^{c} is selected from one of formulae L^{c-i} to L^{c-xxiii}. In one embodiment, L^{c} is selected from one of formulae L^{c-i} to L^{c-xxiii} and d and e are both 0.

| | |
|---|---|
| L^{c-i} | -(CH₂)₂O(CH₂)₂- |
| L^{c-ii} | -(CH₂)₄- |
| L^{c-iii} | -CO(CH₂)₂CO- |
| L^{c-iv} | -CO- |
| L^{c-v} | -COCH₂OCH₂CO- |
| L^{c-vi} | -(CH₂)₂O(CH₂)₂NHCO- |
| L^{c-vii} | -(CH₂)₃O(CH₂)₃- |
| L^{c-viii} | -(CH₂)₂- |
| L^{c-ix} | -(CH₂)₂O(CH₂)₂O(CH₂)₂O(CH₂)₂- |
| L^{c-x} | -(CH₂)₂O(CH₂)₂O(CH₂)₂- |
| L^{c-xi} | 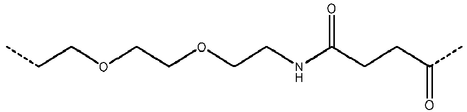 |
| L^{c-xii} | 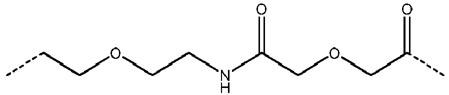 |
| L^{c-xiii} | 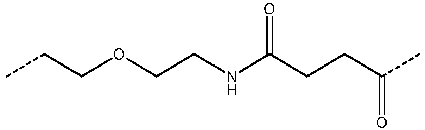 |
| L^{c-xiv} | 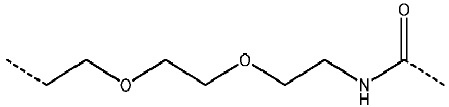 |
| L^{c-xv} | -(CH₂)₂O(CH₂)₂OCH(CH₃)- |
| L^{c-xvi} | -(CH₂)₂O(CH₂)₂OC(=O)(CH₂)₂CO- |
| L^{c-xvii} | -(CH₂)₂OC(=O)(CH₂)₂CO- |
| L^{c-xviii} | -(CH₂)₂O(CH₂)₂OCO- |
| L^{c-xix} | -(CH₂)₂NHC(=O)CH₂OCH₂C(=O)- |
| L^{c-xx} | -(CH₂)₂NHC(=O)(CH₂)₂C(=O)- |
| L^{c-xxi} | -(CH₂)₂NHC(=O)- |
| L^{c-xxii} | -(CH₂)₂NHC(=O)CH₂NHC(=O)- |
| L^{c-xxiii} | -(CH₂)₂NHC(=O)CH(side-chain-1)NHC(=O)-, wherein side-chain-1 represents the group 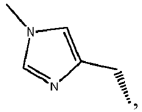 the dashed line representing the bond to the rest of the molecule. |

Since groups in which L comprises at least one heteroatom are preferred, L^{c} is preferably selected from L^{c-i}, L^{c-v} to L^{c-vii} and L^{c-ix} to L^{c-xxiii}.

In one embodiment, L^{c} is optionally substituted C₁₋₁₅heteroalkylene.

In one embodiment, L^{c} is an optionally substituted C₁₋₁₁ group. In a further embodiment, L^{c} is an optionally substituted C₁₋₉ group. In a further embodiment, L^{c} is an optionally substituted C₃₋₈ group. In a further embodiment, wherein L^{c} is an optionally substituted C₄₋₇ group. In a further embodiment, L^{c} is an optionally substituted C₅, C₆ or C₇ group.

In a preferred embodiment, d is 0; e is 0, and f is 1. In a preferred embodiment, d is 0; e is 0, and f is 1 and L^{c} is, within the chain lengths set out above, heteroalkylene.

### Y¹

In one embodiment, Y¹ is a C₁₂₋₂₈ group. In a further embodiment, Y¹ is a C₁₄₋₂₆ group. In a further embodiment, Y¹ is a C₁₆₋₂₄ group. In a further embodiment, Y¹ is a C₁₆₋₂₂ group. In a further embodiment, the Y¹ chain is 18, 19, 20 or 21 atoms long.

Within the carbon ranges set out above, Y¹ is preferably alkenyl or heteroalkenyl.

In one embodiment, Y¹ has at least one alkene group. In a further embodiment, Y¹ has 1, 2 or 3 alkene groups.

In one embodiment, Y¹ has an alkene group at the omega-3 position. In another embodiment, Y¹ has an alkene group at the omega-6 position. In another embodiment, Y¹ has an alkene group at the omega-9 position. In a further embodiment, Y¹ has an alkene group at two or three of the omega-3, omega-6 and omega-9 positions. In one embodiment, Y¹ is unsaturated at the omega-6 and omega-9 positions. In another embodiment, Y¹ is unsaturated at the omega-3, omega-6 and omega-9 positions. In one embodiment, Y¹ is unsaturated at the omega-9 position.

In one embodiment, Y¹ has at least one cis unsaturated alkene group. In a further embodiment, Y¹ has at least two cis unsaturated alkene groups. In a further embodiment, Y¹ has at least three cis unsaturated alkene groups. The at least one cis unsaturated alkene group may be at one, two or three of the omega-3, omega-6 and omega-9 positions. Unsaturation in lipid chains is discussed in MacLachlan et al., Journal of Controlled Release 107 (2005) 276-287.

In one embodiment Y¹ is selected from Y¹⁻ⁱ to Y^{1-vi} as provided in Table 2.

**Table 2. Y1 related Moieties named Y¹⁻ⁱ to Y^{1-vi}**

| Name | Structure | Name | Structure |
|---|---|---|---|
| Y¹⁻ⁱ | 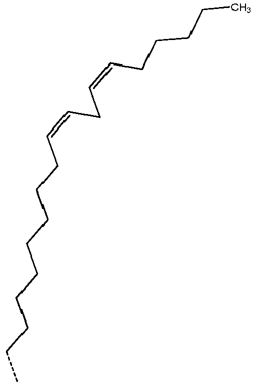 | Y¹⁻ⁱⁱ | 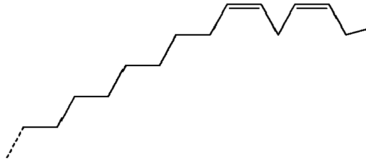 |
| Y¹⁻ⁱⁱⁱ | 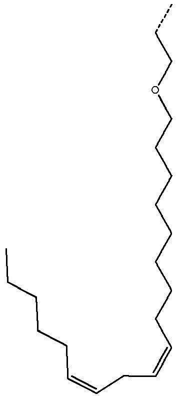 | Y^{1-iv} | 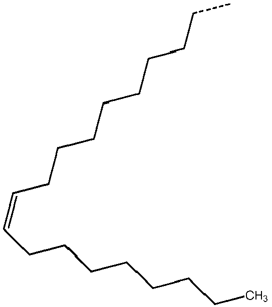 |

| Name | Structure | Name | Structure |
|---|---|---|---|
| Y^{1-v} | 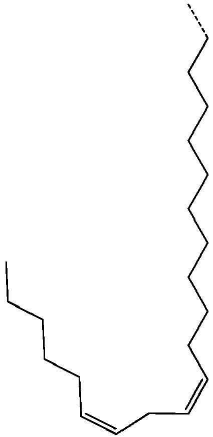 | Y^{1-vi} | 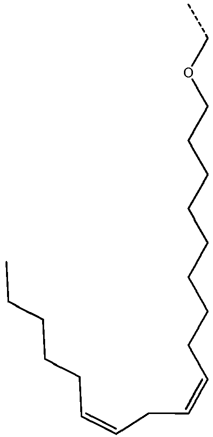 |

### Y²

In one embodiment, Y² is linked to L via an oxygen atom on the optionally substituted steroid. In a further embodiment, Y² is linked to L via an oxygen atom on the 3-position of the A steroid ring. In a further embodiment Y² is a sterol in which the hydrogen atom of the hydroxy group at the 3-position of the A steroid ring has been removed (and the connection to L is through the oxygen atom of said hydroxy group).

In one embodiment said sterol is selected from the group consisting of: annasterol; avenasterol; beta-sitosterol; brassicasterol; calciferol; campesterol; chalinosterol; chinasterol; cholestanol; cholesterol; coprostanol; cycloartenol; dehydrocholesterol; desmosterol; dihydrocalciferol; dihydrocholesterol; dihydroergosterol; dinosterol; epicholesterol; ergosterol; fucosterol; hexahydrolumisterol; hexaol; hydroxycholesterol; lanosterol; lumisterol; parkeol; poriferasterol; saringosterol; sitostanol; sitosterol; stigmastanol; stigmasterol; weinbersterol; zymosterol; sterol bile acids (such as cholic acid; chenodeoxycholic acid; glycocholic acid; taurocholic acid; deoxycholic acid, and lithocholic acid); and salts thereof.

In a further embodiment, the sterol is cholesterol.

### pKa

The pKa of a lipid is the pH at which 50% of the lipids are charged, lying halfway between the point where the lipids are completely charged and the point where the lipids are completely uncharged. It can be measured in various ways, but is preferably measured using the method disclosed below. The pKa typically should be measured for the lipid alone rather than for the lipid in the context of a mixture which also includes other lipids (e.g. not as performed in reference 2, which looks at the pKa of a SNALP rather than of the individual lipids).

The pKa of a lipid is measured in water at standard temperature and pressure using the following technique:
- 2mM solution of lipid in ethanol is prepared by weighing the lipid and dissolving in ethanol. 0.3mM solution of fluorescent probe toluene nitrosulphonic acid (TNS) in ethanol:methanol 9:1 is prepared by first making 3mM solution of TNS in methanol and then diluting to 0.3mM with ethanol.
- An aqueous buffer containing sodium phosphate, sodium citrate sodium acetate and sodium chloride, at the concentrations 20mM, 25mM, 20mM and 150 mM, respectively, is prepared. The buffer is split into eight parts and the pH adjusted either with 12N HCl or 6N NaOH to 4.44-4.52, 5.27, 6.15-6.21, 6.57, 7.10-7.20, 7.72-7.80, 8.27-8.33 and 10.47-11.12. 400uL of 2mM lipid solution and 800uL of 0.3mM TNS solution are mixed.
- 7.5µL of probe/lipid mix are added to 242.5µL of buffer in a 1mL 96 well plate. This is done with all eight buffers. After mixing, 100uL of each probe/lipid/buffer mixture is transferred to a 250uL black with clear bottom 96 well plate (*e.g.* model COSTAR 3904, Corning).
- Fluorescence of each probe/lipid/buffer mixture is measured (*e.g*. with a SpectraMax M5 spectrophotometer and SoftMax pro 5.2 software) with 322nm excitation, 431nm emission (auto cutoff at 420nm).
- After the measurement, the background fluorescence value of an empty well on the 96 well plate is subtracted from each probe/lipid/buffer mixture. The fluorescence intensity values are then normalized to the value at lowest pH. The normalized fluorescence intensity is then plotted against pH and a line of best fit is provided.
- The point on the line of best fit at which the normalized fluorescence intensity is equal to 0.5 is found. The pH corresponding to normalized fluorescence intensity equal to 0.5 is found and is considered the pKa of the lipid.

The best immunological results are seen with lipids having a pKa in the range of 5.0 to 7.6. Within this pKa range, preferred lipids have a pKa of 5.5 to 6.7 *e.g.* between 5.6 and 6.8, between 5.6 and 6.3, between 5.6 and 6.0, between 5.5 and 6.2, or between 5.7 and 5.9.

### Specific compounds of formula (I)

Specific compounds of formula (I) which are useful with the invention are disclosed in reference 8. For instance, the compound may be E0024, E0014, E0052, E0118, E0083, E0011, E0008, E0025, E0026, E0069, E0076, E0077, E0078, E0085 or E0088. The compound may be the lipids shown below which were used in the "RV03" to "RV12" liposomes, or in the "RV15" liposomes. Preferred compounds are E0026, E0069 and E0078. Preferred compounds are the lipids shown below which were used in the "RV05", "RV08" and "RV09" liposomes.

In an alternative embodiment, rather than use a compound of formula (I), a liposome of the invention uses compound "RV02" (structure shown below). Except for this substitution, all other aspects of these RV02-containing liposomes are as described elsewhere herein.

### Formula (XI)

Compounds of formula (XI) are cationic lipopeptides which comprise a N-terminal alkylamide and from 2 to 10 amino acids. Formula (XI) is:

R^{a}-(AA)_{z}-R^{b}

wherein
R^{a} is a N-terminal alkylamide
z is an integer from 2 to 10;
each AA is an amino acid, provided that at least one histidine and at least one cationic amino acid are present;
R^{b} is -H, -OH or -NH₂.

The R^{a} moiety has formula R^{c}-C(O)-NR^{d}- where R^{c} is an C₂ to C₂₄ alkyl and R^{d} is -H or C₁ to C₄ alkyl. Suitable R^{c} groups include lauryl ('Lau'; C₁₂) and palmitoyl ('Pal'; C₁₆).

The amide of the R^{a} moiety is attached to an oligopeptide chain of from 2 to 10 amino acids *e.g.* from 3-8 amino acids. This chain includes at least one (*e.g.* 1, 2, 3, 4 or 5) histidine. It also includes at least one cationic amino acid *e.g.* at least one arginine, lysine or ornithine residue. The inclusion of at least one lysine is preferred, and ideally 2 or 3 lysines. Histidine is included because its side chain is weakly basic and predominantly un-ionized at physiological pH, but is more highly protonated in the weakly acidic environment of the endosome. A cationic amino acid, such as lysine or arginine, provides a unit positive charge on the lipopeptide at neutral pH. Useful oligopeptides have amino acid sequence -C-Kᵢ-Hⱼ- where: i is 1, 2 or 3; and j is 1, 2, 3, 4 or 5.

The C-terminus of the oligopeptide chain can be left as -COOH or can instead form an amide.

Compounds of formula (XI) can be described in terms of their alkyl chain, their amino acid sequence, and their C-terminal group. For instance, the lipopeptide "Lau-(C-K-H-H)-NH₂" has a N-terminus lauryl chain, then a cysteine, then lysine, then two histidines, and a C-terminus amine. Suitable lipopeptides of formula (XI) thus include, but are not limited to: Lau-(C-K-K-H)-NH₂, Pal-(C-K-H-H)-NH₂, Pal-(C-K-K-H-H)-NH₂, Pal-(C-K-K-H-H-H)-NH₂, Pal-(C-K-K-H-H-H-H)-NH₂, Pal-(C-K-K-H-H-H-H-H)-NH₂, Pal-(C-K-K-K-H-H)-NH₂ and Pal-(C-K-K-K-H-H-H)-NH₂. These and other compounds are disclosed in reference 9, and include:

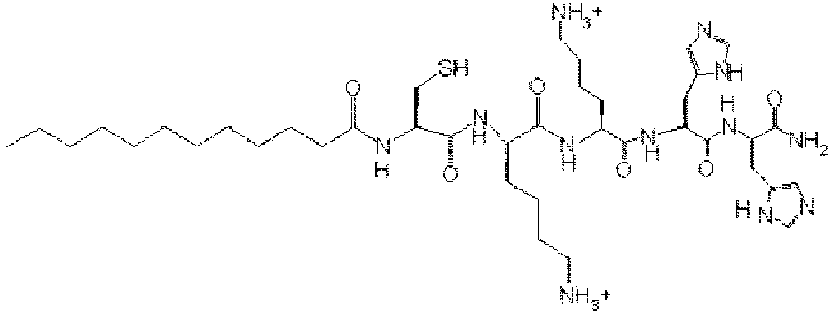

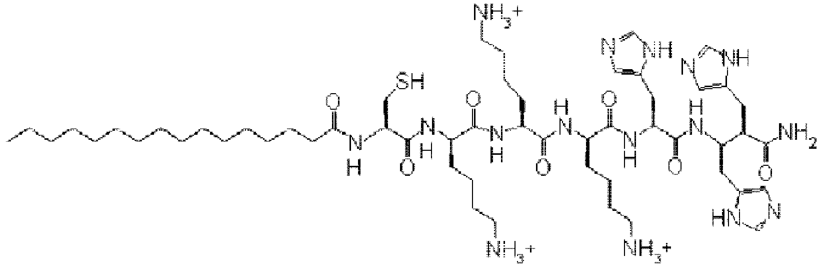

### The RNA

Liposomes of the invention include a RNA molecule which (unlike siRNA, as in reference 2) encodes a polypeptide. After *in vivo* administration of the particles, RNA is released from the particles and is translated inside a cell to provide the polypeptide *in situ.*

The RNA is +-stranded, and so it can be translated by cells without needing any intervening replication steps such as reverse transcription. It can also bind to TLR7 receptors expressed by immune cells, thereby initiating an adjuvant effect which is useful for immunisation purposes.

Preferred +-stranded RNAs are self-replicating. A self-replicating RNA molecule (replicon) can, when delivered to a vertebrate cell even without any proteins, lead to the production of multiple daughter RNAs by transcription from itself (via an antisense copy which it generates from itself). A self-replicating RNA molecule is thus typically a +-strand molecule which can be directly translated after delivery to a cell, and this translation provides a RNA-dependent RNA polymerase which then produces both antisense and sense transcripts from the delivered RNA. Thus the delivered RNA leads to the production of multiple daughter RNAs. These daughter RNAs, as well as collinear subgenomic transcripts, may be translated themselves to provide *in situ* expression of an encoded polypeptide of interest, or may be transcribed to provide further transcripts with the same sense as the delivered RNA which are translated to provide *in situ* expression of the polypeptide of interest. The overall result of this sequence of transcriptions is a huge amplification of the introduced replicon RNAs and so the encoded polypeptide of interest becomes a major product of the cells.

One suitable system for achieving self-replication is to use an alphavirus-based RNA replicon. These +-stranded replicons are translated after delivery to a cell to give of a replicase (or replicase-transcriptase). The replicase is translated as a polyprotein which auto-cleaves to provide a replication complex which creates genomic --strand copies of the +-strand delivered RNA. These --strand transcripts can themselves be transcribed to give further copies of the +-stranded parent RNA and also to give a subgenomic transcript which encodes the polypeptide of interest. Translation of the subgenomic transcript thus leads to *in situ* expression of the polypeptide by the infected cell. Suitable alphavirus replicons can use a replicase from a sindbis virus, a semliki forest virus, an eastern equine encephalitis virus, a venezuelan equine encephalitis virus, *etc.* Mutant or wild-type viruses sequences can be used *e.g.* the attenuated TC83 mutant of VEEV has been used in replicons [10].

A preferred self-replicating RNA molecule thus encodes (i) a RNA-dependent RNA polymerase which can transcribe RNA from the self-replicating RNA molecule and (ii) a polypeptide of interest. The polymerase can be an alphavirus replicase e.g. comprising one or more of alphavirus proteins nsP1, nsP2, nsP3 and nsP4.

Whereas natural alphavirus genomes encode structural virion proteins in addition to the non-structural replicase polyprotein, it is preferred that a self-replicating RNA molecule of the invention does not encode alphavirus structural proteins. Thus a preferred self-replicating RNA can lead to the production of genomic RNA copies of itself in a cell, but not to the production of RNA-containing virions. The inability to produce these virions means that, unlike a wild-type alphavirus, the self-replicating RNA molecule cannot perpetuate itself in infectious form. The alphavirus structural proteins which are necessary for perpetuation in wild-type viruses are absent from self-replicating RNAs of the invention and their place is taken by gene(s) encoding the polypeptide of interest, such that the subgenomic transcript encodes that polypeptide rather than the structural alphavirus virion proteins.

Thus a self-replicating RNA molecule useful with the invention may have two open reading frames. The first (5') open reading frame encodes a replicase; the second (3') open reading frame encodes a polypeptide of interest. In some embodiments the RNA may have additional (*e.g*. downstream) open reading frames *e.g.* to encode further polypeptides of interest (see below) or to encode accessory polypeptides.

A self-replicating RNA molecule can have a 5' sequence which is compatible with the encoded replicase.

Self-replicating RNA molecules can have various lengths but they are typically 5000-25000 nucleotides long *e.g.* 8000-15000 nucleotides, or 9000-12000 nucleotides. Thus the RNA is longer than seen in siRNA delivery.

A RNA molecule useful with the invention may have a 5' cap (*e.g.* a 7-methylguanosine). This cap can enhance *in vivo* translation of the RNA.

The 5' nucleotide of a RNA molecule useful with the invention may have a 5' triphosphate group. In a capped RNA this may be linked to a 7-methylguanosine via a 5'-to-5' bridge. A 5' triphosphate can enhance RIG-I binding and thus promote adjuvant effects.

A RNA molecule may have a 3' poly-A tail. It may also include a poly-A polymerase recognition sequence (e.g. AAUAAA) near its 3' end.

A RNA molecule useful with the invention will typically be single-stranded. Single-stranded RNAs can generally initiate an adjuvant effect by binding to TLR7, TLR8, RNA helicases and/or PKR. RNA delivered in double-stranded form (dsRNA) can bind to TLR3, and this receptor can also be triggered by dsRNA which is formed either during replication of a single-stranded RNA or within the secondary structure of a single-stranded RNA.

A RNA molecule useful with the invention can conveniently be prepared by *in vitro* transcription (IVT). IVT can use a (cDNA) template created and propagated in plasmid form in bacteria, or created synthetically (for example by gene synthesis and/or polymerase chain-reaction (PCR) engineering methods). For instance, a DNA-dependent RNA polymerase (such as the bacteriophage T7, T3 or SP6 RNA polymerases) can be used to transcribe the RNA from a DNA template. Appropriate capping and poly-A addition reactions can be used as required (although the replicon's poly-A is usually encoded within the DNA template). These RNA polymerases can have stringent requirements for the transcribed 5' nucleotide(s) and in some embodiments these requirements must be matched with the requirements of the encoded replicase, to ensure that the IVT-transcribed RNA can function efficiently as a substrate for its self-encoded replicase.

As discussed in reference 11, the self-replicating RNA can include (in addition to any 5' cap structure) one or more nucleotides having a modified nucleobase. Thus the RNA can comprise m5C (5-methylcytidine), m5U (5-methyluridine), m6A (N6-methyladenosine), s2U (2-thiouridine), Um (2'-O-methyluridine), m1A (1-methyladenosine); m2A (2-methyladenosine); Am (2'-O-methyladenosine); ms2m6A (2-methylthio-N6-methyladenosine); i6A (N6-isopentenyladenosine); ms2i6A (2-methylthio-N6isopentenyladenosine); io6A (N6-(cis-hydroxyisopentenyl)adenosine); ms2io6A (2-methylthio-N6-(cis-hydroxyisopentenyl) adenosine); g6A (N6-glycinylcarbamoyladenosine); t6A (N6-threonyl carbamoyladenosine); ms2t6A (2-methylthio-N6-threonyl carbamoyladenosine); m6t6A (N6-methyl-N6-threonylcarbamoyladenosine); hn6A(N6.-hydroxynorvalylcarbamoyl adenosine); ms2hn6A (2-methylthio-N6-hydroxynorvalyl carbamoyladenosine); Ar(p) (2'-O-ribosyladenosine (phosphate)); I (inosine); m11 (1-methylinosine); m'Im (1,2'-O-dimethylinosine); m3C (3-methylcytidine); Cm (2T-O-methylcytidine); s2C (2-thiocytidine); ac4C (N4-acetylcytidine); f5C (5-fonnylcytidine); m5Cm (5,2-0-dimethylcytidine); ac4Cm (N4acetyl2TOmethylcytidine); k2C (lysidine); m1G (1-methylguanosine); m2G (N2-methylguanosine); m7G (7-methylguanosine); Gm (2'-O-methylguanosine); m22G (N2,N2-dimethylguanosine); m2Gm (N2,2'-O-dimethylguanosine); m22Gm (N2,N2,2'-O-trimethylguanosine); Gr(p) (2'-O-ribosylguanosine (phosphate)) ; yW (wybutosine); o2yW (peroxywybutosine); OHyW (hydroxywybutosine); OHyW* (undermodified hydroxywybutosine); imG (wyosine); mimG (methylguanosine); Q (queuosine); oQ (epoxyqueuosine); galQ (galtactosyl-queuosine); manQ (mannosyl-queuosine); preQo (7-cyano-7-deazaguanosine); preQi (7-aminomethyl-7-deazaguanosine); G* (archaeosine); D (dihydrouridine); m5Um (5,2'-O-dimethyluridine); s4U (4-thiouridine); m5s2U (5-methyl-2-thiouridine); s2Um (2-thio-2'-O-methyluridine); acp3U (3-(3-amino-3-carboxypropyl)uridine); ho5U (5-hydroxyuridine); mo5U (5-methoxyuridine); cmo5U (uridine 5-oxyacetic acid); mcmo5U (uridine 5-oxyacetic acid methyl ester); chm5U (5-(carboxyhydroxymethyl)uridine)); mchm5U (5-(carboxyhydroxymethyl)uridine methyl ester); mcm5U (5-methoxycarbonyl methyluridine); mcm5Um (S-methoxycarbonylmethyl-2-O-methyluricjine); mcm5s2U (5-methoxycarbonylmethyl-2-thiouridine); nm5s2U (5-aminomethyl-2-thiouridine); mnm5U (5-methylaminomethyluridine); mnm5s2U (5-methylaminomethyl-2-thiouridine); mnm5se2U (5-methylaminomethyl-2-selenouridine); ncm5U (5-carbamoylmethyl uridine); ncm5Um (5-carbamoylmethyl-2'-O-methyluridine); cmnm5U (5-carboxymethylaminomethyluridine); cnmm5Um (5-carboxymethylaminomethyl-2-L-Omethyluridine); cmnm5s2U (5-carboxymethylaminomethyl-2-thiouridine); m62A (N6,N6-dimethyladenosine); Tm (2'-O-methylinosine); m4C (N4-methylcytidine); m4Cm (N4,2-O-dimethylcytidine); hm5C (5-hydroxymethylcytidine); m3U (3-methyluridine); cm5U (5-carboxymethyluridine); m6Am (N6,T-O-dimethyladenosine); rn62Am (N6,N6,O-2-trimethyladenosine); m2'7G (N2,7-dimethylguanosine); m2'2'7G (N2,N2,7-trimethylguanosine); m3Um (3,2T-O-dimethyluridine); m5D (5-methyldihydrouridine); f5Cm (5-formyl-2'-O-methylcytidine); m1Gm (1,2'-O-dimethylguanosine); m'Am (1,2-O-dimethyl adenosine) irinomethyluridine); tm5s2U (S-taurinomethyl-2-thiouridine)); imG-14 (4-demethyl guanosine); imG2 (isoguanosine); or ac6A (N6-acetyladenosine), hypoxanthine, inosine, 8-oxo-adenine, 7-substituted derivatives thereof, dihydrouracil, pseudouracil, 2-thiouracil, 4-thiouracil, 5-aminouracil, 5-(C1-C6)-alkyluracil, 5-methyluracil, 5-(C2-C6)-alkenyluracil, 5-(C2-C6)-alkynyluracil, 5-(hydroxymethyl)uracil, 5-chlorouracil, 5-fluorouracil, 5-bromouracil, 5-hydroxycytosine, 5-(C1-C6 )-alkylcytosine, 5-methylcytosine, 5-(C2-C6)-alkenylcytosine, 5-(C2-C6)-alkynylcytosine, 5-chlorocytosine, 5-fluorocytosine, 5-bromocytosine, N2-dimethylguanine, 7-deazaguanine, 8-azaguanine, 7-deaza-7-substituted guanine, 7-deaza-7-(C2-C6)alkynylguanine, 7-deaza-8-substituted guanine, 8-hydroxyguanine, 6-thioguanine, 8-oxoguanine, 2-aminopurine, 2-amino-6-chloropurine, 2,4-diaminopurine, 2,6-diaminopurine, 8-azapurine, substituted 7-deazapurine, 7-deaza-7-substituted purine, 7-deaza-8-substituted purine, or an abasic nucleotide. For instance, a self-replicating RNA can include one or more modified pyrimidine nucleobases, such as pseudouridine and/or 5-methylcytosine residues. In some embodiments, however, the RNA includes no modified nucleobases, and may include no modified nucleotides *i.e.* all of the nucleotides in the RNA are standard A, C, G and U ribonucleotides (except for any 5' cap structure, which may include a 7'-methylguanosine). In other embodiments, the RNA may include a 5' cap comprising a 7'-methylguanosine, and the first 1, 2 or 3 5' ribonucleotides may be methylated at the 2' position of the ribose.

A RNA used with the invention ideally includes only phosphodiester linkages between nucleosides, but in some embodiments it can contain phosphoramidate, phosphorothioate, and/or methylphosphonate linkages.

Ideally, a liposome includes fewer than 10 different species of RNA *e.g.* 5, 4, 3, or 2 different species; most preferably, a liposome includes a single RNA species *i.e.* all RNA molecules in the liposome have the same sequence and same length.

The amount of RNA per liposome can vary. The number of individual self-replicating RNA molecules per liposome is typically ≤50 *e.g.* <20, <10, <5, or 1-4 per liposome.

### The encoded polypeptide of interest

RNA molecules used with the invention encode a polypeptide of interest. After administration of the liposomes the RNA is translated *in vivo* and the resulting protein can exert its desired effect *e.g.* it can elicit an immune response in the recipient, or it can provide a function of interest, such as an enzymatic activity.

The RNA molecule can encode a single polypeptide of interest or multiple polypeptides. Multiple polypeptides can be presented as a single polypeptide (fusion polypeptide) or as separate polypeptides. If polypeptides are expressed as separate polypeptides from a replicon then one or more of these may be provided with an upstream IRES or an additional viral promoter element. Alternatively, multiple polypeptides may be expressed from a polyprotein that encodes individual polypeptide fused to a short autocatalytic protease (*e.g*. foot-and-mouth disease virus 2A protein), or as inteins.

Unlike references 1 and 12, the RNA encodes a polypeptide with a useful *in vivo* function. For the avoidance of doubt, the invention does not encompass RNA which encodes a firefly luciferase or which encodes a fusion protein of *E.coli* β-galactosidase or which encodes a green fluorescent protein (GFP). Such polypeptides may be useful as markers but the invention concerns delivery of RNA for *in vivo* expression of a polypeptide which can provide a useful therapeutic or immunological response. Also, the RNA is not total mouse thymus RNA.

### Immunogens

In some embodiments the RNA encodes a polypeptide immunogen. After administration of the liposomes the RNA is translated *in vivo* and the immunogen can elicit an immune response in the recipient. The immunogen may elicit an immune response against a bacterium, a virus, a fungus or a parasite (or, in some embodiments, against an allergen; and in other embodiments, against a tumor antigen). The immune response may comprise an antibody response (usually including IgG) and/or a cell-mediated immune response. The polypeptide immunogen will typically elicit an immune response which recognises the corresponding bacterial, viral, fungal or parasite (or allergen or tumour) polypeptide, but in some embodiments the polypeptide may act as a mimotope to elicit an immune response which recognises a bacterial, viral, fungal or parasite saccharide. The immunogen will typically be a surface polypeptide *e.g.* an adhesin, a hemagglutinin, an envelope glycoprotein, a spike glycoprotein, *etc.*

In some embodiments the immunogen elicits an immune response against one of these bacteria:
*Neisseria meningitidis:* useful immunogens include, but are not limited to, membrane proteins such as adhesins, autotransporters, toxins, iron acquisition proteins, and factor H binding protein. A combination of three useful polypeptides is disclosed in reference 13.
*Streptococcus pneumoniae:* useful polypeptide immunogens are disclosed in reference 14. These include, but are not limited to, the RrgB pilus subunit, the beta-N-acetyl-hexosaminidase precursor (spr0057), spr0096, General stress protein GSP-781 (spr2021, SP2216), serine/threonine kinase StkP (SP1732), and pneumococcal surface adhesin PsaA.
*Streptococcus pyogenes:* useful immunogens include, but are not limited to, the polypeptides disclosed in references 15 and 16.
*Moraxella catarrhalis.*
*Bordetella pertussis:* Useful pertussis immunogens include, but are not limited to, pertussis toxin or toxoid (PT), filamentous haemagglutinin (FHA), pertactin, and agglutinogens 2 and 3.
*Staphylococcus aureus:* Useful immunogens include, but are not limited to, the polypeptides disclosed in reference 17, such as a hemolysin, esxA, esxB, ferrichrome-binding protein (sta006) and/or the sta011 lipoprotein.
*Clostridium tetani:* the typical immunogen is tetanus toxoid.
*Cornynebacterium diphtheriae:* the typical immunogen is diphtheria toxoid.
*Haemophilus influenzae:* Useful immunogens include, but are not limited to, the polypeptides disclosed in references 18 and 19.
*Pseudomonas aeruginosa*
*Streptococcus agalactiae:* useful immunogens include, but are not limited to, the polypeptides disclosed in reference 15.
*Chlamydia trachomatis:* Useful immunogens include, but are not limited to, PepA, LcrE, ArtJ, DnaK, CT398, OmpH-like, L7/L12, OmcA, AtoS, CT547, Eno, HtrA and MurG (*e*.*g*. as disclosed in reference 20. LcrE [21] and HtrA [22] are two preferred immunogens.
*Chlamydia pneumoniae:* Useful immunogens include, but are not limited to, the polypeptides disclosed in reference 23.
*Helicobacter pylori:* Useful immunogens include, but are not limited to, CagA, VacA, NAP, and/or urease [24].
*Escherichia coli:* Useful immunogens include, but are not limited to, immunogens derived from enterotoxigenic *E. coli* (ETEC), enteroaggregative *E. coli* (EAggEC), diffusely adhering *E*. *coli* (DAEC), enteropathogenic *E. coli* (EPEC), extraintestinal pathogenic *E. coli* (ExPEC) and/or enterohemorrhagic *E. coli* (EHEC). ExPEC strains include uropathogenic *E.coli* (UPEC) and meningitis/sepsis-associated *E.coli* (MNEC). Useful UPEC polypeptide immunogens are disclosed in references 25 and 26. Useful MNEC immunogens are disclosed in reference 27. A useful immunogen for several *E.coli* types is AcfD [28].
*Bacillus anthracis*
*Yersinia pestis:* Useful immunogens include, but are not limited to, those disclosed in references 29 and 30.
*Staphylococcus epidermis*
*Clostridium perfringens* or *Clostridium botulinums*
*Legionella pneumophila*
*Coxiella burnetii*
*Brucella,* such as *B.abortus, B.canis, B.melitensis, B.neotomae, B.ovis, B.suis, B.pinnipediae.*
*Francisella,* such as *F.novicida, F.philomiragia, F.tularensis.*
*Neisseria gonorrhoeae*
*Treponema pallidum*
*Haemophilus ducreyi*
*Enterococcus faecalis* or *Enterococcus faecium*
*Staphylococcus saprophyticus*
*Yersinia enterocolitica*
*Mycobacterium tuberculosis*
*Rickettsia*
*Listeria monocytogenes*
*Vibrio cholerae*
*Salmonella typhi*
*Borrelia burgdorferi*
*Porphyromonas gingivalis*
*Klebsiella*

In some embodiments the immunogen elicits an immune response against one of these viruses:
*Orthomyxovirus:* Useful immunogens can be from an influenza A, B or C virus, such as the hemagglutinin, neuraminidase or matrix M2 proteins. Where the immunogen is an influenza A virus hemagglutinin it may be from any subtype *e.g*. H1, H2, H3, H4, H5, H6, H7, H8, H9, H10, H11, H12, H13, H14, H15 or H16.
*Paramyxoviridae* viruses: Viral immunogens include, but are not limited to, those derived from Pneumoviruses (*e.g.* respiratory syncytial virus, RSV), Rubulaviruses (*e.g.* mumps virus), Paramyxoviruses (*e.g.* parainfluenza virus), Metapneumoviruses and Morbilliviruses (*e.g.* measles virus).
*Poxviridae:* Viral immunogens include, but are not limited to, those derived from *Orthopoxvirus* such as *Variola vera,* including but not limited to, *Variola major* and *Variola minor.*
*Picornavirus*: Viral immunogens include, but are not limited to, those derived from Picornaviruses, such as Enteroviruses, Rhinoviruses, Heparnavirus, Cardioviruses and Aphthoviruses. In one embodiment, the enterovirus is a poliovirus *e.g.* a type 1, type 2 and/or type 3 poliovirus. In another embodiment, the enterovirus is an EV71 enterovirus. In another embodiment, the enterovirus is a coxsackie A or B virus.
*Bunyavirus:* Viral immunogens include, but are not limited to, those derived from an *Orthobunyavirus,* such as California encephalitis virus, a *Phlebovirus,* such as Rift Valley Fever virus, or a *Nairovirus,* such as *Crimean-Congo hemorrhagic fever* virus.
*Heparnavirus*: Viral immunogens include, but are not limited to, those derived from a Heparnavirus, such as hepatitis A virus (HAV).
*Filovirus*: Viral immunogens include, but are not limited to, those derived from a filovirus, such as an Ebola virus (including a Zaire, Ivory Coast, Reston or Sudan ebolavirus) or a Marburg virus.
*Togavirus*: Viral immunogens include, but are not limited to, those derived from a Togavirus, such as a Rubivirus, an Alphavirus, or an Arterivirus. This includes rubella virus.
*Flavivirus*: Viral immunogens include, but are not limited to, those derived from a Flavivirus, such as Tick-borne encephalitis (TBE) virus, Dengue (types 1, 2, 3 or 4) virus, Yellow Fever virus, Japanese encephalitis virus, Kyasanur Forest Virus, West Nile encephalitis virus, St. Louis encephalitis virus, Russian spring-summer encephalitis virus, Powassan encephalitis virus.
*Pestivirus*: Viral immunogens include, but are not limited to, those derived from a Pestivirus, such as Bovine viral diarrhea (BVDV), Classical swine fever (CSFV) or Border disease (BDV).
*Hepadnavirus*: Viral immunogens include, but are not limited to, those derived from a Hepadnavirus, such as Hepatitis B virus. A composition can include hepatitis B virus surface antigen (HBsAg).
*Other hepatitis viruses:* A composition can include an immunogen from a hepatitis C virus, delta hepatitis virus, hepatitis E virus, or hepatitis G virus.
*Rhabdovirus*: Viral immunogens include, but are not limited to, those derived from a Rhabdovirus, such as a Lyssavirus (*e.g*. a Rabies virus) and Vesiculovirus (VSV).
*Caliciviridae:* Viral immunogens include, but are not limited to, those derived from Calciviridae, such as Norwalk virus (Norovirus), and Norwalk-like Viruses, such as Hawaii Virus and Snow Mountain Virus.
*Coronavirus:* Viral immunogens include, but are not limited to, those derived from a SARS coronavirus, avian infectious bronchitis (IBV), Mouse hepatitis virus (MHV), and Porcine transmissible gastroenteritis virus (TGEV). The coronavirus immunogen may be a spike polypeptide.
*Retrovirus*: Viral immunogens include, but are not limited to, those derived from an Oncovirus, a Lentivirus (*e*.*g*. HIV-1 or HIV-2) or a Spumavirus.
*Reovirus*: Viral immunogens include, but are not limited to, those derived from an Orthoreovirus, a Rotavirus, an Orbivirus, or a Coltivirus.
*Parvovirus:* Viral immunogens include, but are not limited to, those derived from Parvovirus B19.
*Herpesvirus*: Viral immunogens include, but are not limited to, those derived from a human herpesvirus, such as, by way of example only, Herpes Simplex Viruses (HSV) (*e.g*. HSV types 1 and 2), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8).
*Papovaviruses*: Viral immunogens include, but are not limited to, those derived from Papillomaviruses and Polyomaviruses. The (human) papillomavirus may be of serotype 1, 2, 4, 5, 6, 8, 11, 13, 16, 18, 31, 33, 35, 39, 41, 42, 47, 51, 57, 58, 63 or 65 *e.g.* from one or more of serotypes 6, 11, 16 and/or 18.
*Adenovirus*: Viral immunogens include those derived from adenovirus serotype 36 (Ad-36).

In some embodiments, the immunogen elicits an immune response against a virus which infects fish, such as: infectious salmon anemia virus (ISAV), salmon pancreatic disease virus (SPDV), infectious pancreatic necrosis virus (IPNV), channel catfish virus (CCV), fish lymphocystis disease virus (FLDV), infectious hematopoietic necrosis virus (IHNV), koi herpesvirus, salmon picorna-like virus (also known as picorna-like virus of atlantic salmon), landlocked salmon virus (LSV), atlantic salmon rotavirus (ASR), trout strawberry disease virus (TSD), coho salmon tumor virus (CSTV), or viral hemorrhagic septicemia virus (VHSV).

Fungal immunogens may be derived from Dermatophytres, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme;* or from *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Microsporidia, Encephalitozoon* spp., *Septata intestinalis* and *Enterocytozoon bieneusi*; the less common are *Brachiola* spp, *Microsporidium* spp., *Nosema* spp., *Pleistophora* spp., *Trachipleistophora* spp., *Vittaforma* spp *Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp,* and *Cladosporium spp.*

In some embodiments the immunogen elicits an immune response against a parasite from the *Plasmodium* genus, such as *P.falciparum, P.vivax, P.malariae* or *P.ovale.* Thus the invention may be used for immunising against malaria. In some embodiments the immunogen elicits an immune response against a parasite from the *Caligidae* family, particularly those from the *Lepeophtheirus* and *Caligus* genera *e.g.* sea lice such as *Lepeophtheirus salmonis* or *Caligus rogercresseyi.*

In some embodiments the immunogen elicits an immune response against: pollen allergens (tree-, herb, weed-, and grass pollen allergens); insect or arachnid allergens (inhalant, saliva and venom allergens, *e.g.* mite allergens, cockroach and midges allergens, hymenopthera venom allergens); animal hair and dandruff allergens (from *e.g.* dog, cat, horse, rat, mouse, *etc*.); and food allergens *(e.g.* a gliadin). Important pollen allergens from trees, grasses and herbs are such originating from the taxonomic orders of Fagales, Oleales, Pinales and platanaceae including, but not limited to, birch (Betula), alder (Alnus), hazel (Corylus), hornbeam (Carpinus) and olive (Olea), cedar (Cryptomeria and Juniperus), plane tree (Platanus), the order of Poales including grasses of the genera Lolium, Phleum, Poa, Cynodon, Dactylis, Holcus, Phalaris, Secale, and Sorghum, the orders of Asterales and Urticales including herbs of the genera Ambrosia, Artemisia, and Parietaria. Other important inhalation allergens are those from house dust mites of the genus Dermatophagoides and Euroglyphus, storage mite e.g. Lepidoglyphys, Glycyphagus and Tyrophagus, those from cockroaches, midges and fleas *e.g.* Blatella, Periplaneta, Chironomus and Ctenocepphalides, and those from mammals such as cat, dog and horse, venom allergens including such originating from stinging or biting insects such as those from the taxonomic order of Hymenoptera including bees (*Apidae*)*,* wasps (*Vespidea*)*,* and ants (*Formicoidae*)*.*

In some embodiments the immunogen is a tumor antigen selected from: (a) cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors; (b) mutated antigens, for example, p53 (associated with various solid tumors, *e.g.,* colorectal, lung, head and neck cancer), p21/Ras (associated with, *e.g.,* melanoma, pancreatic cancer and colorectal cancer), CDK4 (associated with, *e*.*g*., melanoma), MUM1 (associated with, *e.g.,* melanoma), caspase-8 (associated with, *e.g.,* head and neck cancer), CIA 0205 (associated with, *e.g.,* bladder cancer), HLA-A2-R1701, beta catenin (associated with, *e.g.,* melanoma), TCR (associated with, *e*.*g*., T-cell non-Hodgkins lymphoma), BCR-abl (associated with, *e*.*g*., chronic myelogenous leukemia), triosephosphate isomerase, KIA 0205, CDC-27, and LDLR-FUT; (c) over-expressed antigens, for example, Galectin 4 (associated with, *e*.*g*., colorectal cancer), Galectin 9 (associated with, *e.g.,* Hodgkin's disease), proteinase 3 (associated with, *e.g.,* chronic myelogenous leukemia), WT 1 (associated with, *e*.*g*., various leukemias), carbonic anhydrase (associated with, *e.g.,* renal cancer), aldolase A (associated with, *e.g.,* lung cancer), PRAME (associated with, *e.g.,* melanoma), HER-2/neu (associated with, *e.g.,* breast, colon, lung and ovarian cancer), mammaglobin, alpha-fetoprotein (associated with, *e*.*g*., hepatoma), KSA (associated with, *e.g.,* colorectal cancer), gastrin (associated with, *e.g.,* pancreatic and gastric cancer), telomerase catalytic protein, MUC-1 (associated with, *e*.*g*., breast and ovarian cancer), G-250 (associated with, *e.g.,* renal cell carcinoma), p53 (associated with, *e.g.,* breast, colon cancer), and carcinoembryonic antigen (associated with, *e*.*g*., breast cancer, lung cancer, and cancers of the gastrointestinal tract such as colorectal cancer); (d) shared antigens, for example, melanoma-melanocyte differentiation antigens such as MART-1/Melan A, gp100, MC1R, melanocyte-stimulating hormone receptor, tyrosinase, tyrosinase related protein-1/TRP1 and tyrosinase related protein-2/TRP2 (associated with, *e*.*g*., melanoma); (e) prostate associated antigens such as PAP, PSA, PSMA, PSH-P1, PSM-P1, PSM-P2, associated with *e*.*g*., prostate cancer; (f) immunoglobulin idiotypes (associated with myeloma and B cell lymphomas, for example). In certain embodiments, tumor immunogens include, but are not limited to, p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein/cyclophilin C-associated protein), TAAL6, TAG72, TLP, TPS, and the like.

### Gene therapy

In some embodiments the RNA encodes a polypeptide which is useful in a gene therapy context. This encoded protein is provided in addition to any polypeptides which are encoded for a RNA's ability to self-replicate. Thus the RNA may encode an enzyme (for example, an enzyme which does not bind to RNA), a cytokine, a transmembrane receptor, an ion channel, a hormone, a blood protein, or an antibody. The RNA preferably encodes a human polypeptide in these categories.

Enzymes of interest include, but are not limited to: DNA polymerase alpha, DNA polymerase delta,

Cytokines of interest include, but are not limited to: interleukin 1; interleukin 2; interleukin 4; interleukin 6; interleukin 7; interleukin 12; interleukin 17; GM-CSF; G-CSF; TNF-alpha; interferon alpha; interferon beta; interferon gamma; and secretoneurin.

Receptors of interest include, but are not limited to: the leptin receptor; the low-density lipoprotein receptor; the bone morphogenetic protein type 2 receptor; the TNF receptor; the gonadotropin-releasing hormone receptor; the dopamine receptor; the somatostatin receptor; the vitamin D receptor; the urokinase plasminogen activator receptor; the transferrin receptor; *etc.*

Ion channels of interest include, but are not limited to: HCN2; HCN4; CFTR; the α-subunit of the Maxi-K channel; KCNQ2; KCNQ3; and Kv1.5.

Hormones of interest include, but are not limited to: chorionic gonadotropin; corticotrophin; erythropoietin; glucagons; IGF-1; oxytocin; platelet-derived growth factor; calcitonin; follicle-stimulating hormone; luteinizing hormone; thyroid-stimulating hormone; insulin; gonadotropin-releasing hormone; vasopressin; somatostatin; prolactin; adrenocorticotropic hormone; antidiuretic hormone; thyrotropin-releasing hormone; octreotide; human growth hormone; relaxin; growth hormone-releasing hormone; parathyroid hormone; calcitrol; calciferol; atrial-natriuretic peptide; gastrin; secretin; cholecystokinin; leptin; neuropeptide Y; ghrelin; angiotensinogen; dopamine; and thrombopoietin. Where a hormone requires multiple polypeptide subunits for activity, the RNA may encode one or more of such subunits *e.g.* the RNA may encode the alpha subunit and/or the beta subunit of follicle-stimulating hormone.

Blood proteins of interest include, but are not limited to: haemoglobin; fibrinogen; factor VII; factor VIIa; factor VIII; factor IX; fibrinogen; thrombin; von Willebrand factor.

### Pharmaceutical compositions

Liposomes of the invention are useful as components in pharmaceutical compositions for immunising subjects against various diseases. These compositions will typically include a pharmaceutically acceptable carrier in addition to the liposomes. A thorough discussion of pharmaceutically acceptable carriers is available in reference 31.

A pharmaceutical composition of the invention may include one or more small molecule immunopotentiators. For example, the composition may include a TLR2 agonist (e.g. Pam3CSK4), a TLR4 agonist (*e.g.* an aminoalkyl glucosaminide phosphate, such as E6020), a TLR7 agonist (*e.g.* imiquimod), a TLR8 agonist (*e.g.* resiquimod) and/or a TLR9 agonist (*e.g.* IC31). Any such agonist ideally has a molecular weight of <2000Da. In some embodiments such agonist(s) are also encapsulated with the RNA inside liposomes, but in other embodiments they are unencapsulated.

Pharmaceutical compositions of the invention may include the liposomes in plain water (*e.g*. w.f.i.) or in a buffer *e.g.* a phosphate buffer, a Tris buffer, a borate buffer, a succinate buffer, a histidine buffer, or a citrate buffer. Buffer salts will typically be included in the 5-20mM range.

Pharmaceutical compositions of the invention may have a pH between 5.0 and 9.5 *e.g.* between 6.0 and 8.0.

Compositions of the invention may include sodium salts (*e.g*. sodium chloride) to give tonicity. A concentration of 10±2 mg/ml NaCl is typical *e.g.* about 9 mg/ml.

Compositions of the invention may include metal ion chelators. These can prolong RNA stability by removing ions which can accelerate phosphodiester hydrolysis. Thus a composition may include one or more of EDTA, EGTA, BAPTA, pentetic acid, *etc..* Such chelators are typically present at between 10-500µM *e.g.* 0.1mM. A citrate salt, such as sodium citrate, can also act as a chelator, while advantageously also providing buffering activity.

Pharmaceutical compositions of the invention may have an osmolality of between 200 mOsm/kg and 400 mOsm/kg, e.g. between 240-360 mOsm/kg, or between 290-310 mOsm/kg.

Pharmaceutical compositions of the invention may include one or more preservatives, such as thiomersal or 2-phenoxyethanol. Mercury-free compositions are preferred, and preservative-free vaccines can be prepared.

Pharmaceutical compositions of the invention are preferably sterile.

Pharmaceutical compositions of the invention are preferably non-pyrogenic e.g. containing <1 EU (endotoxin unit, a standard measure) per dose, and preferably <0.1 EU per dose.

Pharmaceutical compositions of the invention are preferably gluten free.

Pharmaceutical compositions of the invention may be prepared in unit dose form. In some embodiments a unit dose may have a volume of between 0.1-1.0ml *e.g.* about 0.5ml.

The compositions may be prepared as injectables, either as solutions or suspensions. The composition may be prepared for pulmonary administration *e.g.* by an inhaler, using a fine spray. The composition may be prepared for nasal, aural or ocular administration *e.g.* as spray or drops. Injectables for intramuscular administration are typical.

Compositions comprise an immunologically effective amount of liposomes, as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g*. non-human primate, primate, *etc.*), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. The liposome and RNA content of compositions of the invention will generally be expressed in terms of the amount of RNA per dose. A preferred dose has ≤100µg RNA (*e.g*. from 10-100µg, such as about 10µg, 25µg, 50µg, 75µg or 100µg), but expression can be seen at much lower levels *e.g.* ≤1µg/dose, ≤100ng/dose, ≤10ng/dose, ≤1ng/dose, *etc*

The invention also provides a delivery device (*e.g*. syringe, nebuliser, sprayer, inhaler, dermal patch, *etc*.) containing a pharmaceutical composition of the invention. This device can be used to administer the composition to a vertebrate subject.

Liposomes of the invention do not contain ribosomes.

### Methods of treatment and medical uses

In contrast to the particles disclosed in reference 12, liposomes and pharmaceutical compositions of the invention are for *in vivo* use for eliciting an immune response against an immunogen of interest, or for gene therapy.

The invention provides a method for raising an immune response in a vertebrate comprising the step of administering an effective amount of a liposome or pharmaceutical composition of the invention. The immune response is preferably protective and preferably involves antibodies and/or cell-mediated immunity. The method may raise a booster response.

The invention also provides a liposome or pharmaceutical composition of the invention for use in a method for raising an immune response in a vertebrate.

The invention also provides a liposome or pharmaceutical composition of the invention for use in a method of gene therapy in a vertebrate.

The invention also provides the use of a liposome of the invention in the manufacture of a medicament for raising an immune response in a vertebrate.

By raising an immune response in the vertebrate by these uses and methods, the vertebrate can be protected against various diseases and/or infections e.g. against bacterial and/or viral diseases as discussed above. The liposomes and compositions are immunogenic, and are more preferably vaccine compositions. Vaccines according to the invention may either be prophylactic (*i.e.* to prevent infection) or therapeutic (*i.e.* to treat infection), but will typically be prophylactic.

The vertebrate is preferably a mammal, such as a human or a large veterinary mammal (*e*.*g*. horses, cattle, deer, goats, pigs). Where the vaccine is for prophylactic use, the human is preferably a child (*e.g.* a toddler or infant) or a teenager; where the vaccine is for therapeutic use, the human is preferably a teenager or an adult. A vaccine intended for children may also be administered to adults *e.g.* to assess safety, dosage, immunogenicity, *etc.*

Vaccines prepared according to the invention may be used to treat both children and adults. Thus a human patient may be less than 1 year old, less than 5 years old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g.* ≤5 years old), hospitalised patients, healthcare workers, armed service and military personnel, pregnant women, the chronically ill, or immunodeficient patients. The vaccines are not suitable solely for these groups, however, and may be used more generally in a population.

Compositions of the invention will generally be administered directly to a patient. Direct delivery may be accomplished by parenteral injection (*e.g*. subcutaneously, intraperitoneally, intravenously, intramuscularly, intradermally, or to the interstitial space of a tissue; unlike reference 1, intraglossal injection is not typically used with the present invention). Alternative delivery routes include rectal, oral (*e.g.* tablet, spray), buccal, sublingual, vaginal, topical, transdermal or transcutaneous, intranasal, ocular, aural, pulmonary or other mucosal administration. Intradermal and intramuscular administration are two preferred routes. Injection may be via a needle (*e.g.* a hypodermic needle), but needle-free injection may alternatively be used. A typical intramuscular dose is 0.5 ml.

The invention may be used to elicit systemic and/or mucosal immunity, preferably to elicit an enhanced systemic and/or mucosal immunity.

Dosage can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Multiple doses will typically be administered at least 1 week apart (e.g. about 2 weeks, about 3 weeks, about 4 weeks, about 6 weeks, about 8 weeks, about 10 weeks, about 12 weeks, about 16 weeks, *etc*.). In one embodiment, multiple doses may be administered approximately 6 weeks, 10 weeks and 14 weeks after birth, e.g. at an age of 6 weeks, 10 weeks and 14 weeks, as often used in the World Health Organisation's Expanded Program on Immunisation ("EPI"). In an alternative embodiment, two primary doses are administered about two months apart, e.g. about 7, 8 or 9 weeks apart, followed by one or more booster doses about 6 months to 1 year after the second primary dose, e.g. about 6, 8, 10 or 12 months after the second primary dose. In a further embodiment, three primary doses are administered about two months apart, e.g. about 7, 8 or 9 weeks apart, followed by one or more booster doses about 6 months to 1 year after the third primary dose, e.g. about 6, 8, 10, or 12 months after the third primary dose.

### Chemical terms and definitions

### Halo

The term "halogen" (or "halo") includes fluorine, chlorine, bromine and iodine.

### Alkyl, alkylene, alkenyl, alkynyl, cycloalkyl etc.

The terms "alkyl", "alkylene", "alkenyl" and "alkynyl" are used herein to refer to both straight and branched chain acyclic forms. Cyclic analogues thereof are referred to as cycloalkyl, *etc.*

The term "alkyl" includes monovalent, straight or branched, saturated, acyclic hydrocarbyl groups. In one embodiment alkyl is C₁₋₁₀alkyl, in another embodiment C₁₋₆alkyl, in another embodiment C₁₋₄alkyl, such as methyl, ethyl, n-propyl, i-propyl or t-butyl groups.

The term "cycloalkyl" includes monovalent, saturated, cyclic hydrocarbyl groups. In one embodiment cycloalkyl is C₃₋₁₀cycloalkyl, in another embodiment C₃₋₆cycloalkyl such as cyclopentyl and cyclohexyl.

The term "alkoxy" means alkyl-O-.

The term "alkenyl" includes monovalent, straight or branched, unsaturated, acyclic hydrocarbyl groups having at least one carbon-carbon double bond and, in one embodiment, no carbon-carbon triple bonds. In one embodiment alkenyl is C₂₋₁₀alkenyl, in another embodiment C₂₋₆alkenyl, in another embodiment C₂₋₄alkenyl.

The term "cycloalkenyl" includes monovalent, partially unsaturated, cyclic hydrocarbyl groups having at least one carbon-carbon double bond and, in one embodiment, no carbon-carbon triple bonds. In one embodiment cycloalkenyl is C₃₋₁₀cycloalkenyl, in another embodiment C₅₋₁₀cycloalkenyl, *e*.*g*. cyclohexenyl or benzocyclohexyl.

The term "alkynyl" includes monovalent, straight or branched, unsaturated, acyclic hydrocarbyl groups having at least one carbon-carbon triple bond and, in one embodiment, no carbon-carbon double bonds. In one embodiment, alkynyl is C₂₋₁₀alkynyl, in another embodiment C₂₋₆alkynyl, in another embodiment C₂₋₄alkynyl.

The term "cycloalkynyl" includes monovalent, partially unsaturated, cyclic hydrocarbyl groups having at least one carbon-carbon triple bond and, in one embodiment, no carbon-carbon double bonds. In one embodiment cycloalkynyl is C₃₋₁₀cycloalkenyl, in another embodiment C₅₋₁₀cycloalkynyl.

The term "alkylene" includes divalent, straight or branched, saturated, acyclic hydrocarbyl groups. In one embodiment alkylene is C₁₋₁₀alkylene, in another embodiment C₁₋₆alkylene, in another embodiment C₁₋₄alkylene, such as methylene, ethylene, n-propylene, i-propylene or t-butylene groups.

The term "alkenylene" includes divalent, straight or branched, unsaturated, acyclic hydrocarbyl groups having at least one carbon-carbon double bond and, in one embodiment, no carbon-carbon triple bonds. In one embodiment alkenylene is C₂₋₁₀alkenylene, in another embodiment C₂₋₆alkenylene, in another embodiment C₂₋₄alkenylene.

The term "alkynylene" includes divalent, straight or branched, unsaturated, acyclic hydrocarbyl groups having at least one carbon-carbon triple bond and, in one embodiment, no carbon-carbon double bonds. In one embodiment alkynylene is C₂₋₁₀alkynylene, in another embodiment C₂₋₆alkynylene, in another embodiment C₂₋₄alkynylene.

### Heteroalkyl etc.

The term "heteroalkyl" includes alkyl groups in which up to six carbon atoms, in one embodiment up to five carbon atoms, in another embodiment up to four carbon atoms, in another embodiment up to three carbon atoms, in another embodiment up to two carbon atoms, in another embodiment one carbon atom, are each replaced independently by O, S(O)_{q}, N, P(O)ᵣ or Si (and preferably O, S(O)_{q} or N), provided at least one of the alkyl carbon atoms remains. The heteroalkyl group may be C-linked or hetero-linked, *i.e.* it may be linked to the remainder of the molecule through a carbon atom or through O, S(O)_{q}, N, P(O)ᵣ or Si.

The term "heterocycloalkyl" includes cycloalkyl groups in which up to six carbon atoms, in one embodiment up to five carbon atoms, in another embodiment up to four carbon atoms, in another embodiment up to three carbon atoms, in another embodiment up to two carbon atoms, in another embodiment one carbon atom, are each replaced independently by O, S(O)_{q} or N, provided at least one of the cycloalkyl carbon atoms remains. Examples of heterocycloalkyl groups include oxiranyl, thiaranyl, aziridinyl, oxetanyl, thiatanyl, azetidinyl, tetrahydrofuranyl, tetrahydrothiophenyl, pyrrolidinyl, tetrahydropyranyl, tetrahydrothiopyranyl, piperidinyl, 1,4-dioxanyl, 1,4-oxathianyl, morpholinyl, 1,4-dithianyl, piperazinyl, 1,4-azathianyl, oxepanyl, thiepanyl, azepanyl, 1,4-dioxepanyl, 1,4-oxathiepanyl, 1,4-oxaazepanyl, 1,4-dithiepanyl, 1,4-thieazepanyl and 1,4-diazepanyl. The heterocycloalkyl group may be C-linked or N-linked, *i.e.* it may be linked to the remainder of the molecule through a carbon atom or through a nitrogen atom.

The term "heteroalkenyl" includes alkenyl groups in which up to three carbon atoms, in one embodiment up to two carbon atoms, in another embodiment one carbon atom, are each replaced independently by O, S(O)_{q} or N, provided at least one of the alkenyl carbon atoms remains. The heteroalkenyl group may be C-linked or hetero-linked, *i.e.* it may be linked to the remainder of the molecule through a carbon atom or through O, S(O)_{q} or N.

The term "heterocycloalkenyl" includes cycloalkenyl groups in which up to three carbon atoms, in one embodiment up to two carbon atoms, in another embodiment one carbon atom, are each replaced independently by O, S(O)_{q} or N, provided at least one of the cycloalkenyl carbon atoms remains. Examples of heterocycloalkenyl groups include 3,4-dihydro-2H-pyranyl, 5-6-dihydro-2H-pyranyl, 2H-pyranyl, 1,2,3,4-tetrahydropyridinyl and 1,2,5,6-tetrahydropyridinyl. The heterocycloalkenyl group may be C-linked or N-linked, *i.e.* it may be linked to the remainder of the molecule through a carbon atom or through a nitrogen atom.

The term "heteroalkynyl" includes alkynyl groups in which up to three carbon atoms, in one embodiment up to two carbon atoms, in another embodiment one carbon atom, are each replaced independently by O, S(O)_{q} or N, provided at least one of the alkynyl carbon atoms remains. The heteroalkynyl group may be C-linked or hetero-linked, *i.e.* it may be linked to the remainder of the molecule through a carbon atom or through O, S(O)_{q} or N.

The term "heterocycloalkynyl" includes cycloalkynyl groups in which up to three carbon atoms, in one embodiment up to two carbon atoms, in another embodiment one carbon atom, are each replaced independently by O, S(O)_{q} or N, provided at least one of the cycloalkynyl carbon atoms remains. The heterocycloalkenyl group may be C-linked or N-linked, *i.e.* it may be linked to the remainder of the molecule through a carbon atom or through a nitrogen atom.

The term "heteroalkylene" includes alkylene groups in which up to three carbon atoms, in one embodiment up to two carbon atoms, in another embodiment one carbon atom, are each replaced independently by O, S(O)_{q} or N, provided at least one of the alkylene carbon atoms remains.

The term "heteroalkenylene" includes alkenylene groups in which up to three carbon atoms, in one embodiment up to two carbon atoms, in another embodiment one carbon atom, are each replaced independently by O, S(O)_{q} or N, provided at least one of the alkenylene carbon atoms remains.

The term "heteroalkynylene" includes alkynylene groups in which up to three carbon atoms, in one embodiment up to two carbon atoms, in another embodiment one carbon atom, are each replaced independently by O, S(O)_{q} or N, provided at least one of the alkynylene carbon atoms remains.

### Aryl

The term "aryl" includes monovalent, aromatic, cyclic hydrocarbyl groups, such as phenyl or naphthyl (e.g. 1-naphthyl or 2-naphthyl). In general, the aryl groups may be monocyclic or polycyclic fused ring aromatic groups. Preferred aryl are C₆-C₁₄aryl.

Other examples of aryl groups are monovalent derivatives of aceanthrylene, acenaphthylene, acephenanthrylene, anthracene, azulene, chrysene, coronene, fluoranthene, fluorene, as-indacene, s-indacene, indene, naphthalene, ovalene, perylene, phenalene, phenanthrene, picene, pleiadene, pyrene, pyranthrene and rubicene.

The term "arylalkyl" means alkyl substituted with an aryl group, e.g. benzyl.

The term "arylene" includes divalent aromatic, cyclic hydrocarbyl groups, such as phenylene. In general, the arylene groups may be monocyclic or polycyclic fused ring aromatic groups. Preferred arylene are C₆-C₁₄arylene. Other examples of arylene groups are divalent derivatives of aceanthrylene, acenaphthylene, acephenanthrylene, anthracene, azulene, chrysene, coronene, fluoranthene, fluorene, as-indacene, s-indacene, indene, naphthalene, ovalene, perylene, phenalene, phenanthrene, picene, pleiadene, pyrene, pyranthrene and rubicene.

### Heteroaryl

The term "heteroaryl" includes monovalent, heteroaromatic, cyclic hydrocarbyl groups additionally containing one or more heteroatoms independently selected from O, S, N and NR^{N}, where R^{N} is defined below (and in one embodiment is H or alkyl (*e.g.* C₁₋₆alkyl)).

In general, the heteroaryl groups may be monocyclic or polycyclic (*e*.*g*. bicyclic) fused ring heteroaromatic groups. In one embodiment, heteroaryl groups contain 5-13 ring members (preferably 5-10 members) and 1, 2, 3 or 4 ring heteroatoms independently selected from O, S, N and NR^{N}. In one embodiment, a heteroaryl group may be 5, 6, 9 or 10 membered, *e.g.* 5-membered monocyclic, 6-membered monocyclic, 9-membered fused-ring bicyclic or 10-membered fused-ring bicyclic.

Monocyclic heteroaromatic groups include heteroaromatic groups containing 5-6 ring members and 1, 2, 3 or 4 heteroatoms selected from O, S, N or NR^{N}.

In one embodiment, 5-membered monocyclic heteroaryl groups contain 1 ring member which is an -NR^{N}- group, an -O- atom or an -S- atom and, optionally, 1-3 ring members (*e.g*. 1 or 2 ring members) which are =N- atoms (where the remainder of the 5 ring members are carbon atoms).

Examples of 5-membered monocyclic heteroaryl groups are pyrrolyl, furanyl, thiophenyl, pyrazolyl, imidazolyl, isoxazolyl, oxazolyl, isothiazolyl, thiazolyl, 1,2,3 triazolyl, 1,2,4 triazolyl, 1,2,3 oxadiazolyl, 1,2,4 oxadiazolyl, 1,2,5 oxadiazolyl, 1,3,4 oxadiazolyl, 1,3,4 thiadiazolyl, pyridyl, pyrimidinyl, pyridazinyl, pyrazinyl, 1,3,5 triazinyl, 1,2,4 triazinyl, 1,2,3 triazinyl and tetrazolyl.

Examples of 6-membered monocyclic heteroaryl groups are pyridinyl, pyridazinyl, pyrimidinyl and pyrazinyl.

In one embodiment, 6-membered monocyclic heteroaryl groups contain 1 or 2 ring members which are =N- atoms (where the remainder of the 6 ring members are carbon atoms).

Bicyclic heteroaromatic groups include fused-ring heteroaromatic groups containing 9-13 ring members and 1, 2, 3, 4 or more heteroatoms selected from O, S, N or NR^{N}.

In one embodiment, 9-membered bicyclic heteroaryl groups contain 1 ring member which is an -NR^{N}- group, an -O- atom or an -S- atom and, optionally, 1-3 ring members (*e.g*. 1 or 2 ring members) which are =N- atoms (where the remainder of the 9 ring members are carbon atoms).

Examples of 9-membered fused-ring bicyclic heteroaryl groups are benzofuranyl, benzothiophenyl, indolyl, benzimidazolyl, indazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,2-a]pyridinyl, pyrrolo[1,2-b]pyridazinyl and imidazo[1,2-c]pyrimidinyl.

In one embodiment, 10-membered bicyclic heteroaryl groups contain 1-3 ring members which are =N- atoms (where the remainder of the 10 ring members are carbon atoms).

Examples of 10-membered fused-ring bicyclic heteroaryl groups are quinolinyl, isoquinolinyl, cinnolinyl, quinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "heteroarylalkyl" means alkyl substituted with a heteroaryl group.

The term "heteroarylene" includes divalent heteroaromatic, cyclic hydrocarbyl groups additionally containing one or more heteroatoms independently selected from O, S, N and NR^{N}, where R^{N} is defined below (and in one embodiment is H or alkyl (*e.g.* C₁₋₆alkyl)). In general, the heteroarylene groups may be monocyclic or polycyclic (*e*.*g*. bicyclic) fused ring heteroaromatic groups. In one embodiment, heteroarylene groups contain 5-13 ring members (preferably 5-10 members) and 1, 2, 3 or 4 ring heteroatoms independently selected from O, S, N and NR^{N}. In one embodiment, a heteroarylene group may be 5, 6, 9 or 10 membered, *e.g.* 5-membered monocyclic, 6-membered monocyclic, 9-membered fused-ring bicyclic or 10-membered fused-ring bicyclic. The term "heteroarylene" includes divalent derivatives of each of the heteroaryl groups discussed above.

The terms "aryl", "aromatic", "heteroaryl" and "heteroaromatic" also include groups that are partially reduced. Thus, for example, "heteroaryl" includes fused species in which one of the rings has been reduced to a saturated ring (e.g. 1,2,3,4-tetrahydro-1,8-naphthyridin-2-yl).

### Absent groups

When group a, b or c in formula (I) is "absent", what is meant is that a single bond is present instead, i.e. that the two groups either side of group a, b or c are directly bonded to each other.

### General

Unless indicated explicitly otherwise, where combinations of groups are referred to herein as one moiety, e.g. arylalkyl, the last mentioned group contains the atom by which the moiety is attached to the rest of the molecule.

Where reference is made to a carbon atom of an alkyl group or other group being replaced by O, S(O)_{q}, N or P(O)ᵣ, what is intended is that: 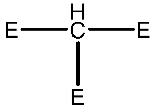 is replaced by 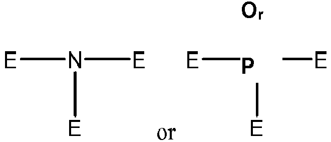 (wherein E cannot be H);
-CH= is replaced by -N= or -P(O)ᵣ=;
≡ C-H is replaced by ≡N or ≡P(O)ᵣ; or
-CH₂- is replaced by -O-, -S(O)_{q}-, -NR^{N}- or -P(O)ᵣR^{N}-, where R^{N} is H or optionally substituted C₁₋₆alkyl, C₁₋₆heteroalkyl, C₃₋₆cycloalkyl, C₃₋₆heterocycloalkyl, C₂₋₆alkenyl, C₂₋₆heteroalkenyl, C₃₋₆cycloalkenyl, C₃₋₆heterocycloalkenyl, phenyl, or heteroaryl containing 5 or 6 ring members. R^{N} is preferably H, C₁₋₆alkyl or C₃₋₆cycloalkyl.

q is independently 0, 1 or 2. In one embodiment, q is 0.

r is independently 0 or 1. In one embodiment, r is 0.

Where reference is made to a carbon atom being replaced by Si, what is intended is that the carbon atom is swapped for a silicon atom but that the bonds otherwise remain the same. Thus, for example, -CH₂- is replaced by -SiH₂-; -CH= is replaced by -SiH=; and ≡C-H is replaced by ≡Si-H.

By way of clarification, in relation to the above mentioned heteroatom containing groups (such as heteroalkyl *etc*.), where a numerical of carbon atoms is given, for instance C₃₋₆heteroalkyl, what is intended is a group based on C₃₋₆alkyl in which one or more of the 3-6 chain carbon atoms is replaced by O, S(O)_{q} or N. Accordingly, a C₃₋₆heteroalkyl group would, for example, contain less than 3-6 chain carbon atoms. As another example, a pyridyl group would be classed as a C₆ heteroaryl group even though it contains 5 carbon atoms.

### Substitution

Groups of the compounds of the invention (*e*.*g*. alkyl, cycloalkyl, alkoxy, alkenyl, cycloalkenyl, alkynyl, alkylene, alkenylene, heteroalkyl, heterocycloalkyl, heteroalkenyl, heterocycloalkenyl, heteroalkynyl, heteroalkylene, heteroalkenylene aryl, arylalkyl, arylheteroalkyl, heteroaryl, heteroarylalkyl or heteroarylheteroalkyl groups *etc*.) may be substituted or unsubstituted, in one embodiment unsubstituted. Typically, substitution involves the notional replacement of a hydrogen atom with a substituent group, or two hydrogen atoms in the case of substitution by =O.

Where substituted, there will generally be 1 to 5 substituents on each group, in one embodiment 1 to 3 substituents, in one embodiment 1 or 2 substituents, in one embodiment 1 substituent. One embodiment includes more than one substituent on the same atom, e.g. an acetal group.

In one embodiment, the substituent(s) is/are independently Sub¹ or Sub² (in one embodiment Sub²) wherein:
Sub¹ is independently halogen, trihalomethyl, trihaloethyl, -NO₂, -CN, -N⁺(R^{s})₂O⁻, -CO₂H, -CO₂R^{s}, -SO₃H, -SOR^{s}, -SO₂R^{s}, -SO₃R^{s}, -OC(=O)OR^{s}, -C(=O)H, -C(=O)R^{s}, -OC(=O)R^{s}, =O, -NR^{s}₂, -C(=O)NH₂, -C(=O)NR^{s}₂, -N(R^{s})C(=O)OR^{s}, -N(R^{s})C(=O)NR^{s}₂, -OC(=O)NR^{s}₂, -N(R^{s})C(=O)R^{s}, -C(=S)NR^{s}₂, -NR^{s}C(=S)R^{s}, -SO₂NR^{s}₂, -NR^{s}SO₂R^{s}, -N(R^{s})C(=S)NR^{s}₂, -N(R^{s})SO₂NR^{s}₂, -R^{s} or -Z^{s}R^{s}, wherein;
   Z^{s} is independently O, S or NR^{s};
   R^{s} is independently H or C₁₋₆alkyl, C₁₋₆heteroalkyl, -(Alk^{a})_{f}-C₃₋₆cycloalkyl, -(Alk^{a})_{f}-C₃₋₆heterocycloalkyl, C₂₋₆alkenyl, C₂₋₆heteroalkenyl, -(Alk^{a})_{f}-C₃₋₆cycloalkenyl, -(Alk^{a})_{f}-C₃₋₆heterocycloalkenyl, C₂₋₆alkynyl, C₂₋₆heteroalkynyl, -(Alk^{a})_{f}-C₆₋₁₄aryl, -(Alk^{a})_{f}-C₆₋₁₄aryl or -(Alk^{a})_{f}-heteroaryl (where heteroaryl contains 5-13 ring members), where
   f is 0 or 1;
   Alk^{a} is C₁₋₆alkylene or C₁₋₆heteroalkylene; and
   R^{s} is optionally substituted itself (in one embodiment unsubstituted) by 1 to 3 substituents Sub²;
Sub² is independently halogen, trihalomethyl, trihaloethyl, -NO₂, -CN, -N⁺(C₁₋₆alkyl)₂O⁻, -CO₂H, -CO₂C₁₋₆alkyl, -SO₃H, -SOC₁₋₆alkyl, -SO₂C₁₋₆alkyl, -SO₃C₁₋₆alkyl, -OC(=O)OC₁₋₆alkyl, -C(=O)H, -C(=O)C₁₋₆alkyl, -OC(=O)C₁₋₆alkyl, =O, -N(C₁₋₆alkyl)₂, -C(=O)NH₂, -C(=O)N(C₁₋₆alkyl)₂, -N(C₁₋₆alkyl)C(=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=O)C₁₋₆alkyl, -C(=S)N(C₁₋₆alkyl)₂, -N(C₁₋₆alkyl)C(=S)C₁₋₆alkyl, -SO₂N(C₁₋₆alkyl)₂, -N(C₁₋₆alkyl)SO₂C₁₋₆alkyl, -N(C₁₋₆alkyl)C(=S)N(C₁₋₆alkyl)₂, -N(C₁₋₆alkyl)SO₂N(C₁₋₆alkyl)₂, -C₁₋₆alkyl, -C₁₋₆heteroalkyl, -C₃₋₆cycloalkyl, -C₃₋₆heterocycloalkyl, -C₂₋₆alkenyl, -C₂₋₆heteroalkenyl, -C₃₋₆cycloalkenyl, -C₃₋₆heterocycloalkenyl, -C₂₋₆alkynyl, -C₂₋₆heteroalkynyl, -C₆₋₁₄aryl, -C₅₋₁₃heteroaryl, -Z^{t}-C₁₋₆alkyl, -Z^{t}-C₃₋₆cycloalkyl, -Z^{t}-C₂₋₆alkenyl, -Z^{t-}C₃₋₆cycloalkenyl, or -Z^{t}-C₂₋₆alkynyl; and
Z^{t} is independently O, S, NH or N(C₁₋₆alkyl).

While R^{s} in Sub¹ can be optionally substituted by 1 to 3 substituents Sub², Sub² is unsubstituted. However, in one embodiment, R^{s} is unsubstituted.

In one embodiment, R^{s} is H or C₁₋₆alkyl, optionally substituted by 1 to 3 substituents Sub².

In one embodiment, Sub² is independently halogen, trihalomethyl, trihaloethyl, -NO₂, -CN, -N⁺(C₁₋₆alkyl)₂O⁻, -CO₂H, -SO₃H, -SOC₁₋₆alkyl, -SO₂C₁₋₆alkyl, -C(=O)H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{t}-C₁₋₆alkyl or - Z^{t}-C₃₋₆cycloalkyl.

In one embodiment, where the substituted group is acyclic (*e.g.* alkyl, heteroalkyl, alkenyl *etc*.), Sub¹ is not -R^{s} and Sub² is not -C₁₋₆alkyl, -C₁₋₆heteroalkyl, -C₂₋₆alkenyl, -C₂₋₆heteroalkenyl, -C₂₋₆alkynyl or -C₂₋₆heteroalkynyl.

Where a group other than Sub² has at least 2 positions which may be substituted, the group may be substituted by both ends of an alkylene, alkenylene, alkynylene, heteroalkylene, heteroalkenylene or heteroalkynylene chain (in one embodiment containing 1 to 6 atoms, in a further embodiment 3 to 6 atoms, and in a further embodiment 3 or 4 atoms) to form a cyclic moiety. That chain is optionally substituted by 1 to 3 substituents Sub². In one embodiment that chain is not substituted. Thus, the terms optionally substituted "cycloalkyl", "cycloalkenyl", "cycloalkynyl", "heterocycloalkyl", "heterocycloalkenyl", "heterocycloalkynyl", "aryl" and "heteroaryl" include fused species. E.g. "optionally substituted cycloalkyl" includes a species in which two cycloalkyl rings are fused, and "optionally substituted heteroaryl" includes a species in which a heterocycloalkyl ring is fused to the aromatic ring (e.g. 5,6,7,8-tetrahydro-1,8-naphthyridin-2-yl).

Where a group other than Sub² has an atom which may be substituted twice, that atom may be substituted by both ends of an alkylene, alkenylene, alkynylene, heteroalkylene, heteroalkenylene or heteroalkynylene chain (in one embodiment containing 2 to 8 atoms, in a further embodiment 3 to 6 atoms, and in a further embodiment 4 or 5 atoms) to form a cyclic moiety. That chain is optionally substituted by 1 to 3 substituents Sub². In one embodiment that chain is not substituted. Thus, the terms optionally substituted "cycloalkyl", "cycloalkenyl", "cycloalkynyl", "heterocycloalkyl", "heterocycloalkenyl", "heterocycloalkynyl", "aryl" and "heteroaryl" include spiro species.

By way of clarification, when a group has a heteroatom, a substituent may be bonded to the heteroatom. Thus, for example, "optionally substituted heteroalkyl" includes -CH₂-N(Sub¹)-CH₂-, -CH(Sub¹)-NH-CH₂- and -CH(Sub¹)-N(Sub¹)-CH₂- etc.

### Modifier terms

When a list is preceded by a modifier, it is intended that the modifier is to be understood as applying to each of the items in the list. For example, the phrase "optionally substituted C₃₋₂₀-heterocycloalkyl, C₃₋₂₀-heterocycloalkenyl, C₃₋₂₀-heterocycloalkynyl or C₅₋₂₀-heteroaryl group" means that each of the four items in the list, namely the C₃₋₂₀-heterocycloalkyl group, the C₃₋₂₀-heterocycloalkenyl group, the C₃₋₂₀-heterocycloalkynyl group and the C₆₋₂₀-heteroaryl group, may be optionally substituted.

When a group is characterised by a first modifier and then, later on, the same group is characterised by a subsequent modifier, what is meant is that the group is characterised by both modifiers simultaneously. For example, if a group is described as a "C₃₋₂₀-heterocycloalkynyl" (the first modifier) group and then later the same group is described as a "C₅₋₁₆" (the subsequent modifier) group, what is meant is a C₅₋₁₆ heterocycloalkynyl group.

### Steroids

As used herein, the term "steroid" refers to any group comprising the following structure (which structure is referred to herein as the "steroid skeleton").

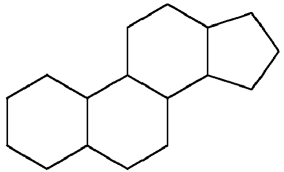

Purely for the purposes of illustration, the steroid skeleton has been drawn above as fully saturated. The term steroid, however, is also intended to cover instances where there is unsaturation in the steroid skeleton. For example, the term steroid covers a group which comprises the fully unsaturated (mancude) basic skeleton, 15*H*-cyclopenta[*a*]phenanthrene:

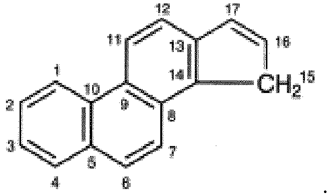

The term steroid also covers a group which comprises a partially unsaturated steroid skeleton.

The term steroid also covers "seco" derivatives of the steroid skeleton, i.e. groups in which ring cleavage has been effected; "nor" and "homo" derivatives of the steroid skeleton which involve ring contraction and expansion, respectively (see Systemic Nomenclature of Organic Chemistry, by D. Hellwinkel, published by Springer, 2001, ISBN: 3-540-41138-0, page 203 for "seco" and page 204 for "nor" and "homo"). In one embodiment, however, such seco derivatives are not encompassed by the term "steroid". In another embodiment, such nor derivatives are not encompassed by the term "steroid". In another embodiment, such homo derivatives are not encompassed by the term "steroid". Thus in one embodiment, such seco, nor and homo derivatives are not encompassed by the term "steroid".

The term steroid also covers instances where one or more of the carbon atoms in the structure labelled steroid skeleton is replaced by a heteroatom. In one such embodiment, up to six carbon atoms, in one embodiment up to five carbon atoms, in another embodiment up to four carbon atoms, in another embodiment up to three carbon atoms, in another embodiment up to two carbon atoms, in another embodiment one carbon atom, are each replaced independently by O, S(O)_{q}, N, P(O)ᵣ or Si (and preferably O, S(O)_{q} or N). In one embodiment, however, the term "steroid" comprises species in which the "steroid basic skeleton" contains no heteroatoms.

A steroid ring system is numbered according to the convention set out below.

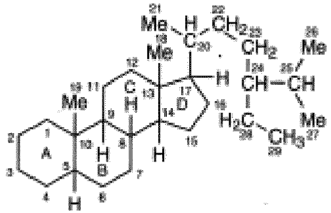

The term steroid encompasses sterols, steroid hormones, bile acids and salts of bile acids. A sterol is any steroid with a hydroxyl group at the 3-position of the A-ring.

### Unsaturation

In accordance with standard use, the omega-3 position refers to the third bond from the (methyl) terminal of the chain; the omega-6 position refers to the sixth bond from the (methyl) terminal of the chain and the omega-9 position refers to the ninth bond from the (methyl) terminal of the chain.

### General

The practice of the present invention will employ, unless otherwise indicated, conventional methods of chemistry, biochemistry, molecular biology, immunology and pharmacology, within the skill of the art. Such techniques are explained fully in the literature. See, *e.g.*, references 32-38, *etc.*

The term "comprising" encompasses "including" as well as "consisting" *e.g*. a composition "comprising" X may consist exclusively of X or may include something additional *e.g*. X + Y.

The term "about" in relation to a numerical value *x* is optional and means, for example, *x*±10%.

The word "substantially" does not exclude "completely" *e.g*. a composition which is "substantially free" from Y may be completely free from Y. Where necessary, the word "substantially" may be omitted from the definition of the invention.

References to charge, to cations, to anions, to zwitterions, *etc.*, are taken at pH 7.

TLR3 is the Toll-like receptor 3. It is a single membrane-spanning receptor which plays a key role in the innate immune system. Known TLR3 agonists include poly(I:C). "TLR3" is the approved HGNC name for the gene encoding this receptor, and its unique HGNC ID is HGNC: 11849. The RefSeq sequence for the human TLR3 gene is GI:2459625.

TLR7 is the Toll-like receptor 7. It is a single membrane-spanning receptor which plays a key role in the innate immune system. Known TLR7 agonists include e.g. imiquimod. "TLR7" is the approved HGNC name for the gene encoding this receptor, and its unique HGNC ID is HGNC:15631. The RefSeq sequence for the human TLR7 gene is GI:67944638.

TLR8 is the Toll-like receptor 8. It is a single membrane-spanning receptor which plays a key role in the innate immune system. Known TLR8 agonists include *e.g*. resiquimod. "TLR8" is the approved HGNC name for the gene encoding this receptor, and its unique HGNC ID is HGNC:15632. The RefSeq sequence for the human TLR8 gene is GI:20302165.

The RIG-I-like receptor ("RLR") family includes various RNA helicases which play key roles in the innate immune system[39]. RLR-1 (also known as RIG-1 or retinoic acid inducible gene I) has two caspase recruitment domains near its N-terminus. The approved HGNC name for the gene encoding the RLR-1 helicase is "DDX58" (for DEAD (Asp-Glu-Ala-Asp) box polypeptide 58) and the unique HGNC ID is HGNC:19102. The RefSeq sequence for the human RLR-1 gene is GI:77732514. RLR-2 (also known as MDA5 or melanoma differentiation-associated gene 5) also has two caspase recruitment domains near its N-terminus. The approved HGNC name for the gene encoding the RLR-2 helicase is "IFIH1" (for interferon induced with helicase C domain 1) and the unique HGNC ID is HGNC: 18873. The RefSeq sequence for the human RLR-2 gene is GI: 27886567. RLR-3 (also known as LGP2 or laboratory of genetics and physiology 2) has no caspase recruitment domains. The approved HGNC name for the gene encoding the RLR-3 helicase is "DHX58" (for DEXH (Asp-Glu-X-His) box polypeptide 58) and the unique HGNC ID is HGNC:29517. The RefSeq sequence for the human RLR-3 gene is GI:149408121.

PKR is a double-stranded RNA-dependent protein kinase. It plays a key role in the innate immune system. "EIF2AK2" (for eukaryotic translation initiation factor 2-alpha kinase 2) is the approved HGNC name for the gene encoding this enzyme, and its unique HGNC ID is HGNC:9437. The RefSeq sequence for the human PKR gene is GI:208431825.

### BRIEF DESCRIPTION OF DRAWINGS

FIG. 1 shows a gel with stained RNA. Lanes show (1) markers (2) naked replicon (3) replicon after RNase treatment (4) replicon encapsulated in liposome (5) liposome after RNase treatment (6) liposome treated with RNase then subjected to phenol/chloroform extraction.
FIG. 2 is an electron micrograph of liposomes.
FIG. 3 shows protein expression (as relative light units, RLU) at day 6 after delivery of RNA in liposomes with various cationic lipids.
FIG. 4 shows a gel with stained RNA. Lanes show (1) markers (2) naked replicon (3) replicon encapsulated in liposome (4) liposome treated with RNase then subjected to phenol/chloroform extraction.
FIG. 5 shows protein expression at days 1, 3 and 6 after delivery of RNA as a virion-packaged replicon (squares), as naked RNA (diamonds), or in liposomes (+ = 0.1µg, x = 1µg).
FIG. 6 shows protein expression at days 1, 3 and 6 after delivery of four different doses of liposome-encapsulated RNA.
FIG. 7 shows anti-F IgG titers in animals receiving virion-packaged replicon (VRP or VSRP), 1µg naked RNA, and 1µg liposome-encapsulated RNA.
FIG. 8 shows anti-F IgG titers in animals receiving VRP, 1µg naked RNA, and 0.1g or 1µg liposome-encapsulated RNA.
FIG. 9 shows neutralising antibody titers in animals receiving VRP or either 0.1g or 1µg liposome-encapsulated RNA.
FIG. 10 shows expression levels after delivery of a replicon as naked RNA (circles), liposome-encapsulated RNA (triangle & square), or as a lipoplex (inverted triangle).
FIG. 11 shows F-specific IgG titers (2 weeks after second dose) after delivery of a replicon as naked RNA (0.01-1µg), liposome-encapsulated RNA (0.01-10µg), or packaged as a virion (VRP, 10⁶ infectious units or IU).
FIG. 12 shows F-specific IgG titers (circles) and PRNT titers (squares) after delivery of a replicon as naked RNA (1µg), liposome-encapsulated RNA (0.1 or 1µg), or packaged as a virion (VRP, 10⁶ IU). Titers in naive mice are also shown. Solid lines show geometric means.
FIG. 13 shows intracellular cytokine production after restimulation with synthetic peptides representing the major epitopes in the F protein, 4 weeks after a second dose. The y-axis shows the % cytokine+ of CD8+CD4-.
FIG. 14 shows F-specific IgG titers (mean log₁₀ titers ± std dev) over 63 days after immunisation of cows at days 0 & 21.

### MODES FOR CARRYING OUT THE INVENTION

### RNA replicons

Various replicons are used below. In general these are based on a hybrid alphavirus genome with non-structural proteins from venezuelan equine encephalitis virus (VEEV), a packaging signal from sindbis virus, and a 3' UTR from Sindbis virus or a VEEV mutant. The replicon is about 10kb long and has a poly-A tail.

Plasmid DNA encoding alphavirus replicons (named: pT7-mVEEV-FL.RSVF or A317; pT7-mVEEV-SEAP or A306; pSP6-VCR-GFP or A50) served as a template for synthesis of RNA *in vitro.* The replicons contain the alphavirus genetic elements required for RNA replication but lack those encoding gene products necessary for particle assembly; the structural proteins are instead replaced by a protein of interest (either a reporter, such as SEAP or GFP, or an immunogen, such as full-length RSV F protein) and so the replicons are incapable of inducing the generation of infectious particles. A bacteriophage (T7 or SP6) promoter upstream of the alphavirus cDNA facilitates the synthesis of the replicon RNA *in vitro* and a hepatitis delta virus (HDV) ribozyme immediately downstream of the poly(A)-tail generates the correct 3'-end through its self-cleaving activity.

Following linearization of the plasmid DNA downstream of the HDV ribozyme with a suitable restriction endonuclease, run-off transcripts were synthesized in vitro using T7 or SP6 bacteriophage derived DNA-dependent RNA polymerase. Transcriptions were performed for 2 hours at 37°C in the presence of 7.5 mM (T7 RNA polymerase) or 5 mM (SP6 RNA polymerase) of each of the nucleoside triphosphates (ATP, CTP, GTP and UTP) following the instructions provided by the manufacturer (Ambion). Following transcription the template DNA was digested with TURBO DNase (Ambion). The replicon RNA was precipitated with LiCl and reconstituted in nuclease-free water. Uncapped RNA was capped post-transcriptionally with Vaccinia Capping Enzyme (VCE) using the ScriptCap m7G Capping System (Epicentre Biotechnologies) as outlined in the user manual; replicons capped in this way are given the "v" prefix e.g. vA317 is the A317 replicon capped by VCE. Post-transcriptionally capped RNA was precipitated with LiCl and reconstituted in nuclease-free water. The concentration of the RNA samples was determined by measuring OD₂₆₀ₙₘ. Integrity of the *in vitro* transcripts was confirmed by denaturing agarose gel electrophoresis.

### Encapsulation in DlinDMA-based liposomes

RNA was encapsulated in liposomes made essentially by the method of references 7 and 40. The liposomes were made of 10% DSPC (zwitterionic), 40% DlinDMA (cationic), 48% cholesterol and 2% PEG-conjugated DMG (2kDa PEG). These proportions refer to the % moles in the total liposome.

DlinDMA (1,2-dilinoleyloxy-N,N-dimethyl-3-aminopropane) was synthesized using the procedure of reference 2. DSPC (1,2-Diastearoyl-sn-glycero-3-phosphocholine) was purchased from Genzyme. Cholesterol was obtained from Sigma-Aldrich. PEG-conjugated DMG (1,2-dimyristoyl-sn-glycero-3-phosphoethanolamine-N-[methoxy(polyethylene glycol), ammonium salt), DOTAP (1,2-dioleoyl-3-trimethylammonium-propane, chloride salt) and DC-chol (3β-[N-(N',N'-dimethylaminoethane)-carbamoyl]cholesterol hydrochloride) were from Avanti Polar Lipids.

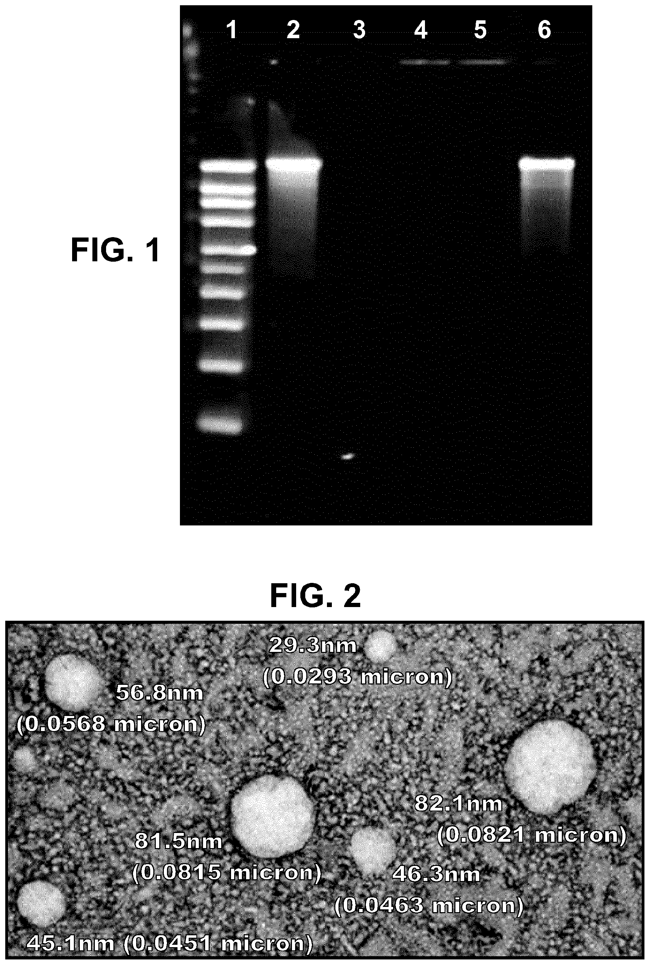

Briefly, lipids were dissolved in ethanol (2ml), a RNA replicon was dissolved in buffer (2ml, 100mM sodium citrate, pH 6) and these were mixed with 2ml of buffer followed by 1 hour of equilibration. The mixture was diluted with 6ml buffer then filtered. The resulting product contained liposomes, with ∼95% encapsulation efficiency. FIG. 2 shows an example electron micrograph of liposomes prepared by these methods. These liposomes contain encapsulated RNA encoding full-length RSV F antigen. Dynamic light scattering of one batch showed an average diameter of 141nm (Zav by intensity) or 78nm (by number).

In one particular encapsulation method, fresh lipid stock solutions in ethanol were prepared. 37 mg of DlinDMA, 11.8 mg of DSPC, 27.8 mg of Cholesterol and 8.07 mg of PEG-conjugated DMG were weighed and dissolved in 7.55 mL of ethanol. Three different conjugated PEGs were used: PEG-1000, PEG-2000 or PEG-3000. The freshly prepared lipid stock solution was gently rocked at 37°C for about 15 min to form a homogenous mixture. Then, 226.7 µL of the stock was added to 1.773 mL ethanol to make a working lipid stock solution of 2 mL. A 2 mL working solution of RNA was also prepared from a stock solution of ∼ 1µg/µL in 100 mM citrate buffer (pH 6). Three 20 mL glass vials (with stir bars) were rinsed with RNase Away solution and washed with plenty of MilliQ water before use to decontaminate the vials of RNAses. One of the vials was used for the RNA working solution and the others for collecting the lipid and RNA mixes (as described later). The working lipid and RNA solutions were heated at 37°C for 10 min before being loaded into 3cc luer-lok syringes. 2 mL of citrate buffer (pH 6) was loaded in another 3 cc syringe. Syringes containing RNA and the lipids were connected to a T mixer (PEEK™ 500 µm ID junction) using FEP tubing (fluorinated ethylene-propylene; all FEP tubing used had a 2mm internal diameter and a 3mm outer diameter; obtained from Idex Health Science). The outlet from the T mixer was also FEP tubing. The third syringe containing the citrate buffer was connected to a separate piece of tubing. All syringes were then driven at a flow rate of 7 mL/min using a syringe pump. The tube outlets were positioned to collect the mixtures in a 20 mL glass vial (while stirring). The stir bar was taken out and the ethanol/aqueous solution was allowed to equilibrate to room temperature for 1 hour. 4 ml of the mixture was loaded into a 5 cc syringe, which was connected to a piece of FEP tubing and in another 5 cc syringe connected to an equal length of FEP tubing, an equal amount of 100 mM citrate buffer (pH 6) was loaded. The two syringes were driven at 7mL/min flow rate using the syringe pump and the final mixture collected in a 20 mL glass vial (while stirring). Next, the mixture collected from the second mixing step (liposomes) were passed through a Mustang Q membrane (an anion-exchange support that binds and removes anionic molecules, obtained from Pall Corporation). Before using this membrane for the liposomes, 4 mL of 1 M NaOH, 4 mL of 1 M NaCl and 10 mL of 100 mM citrate buffer (pH 6) were successively passed through it. Liposomes were warmed for 10 min at 37°C before passing through the membrane. Next, liposomes were concentrated to 2 mL and dialyzed against 10-15 volumes of IX PBS using by tangential flow filtration before recovering the final product. The TFF system and hollow fiber filtration membranes were purchased from Spectrum Labs (Rancho Dominguez) and were used according to the manufacturer's guidelines. Polysulfone hollow fiber filtration membranes with a 100 kD pore size cutoff and 8 cm² surface area were used. For *in vitro* and *in vivo* experiments formulations were diluted to the required RNA concentration with IX PBS.

The percentage of encapsulated RNA and RNA concentration were determined by Quant-iT RiboGreen RNA reagent kit (Invitrogen), following manufacturer's instructions. The ribosomal RNA standard provided in the kit was used to generate a standard curve. Liposomes were diluted 10x or 100x in IX TE buffer (from kit) before addition of the dye. Separately, liposomes were diluted 10x or 100x in IX TE buffer containing 0.5% Triton X before addition of the dye (to disrupt the liposomes and thus to assay total RNA). Thereafter an equal amount of dye was added to each solution and then ∼180 µL of each solution after dye addition was loaded in duplicate into a 96 well tissue culture plate. The fluorescence (Ex 485 nm, Em 528 nm) was read on a microplate reader. All liposome formulations were dosed *in vivo* based on the encapsulated amount of RNA.

To obtain smaller liposomes the syringe/tube method was replaced by a method in which the lipid and RNA solutions are mixed in channels on a microfluidic chip. Fresh lipid stock solutions in ethanol were prepared. 37 mg of DlinDMA, 11.8 mg of DSPC, 27.8 mg of cholesterol and 8.07 mg of PEG-DMG were weighed and dissolved in 7.55 mL of ethanol. The freshly prepared lipid stock solution was gently rocked at 37°C for about 15 min to form a homogenous mixture. Then, 226.7 µL of the stock was added to 1.773 mL ethanol to make a working lipid stock solution of 2 mL. A 4 mL working solution of RNA was also prepared from a stock solution of ∼ 1µg/µL in 100 mM citrate buffer (pH 6). Four 20 mL glass vials (with stir bars) were rinsed with RNase Away solution and washed with plenty of MilliQ water before use to decontaminate the vials of RNAses. Two of the vials were used for the RNA working solution (2 mL in each vial) and the others for collecting the lipid and RNA mixes. The working lipid and RNA solutions were heated at 37°C for 10 min before being loaded into 3cc luer-lok syringes. Syringes containing RNA and the lipids were connected to a Mitos Droplet junction Chip (a glass microfluidic device obtained from Syrris, Part no. 3000158) using PTFE tubing 0.03 inches ID x 1/16 inch OD, (Syrris) using a 4-way edge connector. Two RNA streams and one lipid stream were driven by syringe pumps and the mixing of the ethanol and aqueous phase was done at the X junction (100 µm x 105 µm) of the chip. The flow rate of all three streams was kept at 1.5 mL/min, hence the ratio of total aqueous to ethanolic flow rate was 2:1. The tube outlet was positioned to collect the mixtures in a 20 mL glass vial (while stirring). The stir bar was taken out and the ethanol/aqueous solution was allowed to equilibrate to room temperature for 1 hour. Then the mixture was loaded in a 5 cc syringe which was fitted to a piece of PTFE tubing 0.03 inches ID x 1/16inches OD and in another 5 cc syringe with equal length of PTFE tubing, an equal volume of 100 mM citrate buffer (pH 6) was loaded. The two syringes were driven at 3mL/min flow rate using a syringe pump and the final mixture collected in a 20 mL glass vial (while stirring). Next, liposomes were concentrated to 2 mL and dialyzed against 10-15 volumes of IX PBS using the TFF system before recovering the final product. Hollow fiber filtration membranes with a 100 kDa pore size cutoff and 20cm² surface area were used. For *in vitro* and *in vivo* experiments, formulations were diluted to the required RNA concentration with IX PBS. Whereas liposomes prepared using the syringe/tube method with 75µg RNA had a Z average diameter of 148nm and a polydispersity index of 0.122, the chip mixing gave liposomes with a Z average diameter of 97nm and a polydispersity index of 0.086. The proportion of encapsulated RNA decreased slightly from 90% to 87%.

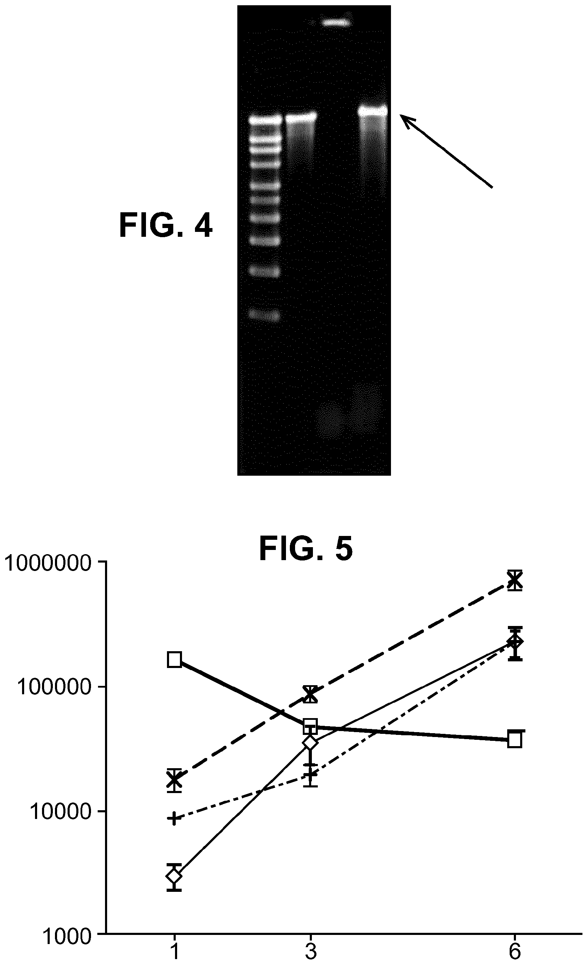

Encapsulation in liposomes was shown to protect RNA from RNase digestion. Experiments used 3.8mAU of RNase A per microgram of RNA, incubated for 30 minutes at room temperature. RNase was inactivated with Proteinase K at 55°C for 10 minutes. A 1:1 v/v mixture of sample to 25:24:1 v/v/v, phenol:chloroform:isoamyl alcohol was then added to extract the RNA from the lipids into the aqueous phase. Samples were mixed by vortexing for a few seconds and then placed on a centrifuge for 15 minutes at 12k RPM. The aqueous phase (containing the RNA) was removed and used to analyze the RNA. Prior to loading (400 ng RNA per well) all the samples were incubated with formaldehyde loading dye, denatured for 10 minutes at 65°C and cooled to room temperature. Ambion Millennium markers were used to approximate the molecular weight of the RNA construct. The gel was run at 90 V. The gel was stained using 0.1% SYBR gold according to the manufacturer's guidelines in water by rocking at room temperature for 1 hour. FIG. 1 shows that RNase completely digests RNA in the absence of encapsulation (lane 3). RNA is undetectable after encapsulation (lane 4), and no change is seen if these liposomes are treated with RNase (lane 4). After RNase-treated liposomes are subjected to phenol extraction, undigested RNA is seen (lane 6). Even after 1 week at 4°C the RNA could be seen without any fragmentation (FIG. 4, arrow). Protein expression *in vivo* was unchanged after 6 weeks at 4 °C and one freeze-thaw cycle. Thus liposome-encapsulated RNA is stable.

To assess *in vivo* expression of the RNA a reporter enzyme (SEAP; secreted alkaline phosphatase) was encoded in the replicon, rather than an immunogen. Expression levels were measured in sera diluted 1:4 in IX Phospha-Light dilution buffer using a chemiluminescent alkaline phosphate substrate. 8-10 week old BALB/c mice (5/group) were injected intramuscularly on day 0, 50µl per leg with 0.1µg or 1µg RNA dose. The same vector was also administered without the liposomes (in RNase free IX PBS) at 1µg. Virion-packaged replicons were also tested. Virion-packaged replicons used herein (referred to as "VRPs") were obtained by the methods of reference 41, where the alphavirus replicon is derived from the mutant VEEV or a chimera derived from the genome of VEEV engineered to contain the 3' UTR of Sindbis virus and a Sindbis virus packaging signal (PS), packaged by co-electroporating them into BHK cells with defective helper RNAs encoding the Sindbis virus capsid and glycoprotein genes.

As shown in FIG. 5, encapsulation increased SEAP levels by about ½ log at the 1µg dose, and at day 6 expression from a 0.1µg encapsulated dose matched levels seen with 1µg unencapsulated dose. By day 3 expression levels exceeded those achieved with VRPs (squares). Thus expressed increased when the RNA was formulated in the liposomes relative to the naked RNA control, even at a 10x lower dose. Expression was also higher relative to the VRP control, but the kinetics of expression were very different (see FIG. 5). Delivery of the RNA with electroporation resulted in increased expression relative to the naked RNA control, but these levels were lower than with liposomes.

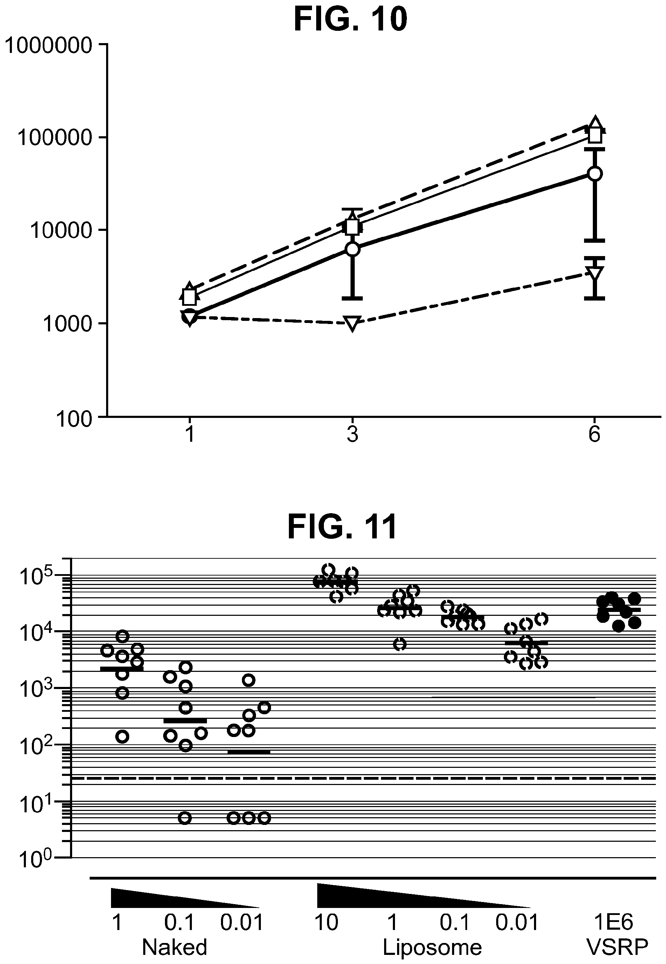

To assess whether the effect seen in the liposome groups was due merely to the liposome components, or was linked to the encapsulation, the replicon was administered in encapsulated form (with two different purification protocols, 0.1µg RNA), or mixed with the liposomes after their formation (a non-encapsulated "lipoplex", 0.1µg RNA), or as naked RNA (1µg). FIG. 10 shows that the lipoplex gave the lowest levels of expression, showing that shows encapsulation is essential for potent expression.

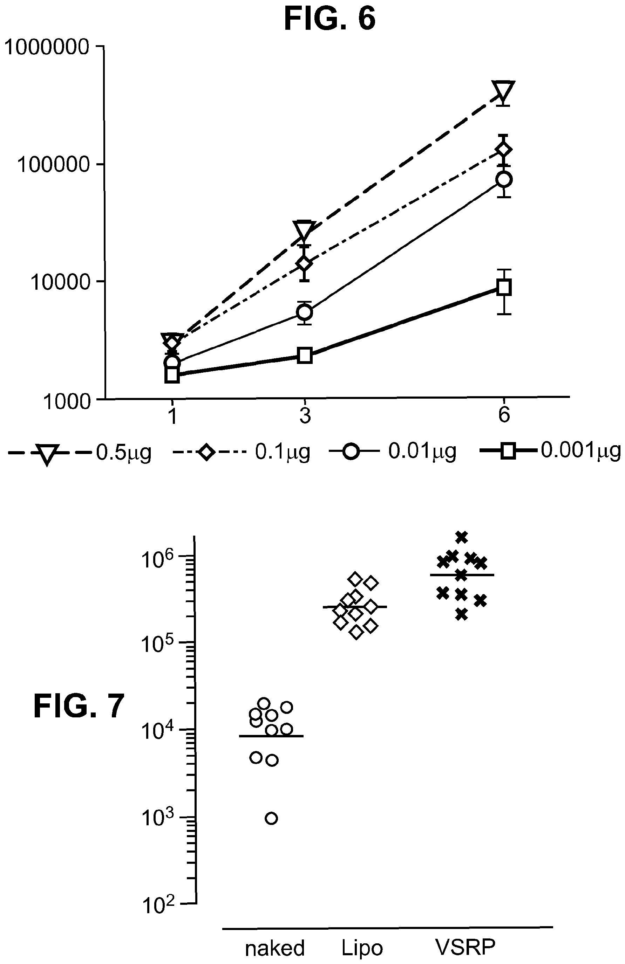

Further SEAP experiments showed a clear dose response *in vivo,* with expression seen after delivery of as little as 1ng RNA (FIG. 6). Further experiments comparing expression from encapsulated and naked replicons indicated that 0.01 µg encapsulated RNA was equivalent to 1µg of naked RNA. At a 0.5µg dose of RNA the encapsulated material gave a 12-fold higher expression at day 6; at a 0.1µg dose levels were 24-fold higher at day 6.

Rather than looking at average levels in the group, individual animals were also studied. Whereas several animals were non-responders to naked replicons, encapsulation eliminated non-responders.

Further experiments replaced DlinDMA with DOTAP ("RV13"). Although the DOTAP liposomes gave better expression than naked replicon, they were inferior to the DlinDMA liposomes (2- to 3-fold difference at day 1).

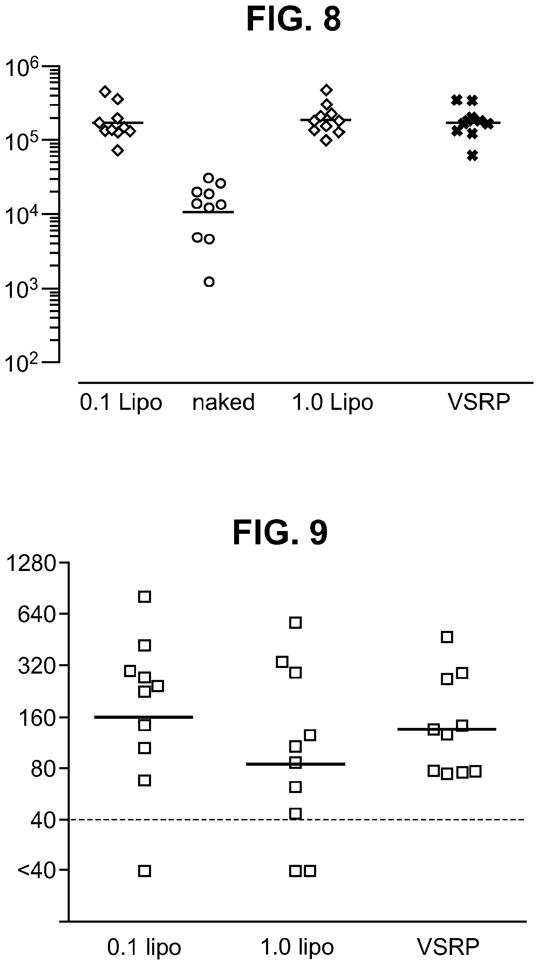

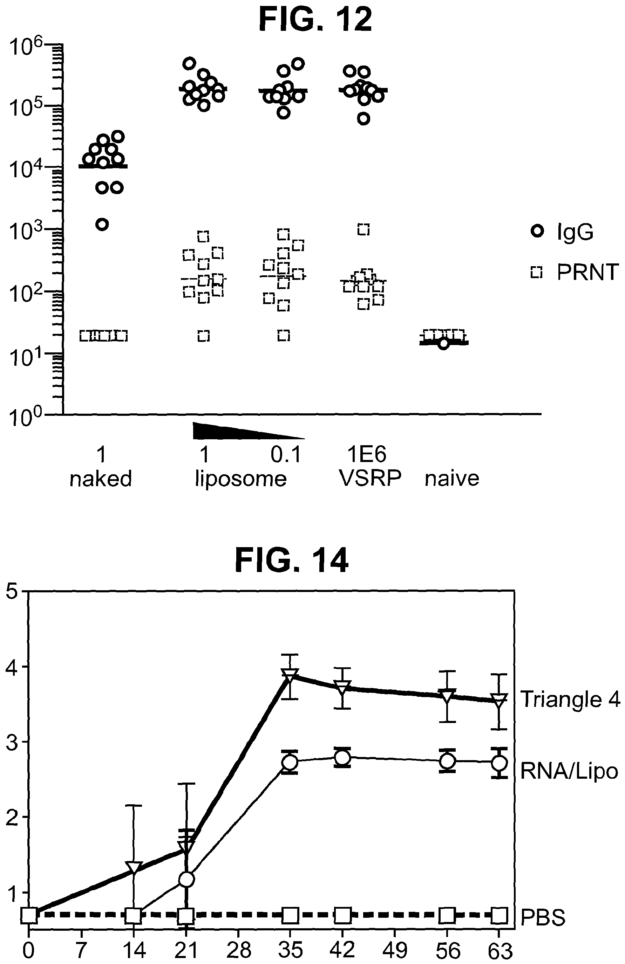

To assess *in vivo* immunogenicity a replicon was constructed to express full-length F protein from respiratory syncytial virus (RSV). This was delivered naked (1 µg), encapsulated in liposomes (0.1 or 1µg), or packaged in virions (10⁶ IU; "VRP") at days 0 and 21. FIG. 7 shows anti-F IgG titers 2 weeks after the second dose, and the liposomes clearly enhance immunogenicity. FIG. 8 shows titers 2 weeks later, by which point there was no statistical difference between the encapsulated RNA at 0.1µg, the encapsulated RNA at 1µg, or the VRP group. Neutralisation titers (measured as 60% plaque reduction, "PRNT60") were not significantly different in these three groups 2 weeks after the second dose (FIG. 9). FIG. 12 shows both IgG and PRNT titers 4 weeks after the second dose.

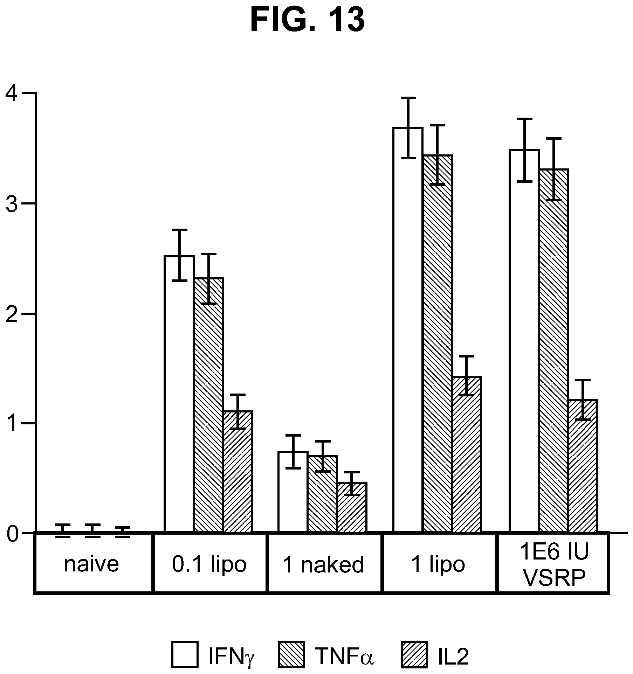

FIG. 13 confirms that the RNA elicits a robust CD8 T cell response.

Further experiments compared F-specific IgG titers in mice receiving VRP, 0.1µg liposome-encapsulated RNA, or 1µg liposome-encapsulated RNA. Titer ratios (VRP: liposome) at various times after the second dose were as follows:

| | **2 weeks** | **4 weeks** | **8 weeks** |
|---|---|---|---|
| **0.1µg** | 2.9 | 1.0 | 1.1 |
| **1µg** | 2.3 | 0.9 | 0.9 |

Thus the liposome-encapsulated RNA induces essentially the same magnitude of immune response as seen with virion delivery.

Further experiments showed superior F-specific IgG responses with a 10µg dose, equivalent responses for 1µg and 0.1µg doses, and a lower response with a 0.01µg dose. FIG. 11 shows IgG titers in mice receiving the replicon in naked form at 3 different doses, in liposomes at 4 different doses, or as VRP (10⁶ IU). The response seen with 1µg liposome-encapsulated RNA was statistically insignificant (ANOVA) when compared to VRP, but the higher response seen with 10µg liposome-encapsulated RNA was statistically significant (p<0.05) when compared to both of these groups.

A further study confirmed that the 0.1µg of liposome-encapsulated RNA gave much higher anti-F IgG responses (15 days post-second dose) than 0.1µg of delivered DNA, and even was more immunogenic than 20µg plasmid DNA encoding the F antigen, delivered by electroporation (Elgen™ DNA Delivery System, Inovio).

A further study was performed in cotton rats (*Sigmodon hispidis*) instead of mice. At a 1µg dose liposome encapsulation increased F-specific IgG titers by 8.3-fold compared to naked RNA and increased neutralisation titers (measured as PRNT60) by 9.5-fold. The magnitude of the antibody response was equivalent to that induced by 5x10⁶ IU VRP. Both naked and liposome-encapsulated RNA were able to protect the cotton rats from RSV challenge (1x10⁵ plaque forming units), reducing lung viral load by at least 3.5 logs. Encapsulation increased the reduction by about 2-fold.

A large-animal study was performed in cattle. Cows were immunised with 66µg of replicon encoding full-length RSV F protein at days 0 and 21, formulated inside liposomes. PBS alone was used as a negative control, and a licensed vaccine was used as a positive control ("Triangle 4" from Fort Dodge, containing killed virus). FIG. 14 shows F-specific IgG titers over a 63 day period starting from the first immunisation. The RNA replicon was immunogenic in the cows, although it gave lower titers than the licensed vaccine. All vaccinated cows showed F-specific antibodies after the second dose, and titers were very stable from the period of 2 to 6 weeks after the second dose (and were particularly stable for the RNA vaccine).

### Encapsulation in liposomes using alternative cationic lipids

As an alternative to using DlinDMA, the cationic lipids of reference 8 are used. These lipids can be synthesised as disclosed in reference 8.

The liposomes formed above using DlinDMA are referred to hereafter as the "RV01" series. The DlinDMA was replaced with various cationic lipids in series "RV02" to "RV12" as described below. Two different types of each liposome were formed, using 2% PEG2000-DMG with either (01) 40% of the cationic lipid, 10% DSPC, and 48% cholesterol, or (02) 60% of the cationic lipid and 38% cholesterol. Thus a comparison of the (01) and (02) liposomes shows the effect of the neutral zwitterionic lipid.

RV02 liposomes were made using the following cationic lipid:

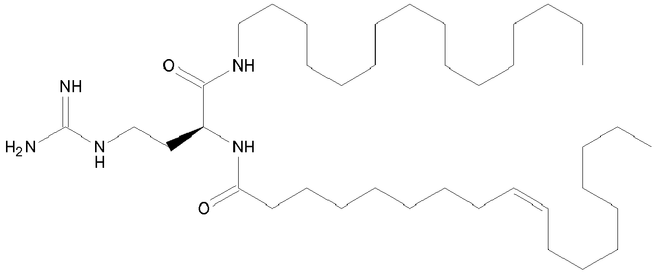

RV03 liposomes were made using the following cationic lipid:

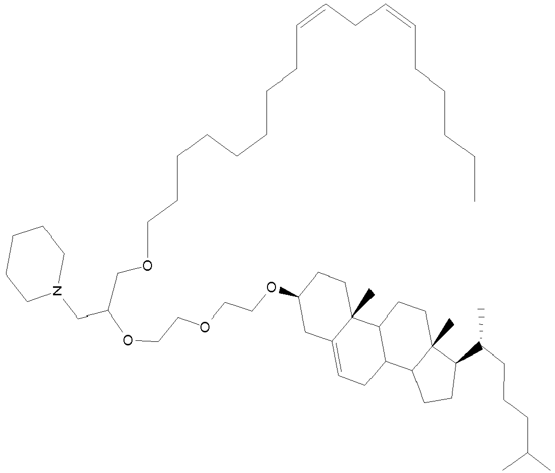

RV04 liposomes were made using the following cationic lipid:

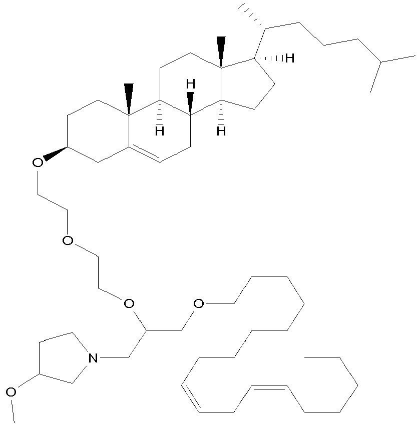

RV05 liposomes were made using the following cationic lipid:

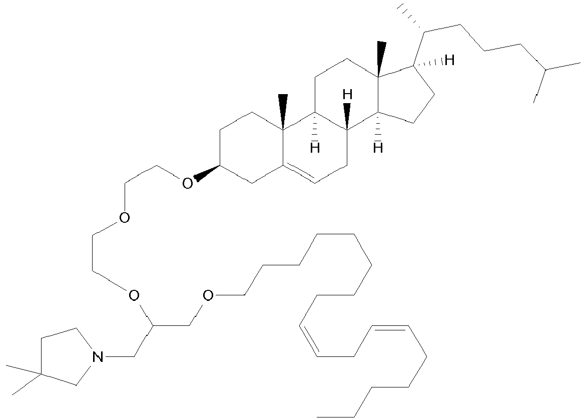

RV06 liposomes were made using the following cationic lipid:

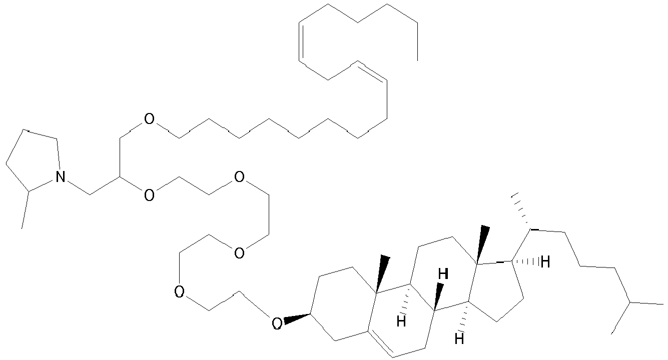

RV07 liposomes were made using the following cationic lipid:

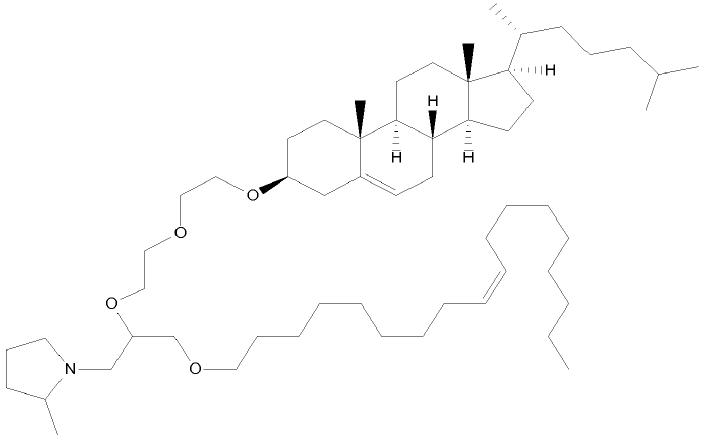

RV08 liposomes were made using the following cationic lipid:

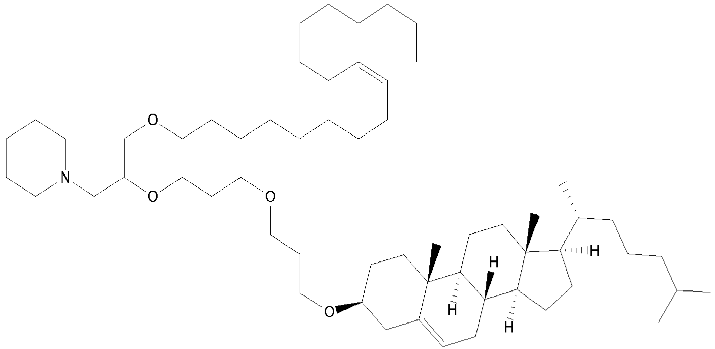

RV09 liposomes were made using the following cationic lipid:

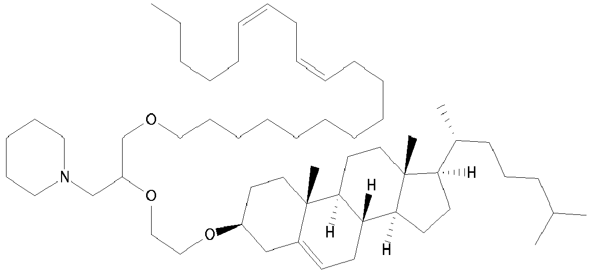

RV10 liposomes were made using the following cationic lipid:

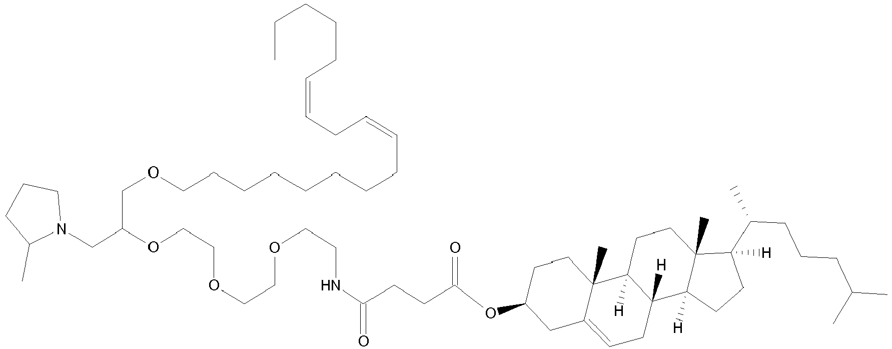

RV11 liposomes were made using the following cationic lipid:

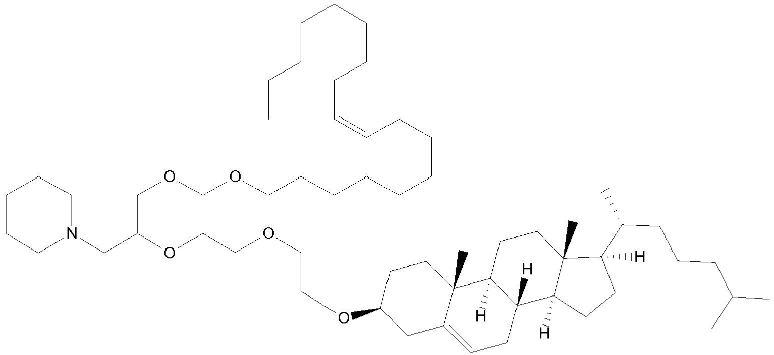

RV12 liposomes were made using the following cationic lipid:

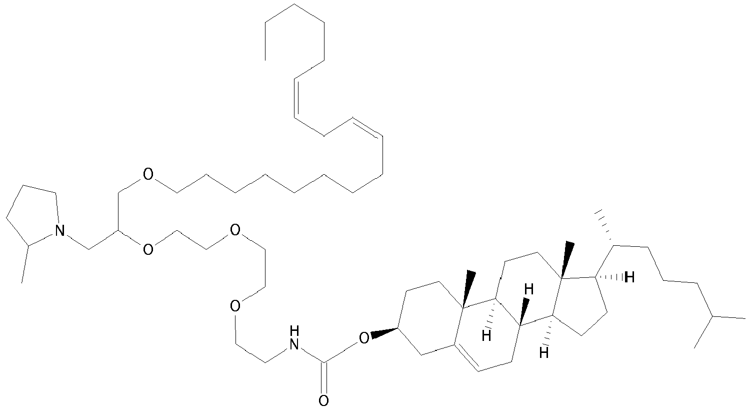

RV13 liposomes were made using the following cationic lipid (DOTAP, for comparative purposes):

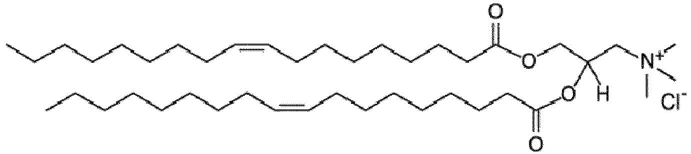

RV14 liposomes were made using the following cationic lipid (DC-cholesterol, for comparison):

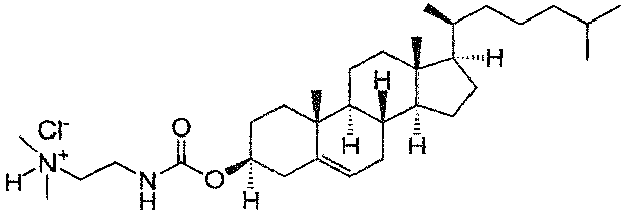

RV15 liposomes were made using the following cationic lipid:

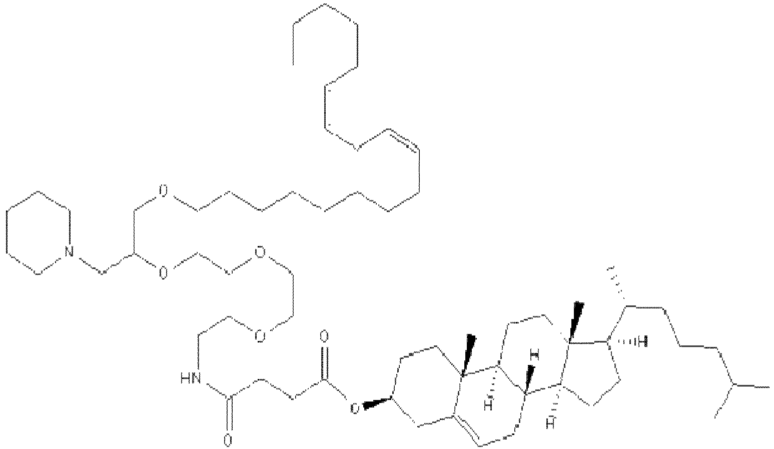

These liposomes were tested with the SEAP reporter described above. The following table shows the size of the liposomes (Z average and polydispersity index), the % of RNA encapsulation in each liposome, together with the SEAP activity detected at days 1 and 6 after injection. SEAP activity is relative to "RV01(02)" liposomes made from DlinDMA, cholesterol and PEG-DMG:

| **RV** | **Zav (pdI)** | **% encapsulation** | **SEAP day 1** | **SEAP day 6** |
|---|---|---|---|---|
| RV01 (01) | 154.6 (0.131) | 95.5 | 80.9 | 71.1 |
| RV01 (02) | 162.0 (0.134) | 85.3 | 100 | 100 |
| RV02 (01) | 133.9 (0.185) | 96.5 | 57 | 45.7 |
| RV02 (02) | 134.6 (0.082) | 97.6 | 54.2 | 4.3 |
| RV03 (01) | 158.3 (0.212) | 62.0 | 65.7 | 44.9 |
| RV03 (02) | 164.2 (0.145) | 86 | 62.2 | 39.7 |
| RV04 (01) | 131.0 (0.145) | 74.0 | 91 | 154.8 |
| RV04 (02) | 134.6 (0.117) | 81.5 | 90.4 | 142.6 |
| RV05 (01) | 164.0 (0.162) | 76.0 | 76.9 | 329.8 |
| RV05 (02) | 177.8 (0.117) | 72.8 | 67.1 | 227.9 |
| RV06 (01) | 116.0 (0.180) | 79.8 | 25.5 | 12.4 |
| RV06 (02) | 136.3 (0.164) | 74.9 | 24.8 | 23.1 |
| RV07 (01) | 140.6 (0.184) | 77 | 26.5 | 163.3 |
| RV07 (02) | 138.6 (0.122) | 87 | 29.7 | 74.8 |
| RV 08 (01) | 176.7 (0.185) | 50 | 76.5 | 187 |
| RV08 (02) | 199.5 (0.191) | 46.3 | 82.4 | 329.8 |
| RV09 (01) | 165.3 (0.169) | 72.2 | 65.1 | 453.9 |
| RV09 (02) | 179.5 (0.157) | 65 | 68.5 | 658.2 |
| RV10(01) | 129.7 (0.184) | 78.4 | 113.4 | 47.8 |
| RV10 (02) | 147.6 (0.131) | 80.9 | 78.2 | 10.4 |
| RV11 (01) | 129.2 (0.186) | 71 | 113.6 | 242.2 |
| RV11 (02) | 139 (0198) | 75.2 | 71.8 | 187.2 |
| RV12 (01) | 135.7 (0.161) | 78.8 | 65 | 10 |
| RV12 (02) | 158.3 (0.287) | 69.4 | 78.8 | 8.2 |

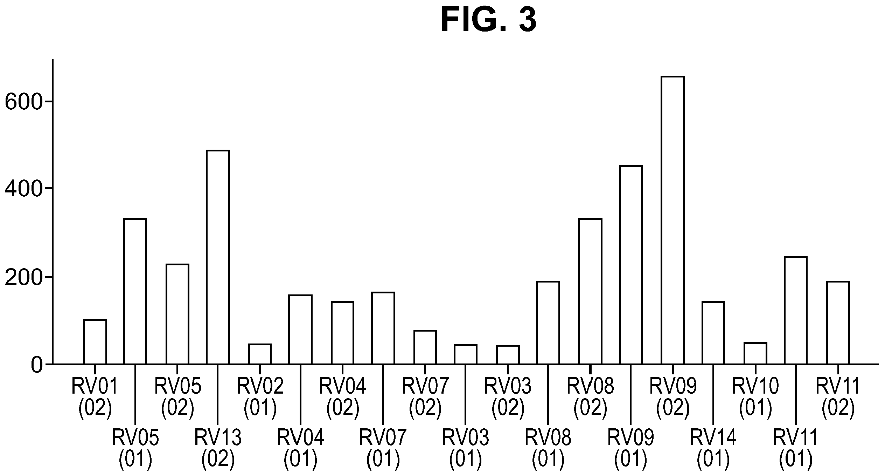

FIG. 3 illustrates the SEAP expression levels seen at day 6. The best results were seen with RV04, RV05, RV07, RV08, RV09, and RV11.

Various of these liposomes were also used to deliver a replicon encoding full-length RSV F protein. One study compared RV01, RV05 and RV13; the highest F-specific serum IgG titers were seen with RV01 and the lowest with RV13. Another study compared RV01, RV02, RV04 and RV07; the best results were again seen with RV01,with RV07 performing poorly. Another study compared RV01, RV03, RV08, RV09 and RV14; the best results were again seen with RV01, with RV03 and RV14 performing poorly. Another study compared RV01, RV10, RV11 and RV15; the best results were again seen with RV01. Overall, the best results were seen with RV01, RV05, RV08 and RV09, whereas RV13 (DOTAP) and RV14 (DC-cholesterol) were poor.

Thus not all of the liposomes were effective for eliciting immune responses. In general, though, it was observed that the best immunological efficacy was seen when the cationic lipid in the liposomes had a pKa in the range of 5.0 to 7.6, and particularly in the range 5.5 to 6.7, between 5.6 and 6.3, between 5.6 and 6.0, or between 5.7 and 5.9.

### BHK expression

Liposomes with different lipids were incubated with BHK cells overnight and assessed for protein expression potency. From a baseline with RV05 lipid, expression could be increased 18x by adding 10% 1,2-diphytanoyl-sn-glycero-3-phosphoethanolamine (DPyPE) to the liposome or 10x by adding 10% 18:2 (cis) phosphatidylcholine. In general, *in vivo* studies showed that unsaturated lipid tails tend to enhance IgG titers raised against encoded antigens.

### RSV immunogenicity

The vA317 self-replicating replicon encoding RSV F protein was administered to BALB/c mice, 4 or 8 animals per group, by bilateral intramuscular vaccinations (50 µL per leg) on days 0 and 21 with the replicon (1µg) alone or formulated as liposomes with RV05 or (for comparison) with RV01 or RV13. The RV01 liposomes had 40% DlinDMA, 10% DSPC, 48% cholesterol and 2% PEG-DMG, but with differing amounts of RNA. The RV05 liposomes had either 40% RV05, 10% DSPC, 48% cholesterol and 2% PEG-DMG or 60% RV05, 38% cholesterol and 2% PEG-DMG. The RV13 liposomes had 40% DOTAP, 10% DOPE, 48% cholesterol and 2% PEG-DMG. The liposomes were prepared using various techniques. For comparison, naked plasmid DNA (20 µg) expressing the same RSV-F antigen was delivered either using electroporation or with RV01(10) liposomes (0.1µg DNA). Four mice were used as a naive control group.

Z average particle diameter and polydispersity index were:

| **RV** | **Zav (nm)** | **pdI** | **Preparation** |
|---|---|---|---|
| RV01 (10) | 158.6 | 0.088 | (A) |
| RV01 (08) | 156.8 | 0.144 | (A) |
| RV01 (05) | 136.5 | 0.136 | (B) |
| RV01 (09) | 153.2 | 0.067 | (A) |
| RV01 (10) | 134.7 | 0.147 | (A) |
| RV05 (01) | 148 | 0.127 | (A) |
| RV05 (02) | 177.2 | 0.136 | (A) |
| RV13 (02) | 128.3 | 0.179 | (A) |

Serum was collected for antibody analysis on days 14, 36 and 49. Spleens were harvested from mice at day 49 for T cell analysis.

F-specific serum IgG titers (GMT) were as follows:

| **RV** | **Day 14** | **Day 36** |
|---|---|---|
| Naked DNA plasmid | 439 | 6712 |
| Naked A317 RNA | 78 | 2291 |
| RV01 (10) | 3020 | 26170 |
| RV01 (08) | 2326 | 9720 |
| RV01 (05) | 5352 | 54907 |
| RV01 (09) | 4428 | 51316 |
| RV05 (01) | 1356 | 5346 |
| RV05 (02) | 961 | 6915 |
| RV01 (10) DNA | 5 | 13 |
| RV13 (02) | 644 | 3616 |

The proportion of T cells which are cytokine-positive and specific for RSV F51-66 peptide are as follows, showing only figures which are statistically significantly above zero:

| **RV** | **CD4+CD8-** | | | | **CD4-CD8+** | | | |
|---|---|---|---|---|---|---|---|---|
| | IFNγ | IL2 | IL5 | TNFα | IFNγ | IL2 | IL5 | TNFα |
| Naked DNA plasmid | 0.04 | 0.07 | | 0.10 | 0.57 | 0.29 | | 0.66 |
| Naked A317 RNA | 0.04 | 0.05 | | 0.08 | 0.57 | 0.23 | | 0.67 |
| RV01 (10) | 0.07 | 0.10 | | 0.13 | 1.30 | 0.59 | | 1.32 |
| RV01 (08) | 0.02 | 0.04 | | 0.06 | 0.46 | 0.30 | | 0.51 |
| RV01 (05) | 0.08 | 0.12 | | 0.15 | 1.90 | 0.68 | | 1.94 |
| RV01 (09) | 0.06 | 0.08 | | 0.09 | 1.62 | 0.67 | | 1.71 |
| RV05 (01) | | | | | 0.06 | 0.04 | | 0.19 |
| RV05 (02) | 0.05 | 0.07 | | 0.11 | 0.64 | 0.35 | | 0.69 |
| RV01 (10) DNA | | | | 0.03 | | | | 0.08 |
| RV13 (02) | 0.03 | 0.04 | | 0.06 | 1.15 | 0.41 | | 1.18 |

Thus the liposome formulations significantly enhanced immunogenicity relative to the naked RNA controls, as determined by increased F-specific IgG titers and T cell frequencies. Plasmid DNA formulated with liposomes, or delivered naked using electroporation, was significantly less immunogenic than liposome-formulated self-replicating RNA.

### RSVimmunogenicity in different mouse strains

Replicon "vA142" encodes the full-length wild type surface fusion (F) glycoprotein of RSV but with the fusion peptide deleted, and the 3' end is formed by ribozyme-mediated cleavage. It was tested in three different mouse strains.

BALB/c mice were given bilateral intramuscular vaccinations (50 µL per leg) on days 0 and 22. Animals were divided into 8 test groups (5 animals per group) and a naive control (2 animals):
Group 1 were given naked replicon (1 µg).
Group 2 were given 1µg replicon delivered in liposomes "RV01(37)" with 40% DlinDMA, 10% DSPC, 48% Chol, 2% PEG-conjugated DMG.
Group 3 were given the same as group 2, but at 0.1µg RNA.
Group 4 were 1µg replicon in "RV05(11)" liposomes (40% RV05 lipid, 30% 18:2 PE (DLoPE, 28% cholesterol, 2% PEG-DMG).
Group 5 were given 5µg RSV-F subunit protein adjuvanted with aluminium hydroxide.
Group 6 were a naive control (2 animals)

Sera were collected for antibody analysis on days 14, 35 and 49. F-specific serum IgG GMTs were:

| **Day** | **1** | **2** | **3** | **4** | **5** | **6** |
|---|---|---|---|---|---|---|
| **14** | 82 | 2463 | 1789 | 1171 | 1293 | 5 |
| **35** | 1538 | 34181 | 25605 | 13718 | 73809 | 5 |

At day 35 F-specific IgG1 and IgG2a titers (GMT) were as follows:

| **IgG** | **1** | **2** | **3** | **4** | **5** |
|---|---|---|---|---|---|
| **IgG1** | 94 | 6238 | 4836 | 8288 | 78604 |
| **IgG2a** | 5386 | 77064 | 59084 | 14437 | 24 |

RSV serum neutralizing antibody titers at days 35 and 49 were as follows (data are 60% plaque reduction neutralization titers of pools of 2-5 mice, 1 pool per group):

| **Day** | **1** | **2** | **3** | **4** | **5** | **6** |
|---|---|---|---|---|---|---|
| **35** | <20 | 143 | 20 | 32 | 111 | <20 |
| **49** | <20 | 139 | <20 | 41 | 1009 | <20 |

Spleens were harvested at day 49 for T cell analysis. Average net F-specific cytokine-positive T cell frequencies (CD4+ or CD8+) were as follows, showing only figures which were statistically significantly above zero (specific for RSV peptides F51-66, F164-178, F309-323 for CD4+, or for peptides F85-93 and F249-258 for CD8+):

| **Group** | **CD4+CD8-** | | | | **CD4-CD8+** | | | |
|---|---|---|---|---|---|---|---|---|
| | IFNγ | IL2 | IL5 | TNFα | IFNγ | IL2 | IL5 | TNFα |
| 1 | 0.03 | 0.06 | | 0.08 | 0.47 | 0.29 | | 0.48 |
| 2 | 0.05 | 0.10 | | 0.08 | 1.35 | 0.52 | | 1.11 |
| 3 | 0.03 | 0.07 | | 0.06 | 0.64 | 0.31 | | 0.61 |
| 4 | 0.03 | 0.08 | | 0.07 | 0.65 | 0.28 | | 0.58 |
| 5 | | 0.02 | | | 0.04 | 0.04 | | |
| 6 | | | | | | | | |

C57BL/6 mice were immunised in the same way, but a 7th group received VRPs (1x10⁶ IU) expressing the full-length wild-type surface fusion glycoprotein of RSV (fusion peptide deletion).

Sera were collected for antibody analysis on days 14, 35 & 49. F-specific IgG titers (GMT) were:

| **Day** | **1** | **2** | **3** | **4** | **5** | **6** | **7** |
|---|---|---|---|---|---|---|---|
| **14** | 1140 | 2133 | 1026 | 3045 | 2975 | 5 | 1101 |
| **35** | 1721 | 5532 | 3184 | 9525 | 39251 | 5 | 12139 |

At day 35 F-specific IgG1 and IgG2a titers (GMT) were as follows:

| **IgG** | **1** | **2** | **3** | **4** | **5** | **6** |
|---|---|---|---|---|---|---|
| **IgG1** | 66 | 247 | 14 | 468 | 56258 | 79 |
| **IgG2a** | 2170 | 7685 | 5055 | 1573 | 35 | 14229 |

RSV serum neutralizing antibody titers at days 35 and 49 were as follows (data are 60% plaque reduction neutralization titers of pools of 2-5 mice, 1 pool per group):

| **Day** | **1** | **2** | **3** | **4** | **5** | **6** | **7** |
|---|---|---|---|---|---|---|---|
| **35** | <20 | 27 | 29 | 36 | 28 | <20 | <20 |
| **49** | <20 | 44 | 30 | 36 | 33 | <20 | 37 |

Spleens were harvested at day 49 for T cell analysis. Average net F-specific cytokine-positive T cell frequencies (CD8+) were as follows, showing only figures which were statistically significantly above zero (specific for RSV peptides F85-93 and F249-258):

| **Group** | **CD4-CD8+** | | | |
|---|---|---|---|---|
| | IFNγ | IL2 | IL5 | TNFα |
| 1 | 0.42 | 0.13 | | 0.37 |
| 2 | 1.21 | 0.37 | | 1.02 |
| 3 | 1.01 | 0.26 | | 0.77 |
| 4 | 2.13 | 0.70 | | 1.77 |
| 5 | 0.10 | 0.05 | | |
| 6 | | | | |
| 7 | 2.83 | 0.72 | | 2.26 |

Nine groups of C3H/HeN mice were immunised in the same way. F-specific IgG titers (GMT) were:

| **Day** | **1** | **2** | **3** | **4** | **5** | **6** | **7** |
|---|---|---|---|---|---|---|---|
| **14** | 5 | 2049 | 1666 | 298 | 3519 | 5 | 806 |
| **35** | 152 | 27754 | 19008 | 3424 | 62297 | 5 | 17249 |

At day 35 F-specific IgG1 and IgG2a titers (GMT) were as follows:

| **IgG** | **1** | **2** | **3** | **4** | **5** | **6** |
|---|---|---|---|---|---|---|
| **IgG1** | 5 | 1323 | 170 | 136 | 83114 | 189 |
| **IgG2a** | 302 | 136941 | 78424 | 15667 | 3800 | 72727 |

RSV serum neutralizing antibody titers at days 35 and 49 were as follows:

| **Day** | **1** | **2** | **3** | **4** | **5** | **6** | **7** |
|---|---|---|---|---|---|---|---|
| **35** | <20 | 539 | 260 | 101 | 443 | <20 | 595 |
| **49** | <20 | 456 | 296 | 82 | 1148 | <20 | 387 |

Thus the different lipids (RV01 & RV05; pKa 5.8 & 5.85) were tested in three different inbred mouse strains. For BALB/c and C3H strains RV05 was less effective than RV01, but it was more effective in B6 strain. In all cases, however, the liposomes were more effective than two cationic nanoemulsions which were tested in parallel.

### Cotton rats

The vA142 replicon was also tested in cotton rats using liposomes formed from:
(a) 40% DlinDMA, 10% DPSC, 48% cholesterol and 2% PEG DMG 2000.
(b) 40% RV05, 30% DLoPE (18:2 PE), 28% cholesterol and 2% PEG DMG 2000.

Cotton rats, 4-8 animals per group, were given intramuscular vaccinations (100 µL in one leg) on days 0 and 21 with:
**Group 1** self-replicating RNA (vA142, 0.1 µg, RSV-F) formulated in liposomes (a)
**Group 2** self-replicating RNA (vA142, 0.1 µg, RSV-F) formulated in liposomes (b)
**Group 3** self-replicating RNA (vA142, 1 µg, RSV-F) formulated in liposomes (a)
**Group 4** self-replicating RNA (vA142, 1 µg, RSV-F) formulated in liposomes (b)
**Group 5** VRPs (1x10⁶ IU) expressing the full-length wild type surface F glycoprotein of RSV
**Group 6** RSV-F subunit protein vaccine (5 µg) adjuvanted with aluminium hydroxide
**Group 7** a naive control (3 animals)

All cotton rats (except group 7) were vaccinated with 5 µg F subunit + aluminium hydroxide on day 49 (four weeks after the second vaccination).

Serum was collected for antibody analysis on days 0, 21, 35, 49, 64.

F-specific serum IgG titers (GMT) were as follows:

| **Group** | **Day 21** | **Day 35** | **Day 49** | **Day 64** |
|---|---|---|---|---|
| 1 | 112 | 1403 | 943 | 15123 |
| 2 | 49 | 1008 | 513 | 15308 |
| 3 | 558 | 3938 | 2383 | 16563 |
| 4 | 342 | 3207 | 2151 | 24494 |
| 5 | 1555 | 7448 | 4023 | 25777 |
| 6 | 8425 | 81297 | 54776 | 82911 |
| 7 | 5 | 5 | 5 | 5 |

RSV serum neutralizing antibody titers were as follows:

| **Group** | **Day 21** | **Day 35** | **Day 49** | **Day 64** |
|---|---|---|---|---|
| 1 | 26 | 162 | 58 | 1772 |
| 2 | 27 | 371 | 163 | 2449 |
| 3 | 66 | 788 | 306 | 161 |
| 4 | 75 | 448 | 201 | 5733 |
| 5 | 137 | 2879 | 1029 | 1920 |
| 6 | 307 | 2570 | 1124 | 2897 |
| 7 | 10 | - | - | 10 |

Thus cotton rats were vaccinated with vA142 replicon formulated with RV01 or RV05, and the replicon was given at two doses (1.0 and 0.1 µg). After the first replicon vaccination F-specific serum IgG titers were higher with RV01 than RV05, but neutralization titers were approximately equal. Titers in all groups were boosted by a homologous second vaccination given on day 21. After the second replicon vaccination, F-specific serum IgG titers were again higher with RV01 than with RV05, and RSV neutralization titers generally followed this same trend.

The protein vaccination at day 49 did not boost antibody titers in cotton rats previously vaccinated with protein, but it provided a large boost to titers in cotton rats previously vaccinated with RNA. The titers (total IgG and neutralization) were higher at day 64 using RV05 than when using RV01.

### Different cationic lipids with vA317 RSV replicon

Further experiments compared four different cationic lipids (RV01, RV02, RV04 & RV07). All liposomes contained 2% PEG-DMG 2000 but remaining lipid compositions varied. The compositions and physical characteristics were as follows:

| **Name** | **Lipid 1** | **Other lipids** | **Zav diam (nm)** | **pdI** | **% encap"** |
|---|---|---|---|---|---|
| A | RV01,40% | 10% DSPC, 48% cholesterol | 158.6 | 0.088 | 90.7 |
| B | RV02, 40% | 10% DSPC, 48% cholesterol | 146.8 | 0.084 | 97.5 |
| C | RV04, 40% | 10% DSPC, 48% cholesterol | 136.7 | 0.165 | 67.3 |
| D | RV04, 60% | 38% cholesterol | 176.3 | 0.157 | 55.2 |
| E | RV07, 40% | 10% DSPC, 48% cholesterol | 144.9 | 0.204 | 82 |
| F | RV07, 60% | 38% cholesterol | 124.1 | 0.195 | 80 |

For comparison of immunogenicity, HT, SUV and MLV liposomes were also made with RV01, using the same components at the same proportions, but with manufacturing methods which are non-scalable (but are quicker). Briefly, an ethanol stock solution was created containing 37mg/ml DLinDMA, 12mg/ml DSPC, 28mg/ml cholesterol, and 8 mg/ml of PEG DMG 2000. 100µl of the stock solution was diluted in a total of 1ml of ethanol. Liposomes were prepared by evaporating the ethanol solution using a rotary evaporator at 150 milliTorr, pressure for 30 minutes at 50°C. Residual ethanol evaporation was insured by placing the samples overnight under vacuum in a freeze dryer. The lipid film was hydrated and dispersed by adding 1.0 mL of filtered deionized water and placed at 50°C to ensure full suspension of the lipids into MLVs. An aliquot was removed from the MLVs and sonicated with a probe sonicator with a 1 second pulse for 5 minutes at 100% power fo form the SUVs. Both of the resulting solutions were complexed with replicon RNA. The HT liposomes were made using an ethanol stock solution containing 37mg/ml DLinDMA, 12mg/ml DSPC, 28mg/ml cholesterol, and 8 mg/ml of PEG DMG 2000. 100µl of the stock solution was diluted to 400µl with ethanol. The resulting ethanol solution was added drop wise to 600µl of 10mM citrate buffer at pH 6.5 containing 40µg of RNA under constant stirring. The resulting solution was dialyzed overnight against 4L of PBS buffer using a 10,000 MWCO dialysis membrane.

BALB/c mice, 8 per group, were given bilateral intramuscular vaccinations (50 µL per leg) on days 0 and 21 with naked replicon (1µg) or 0.1µg encapsulated RNA. F-specific serum IgG titers (GMT) 2 weeks after these two injections were as follows:

| **Liposomes** | **Day 14** | **Day 35** |
|---|---|---|
| Naked A317 RNA | 111 | 469 |
| A | 1834 | 30519 |
| B | 1050 | 5681 |
| C | 430 | 4127 |
| D | 779 | 4693 |
| E | 586 | 6424 |
| F | 121 | 2568 |
| HT | 3878 | 19982 |
| MLV | 1381 | 49480 |
| SUV | 4158 | 37526 |

For RV07 the absence of DSPC caused a large decrease in immunogenicity.

Further lipids (RV01, RV03, RV08, RV09, RV14) were tested in the same way:

| **Name** | **Lipid 1** | **Other lipids** | **Zav diam (nm)** | **pdI** | **% encapⁿ** |
|---|---|---|---|---|---|
| G | RV01, 40% | 10% DSPC, 48% cholesterol | 158.6 | 0.088 | 90.7 |
| H | RV03, 40% | 10% DSPC, 48% cholesterol | 150.3 | 0.188 | 83.1 |
| I | RV03, 60% | 38% cholesterol | 161.1 | 0.239 | 68.4 |
| J | RV08, 40% | 10% DSPC, 48% cholesterol | 191.1 | 0.227 | 51.7 |
| K | RV08, 60% | 38% cholesterol | 214.2 | 0.208 | 43.1 |
| L | RV09, 40% | 10% DSPC, 48% cholesterol | 161.6 | 0.209 | 64.5 |
| M | RV09, 60% | 38% cholesterol | 170.7 | 0.121 | 82.4 |
| N | RV14,60% | 30% DSPC | 155.5 | 0.238 | 63.3 |
| O | RV01, 40% | 10% DSPC, 48% cholesterol | 96.14 | 0.087 | 92 |

| **Liposomes** | **Day 14** | **Day 35** |
|---|---|---|
| Naked A317 RNA | 35 | 457 |
| G | 2421 | 10757 |
| H | 15 | 52 |
| I | 16 | 85 |
| J | 991 | 1921 |
| K | 7 | 610 |
| L | 1082 | 1421 |
| M | 146 | 286 |
| N | 27 | 212 |
| O | 4695 | 19773 |

Liposome N (with DC-cholesterol) performed poorly, even below the naked RNA control. In contrast, the remaining cationic lipids gave useful results. Liposome O was prepared by a different mixing method (microfluidic chip) from liposome G and this smaller liposome gave better results with approximately the same encapsulation.

Further lipids (RV01, RV10, RV11, RV15) were tested in the same way:

| **Name** | **Lipid 1** | **Other lipids** | **Zav diam (nm)** | **pdI** | **% encapⁿ** |
|---|---|---|---|---|---|
| P | RV01, 40% | 10% DSPC, 48% cholesterol | 158.6 | 0.088 | 90.7 |
| Q | RV10, 40% | 10% DSPC, 48% cholesterol | 123.6 | 0.14 | 80.3 |
| R | RV11, 40% | 10% DSPC, 48% cholesterol | 137.1 | 0.155 | 81 |
| S | RV11,60% | 38% cholesterol | 135.4 | 0.175 | 79.7 |
| T | RV15, 40% | 38% cholesterol | 111 | 0.167 | 76.4 |

| **Liposomes** | **Day 14** | **Day 35** |
|---|---|---|
| Naked A317 RNA | 185 | 982 |
| P | 2787 | 27416 |
| Q | 24 | 161 |
| R | 633 | 1715 |
| S | 405 | 2733 |
| T | 761 | 2459 |

Except for liposome Q each of these liposomes performed better than the control. The RV10 lipid in liposome Q has a pKa of 7.86 which seems too high to be useful *in vivo.* Even inside the useful pKa range of 5.0 to 7.6, however, although results were good, none of the lipids with one alkyl tail and one steroid-containing tail gave results as good as RV01.

Further liposomes were made with RV05. The liposomes all had 40% RV05 and 2% PEGylated lipid, but the remaining components varied (although cholesterol was always included). Physical characteristics were:

| **Name** | **PEGylated lipid** | **Other components** | **Zav (nm)** | **pdI** | **% encapsulⁿ** |
|---|---|---|---|---|---|
| U | DMG | 10% DSPC, 48% chol | 102.2 | 0.12 | 76.81 |
| V | Cholesterol | 10% DSPC, 46% chol, 2% αGC | 103.7 | 0.107 | 72.58 |
| W | DMG | 10% DPyPE, 48% chol | 99.6 | 0.115 | 78.34 |
| X | DMG | 10% 18:3 PC, 48% chol | 130 | 0.14 | 87.92 |
| Y | DMG | 10% 18:2 PC, 48% chol | 101.1 | 0.133 | 76.64 |
| Z | DMG | 30% 18:2 PC, 28% chol | 134.3 | 0.158 | 57.76 |

| | | | | | |
|---|---|---|---|---|---|
| αGC = α-galactosylceramide | | | | | |

BALB/c mice were tested as before:

| **Injection** | **Day 14** | **Day 35** |
|---|---|---|
| Naked RNA | 321 | 915 |
| U | 551 | 955 |
| V | 342 | 2531 |
| W | 1127 | 3881 |
| X | 364 | 1741 |
| Y | 567 | 5679 |
| Z | 1251 | 5303 |

For a cationic lipid with an asymmetrical lipid tails (alkyl + cholesterol), changing the neutral lipid from DSPC (saturated C18 lipid tail) to 18:2 or 18:3 PC (with 2 and 3 unsaturated double bonds per tail) increased total IgG titers. Comparable results were observed by replacing DSPC with DPyPE.

In a final experiment with the RV05 lipid a liposome was made with 40% RV05, 10% 18:2 PC, 40% DPyPE, 8% cholesterol and 2% PEG DMG 2000. These liposomes had a Zav diameter of 124.7nm, a pdI of 0.17 and a RNA encapsulation of 61.5%. They were used to vaccinate BALB/c mice as before (0.1µg RNA dose), in comparison with naked RNA (1µg) or with RV01-based liposomes (40% DlinDMA, 10% DPSC, 48% cholesterol, 2% PEG DMG 2000). F-specific serum IgG titers (GMT) were as follows:

| **Group** | **Day 14** | **Day 35** |
|---|---|---|
| Naked RNA | 28 | 721 |
| RV01 | 2237 | 12407 |
| RV05 | 703 | 1732 |

Thus the RV05 liposomes were more immunogenic than naked RNA, but less immunogenic than RV01 liposomes.

Spleens were harvested at day 49 for T cell analysis. Average net F-specific cytokine-positive T cell frequencies (CD4+ or CD8+) were as follows, showing only figures which were statistically significantly above zero (specific for RSV peptides F51-66, F164-178, F309-323 for CD4+, or for peptides F85-93 and F249-258 for CD8+):

| **Group** | **CD4-CD8+** | | | | **CD4-CD8+** | | | |
|---|---|---|---|---|---|---|---|---|
| | **IFNγ** | **IL2** | **IL5** | **TNFα** | **IFNγ** | **IL2** | **IL5** | **TNFα** |
| Naked | 0.02 | 0.02 | 0.04 | | 0.36 | 0.16 | | 0.28 |
| RV01 | 0.03 | 0.03 | 0.04 | | 0.66 | 0.17 | | 0.56 |
| RV05 | 0.06 | 0.06 | 0.09 | | 0.86 | 0.24 | | 0.69 |

In terms of T cell responses, therefore, RV05 gave better results than RV01.

It will be understood that the invention has been described by way of example only and modifications may be made whilst remaining within the scope and spirit of the invention.

**Table 1: useful phospholipids**

| | |
|---|---|
| DDPC | 1,2-Didecanoyl-sn-Glycero-3-phosphatidylcholine |
| DEPA | 1,2-Dierucoyl-sn-Glycero-3-Phosphate |
| DEPC | 1,2-Erucoyl-sn-Glycero-3-phosphatidylcholine |
| DEPE | 1,2-Dierucoyl-sn-Glycero-3-phosphatidylethanolamine |
| DEPG | 1,2- Dierucoyl-sn-Glycero-3[Phosphatidyl-rac-(1-glycerol...) |
| DLOPC | 1,2-Linoleoyl-sn-Glycero-3-phosphatidylcholine |
| DLPA | 1,2-Dilauroyl-sn-Glycero-3-Phosphate |
| DLPC | 1,2-Dilauroyl-sn-Glycero-3-phosphatidylcholine |
| DLPE | 1,2-Dilauroyl-sn-Glycero-3-phosphatidylethanolamine |
| DLPG | 1,2-Dilauroyl-sn-Glycero-3[Phosphatidyl-rac-(1-glycerol...) |
| DLPS | 1,2-Dilauroyl-sn-Glycero-3-phosphatidylserine |
| DMG | 1,2-Dimyristoyl-sn-glycero-3-phosphoethanolamine |
| DMPA | 1,2-Dimyristoyl-sn-Glycero-3-Phosphate |
| DMPC | 1,2-Dimyristoyl-sn-Glycero-3-phosphatidylcholine |
| DMPE | 1,2-Dimyristoyl-sn-Glycero-3-phosphatidylethanolamine |
| DMPG | 1,2-Myristoyl-sn-Glycero-3[Phosphatidyl-rac-(1-glycerol...) |
| DMPS | 1,2-Dimyristoyl-sn-Glycero-3-phosphatidylserine |
| DOPA | 1,2-Dioleoyl-sn-Glycero-3-Phosphate |
| DOPC | 1,2-Dioleoyl-sn-Glycero-3-phosphatidylcholine |
| DOPE | 1,2-Dioleoyl-sn-Glycero-3-phosphatidylethanolamine |
| DOPG | 1,2-Dioleoyl-sn-Glycero-3[Phosphatidyl-rac-(1-glycerol...) |
| DOPS | 1,2-Dioleoyl-sn-Glycero-3-phosphatidylserine |
| DPPA | 1,2-Dipalmitoyl-sn-Glycero-3-Phosphate |
| DPPC | 1,2-Dipalmitoyl-sn-Glycero-3-phosphatidylcholine |
| DPPE | 1,2-Dipalmitoyl-sn-Glycero-3-phosphatidylethanolamine |
| DPPG | 1,2-Dipalmitoyl-sn-Glycero-3[Phosphatidyl-rac-(1-glycerol...) |
| DPPS | 1,2-Dipalmitoyl-sn-Glycero-3-phosphatidylserine |
| DPyPE | 1,2-diphytanoyl-sn-glycero-3-phosphoethanolamine |
| DSPA | 1,2-Distearoyl-sn-Glycero-3-Phosphate |
| DSPC | 1,2-Distearoyl-sn-Glycero-3-phosphatidylcholine |
| DSPE | 1,2-Diostearpyl-sn-Glycero-3-phosphatidylethanolamine |
| DSPG | 1,2-Distearoyl-sn-Glycero-3[Phosphatidyl-rac-(1-glycerol...) |
| DSPS | 1,2-Distearoyl-sn-Glycero-3-phosphatidylserine |
| EPC | Egg-PC |
| HEPC | Hydrogenated Egg PC |
| HSPC | High purity Hydrogenated Soy PC |
| HSPC | Hydrogenated Soy PC |
| LYSOPC MYRISTIC | 1-Myristoyl-sn-Glycero-3-phosphatidylcholine |
| LYSOPC PALMITIC | 1-Palmitoyl-sn-Glycero-3-phosphatidylcholine |
| LYSOPCSTEARIC | 1-Stearoyl-sn-Glycero-3-phosphatidylcholine |
| Milk Sphingomyelin MPPC | 1-Myristoyl,2-palmitoyl-sn-Glycero 3-phosphatidylcholine |
| MSPC | 1-Myristoyl,2-stearoyl-sn-Glycero-3-phosphatidylcholine |
| PMPC | 1-Palmitoyl,2-myristoyl-sn-Glycero-3-phosphatidylcholine |
| POPC | 1-Palmitoyl,2-oleoyl-sn-Glycero-3-phosphatidylcholine |
| POPE | 1-Palmitoyl-2-oleoyl-sn-Glycero-3-phosphatidylethanolamine |
| POPG | 1,2-Dioleoyl-sn-Glycero-3[Phosphatidyl-rac-(1-glycerol)...] |
| PSPC | 1-Palmitoyl,2-stearoyl-sn-Glycero-3-phosphatidylcholine |
| SMPC | 1-Stearoyl,2-myristoyl-sn-Glycero-3-phosphatidylcholine |
| SOPC | 1-Stearoyl,2-oleoyl-sn-Glycero-3-phosphatidylcholine |
| SPPC | 1-Stearoyl,2-palmitoyl-sn-Glycero-3-phosphatidylcholine |

### REFERENCES

[1] Johanning et al. (1995) Nucleic Acids Res 23:1495-1501.
[2] Heyes et al. (2005) J Controlled Release 107:276-87.
[3] WO2005/121348.
[4] Liposomes: Methods and Protocols, Volume 1: Pharmaceutical Nanocarriers: Methods and Protocols. (ed. Weissig). Humana Press, 2009. ISBN 160327359X.
[5] Liposome Technology, volumes I, II & III. (ed. Gregoriadis). Informa Healthcare, 2006.
*[6]* Functional Polymer Colloids and Microparticles volume 4 (Microspheres, microcapsules & liposomes). (eds. Arshady & Guyot). Citus Books, 2002.
[7] Jeffs et al. (2005) Pharmaceutical Research 22 (3):362-372.
[8] WO2011/076807.
[9] Tarwadi et al. (2008) Bioconjugate Chem. 19:940-950.
[10] WO2005/113782.
[11] WO2011/005799.
[12] El Ouahabi et al. (1996) FEBS Letts 380:108-12.
[13] Giuliani et al. (2006) Proc Natl Acad Sci USA 103(29): 10834-9.
[14] WO2009/016515.
[15] WO02/34771.
[16] WO2005/032582.
[17] WO2010/119343.
[18] WO2006/110413.
[19] WO2005/111066.
[20] WO2005/002619.
[21] WO2006/138004.
[22] WO2009/109860.
[23] WO02/02606.
[24] WO03/018054.
[25] WO2006/091517.
[26] WO2008/020330.
[27] WO2006/089264.
[28] WO2009/104092.
[29] WO2009/031043.
[30] WO2007/049155.
[31] Gennaro (2000) Remington: The Science and Practice of Pharmacy. 20th edition, ISBN: 0683306472.
*[32]* Methods In Enzymology (S. Colowick and N. Kaplan, eds., Academic Press, Inc.)
[33] Handbook of Experimental Immunology, Vols. I-IV (D.M. Weir and C.C. Blackwell, eds, 1986, Blackwell Scientific Publications)
[34] Sambrook et al. (2001) Molecular Cloning: A Laboratory Manual, 3rd edition (Cold Spring Harbor Laboratory Press).
[35] Handbook of Surface and Colloidal Chemistry (Birdi, K.S. ed., CRC Press, 1997)
[36] Ausubel et al. (eds) (2002) Short protocols in molecular biology, 5th edition (Current Protocols).
*[37]* Molecular Biology Techniques: An Intensive Laboratory Course, (Ream et al., eds., 1998, Academic Press)
[38] PCR (Introduction to Biotechniques Series), 2nd ed. (Newton & Graham eds., 1997, Springer Verlag)
[39] Yoneyama & Fujita (2007) Cytokine & Growth Factor Reviews 18:545-51.
[40] Maurer et al. (2001) Biophysical Journal, 80: 2310-2326.
[41] Perri et al. (2003) J Virol 77:10394-10403.

### NUMBERED EMBODIMENTS

1. A liposome within which RNA encoding a polypeptide of interest is encapsulated, wherein the liposome includes at least one compound selected from the group consisting of compounds of formula (I) and formula (XI), where
   *Formula (I) is:* 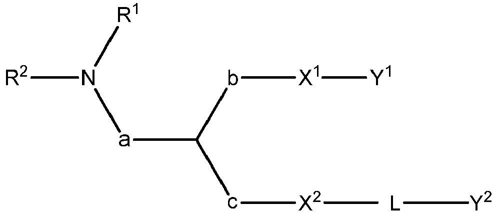 wherein:
   R¹ and R² together with the nitrogen atom to which they are attached form an optionally substituted C₃₋₂₀-heterocycloalkyl, C₃₋₂₀-heterocycloalkenyl, C₃₋₂₀-heterocycloalkynyl or C₅₋₂₀-heteroaryl group;
   a is absent or optionally substituted C₁₋₄ alkylene;
   b is absent or optionally substituted C₁₋₄ alkylene;
   c is absent or optionally substituted C₁₋₄ alkylene;
   X¹ is O or S;
   X² is O or S;
   Y¹ is optionally substituted C₁₀₋₃₀alkenyl, C₁₀₋₃₀alkynyl, C₁₀₋₃₀heteroalkenyl or C₁₀₋₃₀heteroalkynyl;
   L is absent or is -(L^{a})_{d}-(L^{b})ₑ-(L^{c})_{f}-, wherein
      L^{a} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
      L^{b} is optionally substituted C₆₋₁₄arylene or C₅₋₁₃heteroarylene;
      L^{c} is optionally substituted C₁₋₁₅alkylene, C₁₋₁₅alkenylene, C₁₋₁₅alkynylene, C₁₋₁₅heteroalkylene, C₁₋₁₅heteroalkenylene or C₁₋₁₅heteroalkynylene;
      d is 0 or 1;
      e is 0 or 1; and
      f is 0 or 1; and
   Y² is an optionally substituted steroid.
   *Formula (XI) is:*

   R^{a}-(AA)_{z}-R^{b}

   wherein
   R^{a} is a N-terminal alkylamide;
   z is an integer from 2 to 10;
   each AA is an amino acid, provided that at least one histidine is present and at least one cationic amino acid is present;
   R^{b} is -H or -NH₂.
2. The liposome of embodiment 1, wherein the liposome has a diameter in the range of 80-160nm.
3. The liposome of any preceding embodiment, wherein the RNA is a self-replicating RNA.
4. The liposome of embodiment 3, wherein the self-replicating RNA molecule encodes (i) a RNA-dependent RNA polymerase which can transcribe RNA from the self-replicating RNA molecule and (ii) the polypeptide of interest.
5. The liposome of embodiment 4, wherein the RNA molecule has two open reading frames, the first of which encodes an alphavirus replicase and the second of which encodes the polypeptide of interest.
6. The liposome of any preceding embodiment, wherein the RNA molecule is 9000-12000 nucleotides long.
7. The liposome of any preceding embodiment, wherein the polypeptide of interest is an immunogen.
8. The liposome of embodiment 7, wherein the immunogen can elicit an immune response *in vivo* against a bacterium, a virus, a fungus or a parasite.
9. The liposome of embodiment 8, wherein the immunogen can elicit an immune response *in vivo* against respiratory syncytial virus glycoprotein F.
10. A pharmaceutical composition comprising a liposome of any preceding embodiment.
11. A method for raising a protective immune response in a vertebrate, comprising the step of administering to the vertebrate an effective amount of the liposome of embodiments 1-9, or the pharmaceutical composition of embodiment 10.

## Claims

1. A liposome within which RNA encoding a polypeptide of interest is encapsulated, wherein the liposome includes at least one compound selected from the group consisting of compounds of formula (XI) and formula (I), where
Formula (XI) is:
Ra-(AA)z-Rb
wherein
Ra is a N-terminal alkylamide;
z is an integer from 2 to 10;
each AA is an amino acid, provided that at least one histidine is present and at least one cationic amino acid is present;
Rb is -H or -NH2:
Formula (I) is: 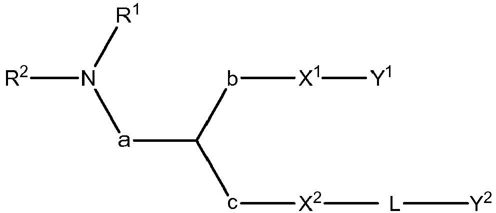 wherein:
R1 and R2 together with the nitrogen atom to which they are attached form an optionally substituted C3-20-heterocycloalkyl, C3-20-heterocycloalkenyl, C3-20-heterocycloalkynyl or C5-20-heteroaryl group;
a is absent or optionally substituted C1-4 alkylene;
b is absent or optionally substituted C1-4 alkylene;
c is absent or optionally substituted C1-4 alkylene;
X1 is O or S;
X2 is O or S;
Y1 is optionally substituted C10-30alkenyl, C10-30alkynyl, C10-30heteroalkenyl or C10-30heteroalkynyl;
L is absent or is -(La)d-(Lb)e-(Lc)f-, wherein
La is optionally substituted C1-15alkylene, C1-15alkenylene, C1-15alkynylene, C1-15heteroalkylene, C1-15heteroalkenylene or C1-15heteroalkynylene;
Lb is optionally substituted C6-14arylene or C5-13heteroarylene;
Lc is optionally substituted C1-15alkylene, C1-15alkenylene, C1-15alkynylene, C1-15heteroalkylene, C1-15heteroalkenylene or C1-15heteroalkynylene;
d is 0 or 1;
e is 0 or 1; and
f is 0 or 1; and
Y2 is an optionally substituted steroid.

2. The liposome of claim 1, wherein the liposome has a diameter in the range of 80-160nm.

3. The liposome of any preceding claim, wherein the RNA is a self-replicating RNA.

4. The liposome of claim 3, wherein the self-replicating RNA molecule encodes (i) a RNA-dependent RNA polymerase which can transcribe RNA from the self-replicating RNA molecule and (ii) the polypeptide of interest.

5. The liposome of claim 4, wherein the RNA molecule has two open reading frames, the first of which encodes an alphavirus replicase and the second of which encodes the polypeptide of interest.

6. The liposome of any preceding claim, wherein the RNA molecule is 9000-12000 nucleotides long.

7. The liposome of any preceding claim, wherein the polypeptide of interest is an immunogen.

8. The liposome of claim 7, wherein the immunogen can elicit an immune response in vivo against a bacterium, a virus, a fungus or a parasite.

9. The liposome of claim 8, wherein the immunogen can elicit an immune response in vivo against respiratory syncytial virus glycoprotein F.

10. A pharmaceutical composition comprising a liposome of any preceding claim.

11. A method for raising a protective immune response in a vertebrate, comprising the step of administering to the vertebrate an effective amount of the liposome of claims 1-9, or the pharmaceutical composition of claim 10.


SEQUENCE LISTING

<110>   NOVARTIS AG

<120>   LIPIDS SUITABLE FOR LIPOSOMAL DELIVERY OF PROTEIN-CODING RNA

<130>   VN54247D1 EP

<150>   PCT/US2011/050100
<151>   2011-08-31

<150>   USSN 61/378,833
<151>   2010-08-31

<160>   8

<170>   SeqWin2010, version 1.0

<210>   1
<211>   4
<212>   PRT
<213>   Artificial Sequence

<220>
<223>   cationic lipopeptide

<400>   1
Cys Lys His His
1

<210>   2
<211>   4
<212>   PRT
<213>   Artificial Sequence

<220>
<223>   cationic lipopeptide

<400>   2
Cys Lys Lys His
1

<210>   3
<211>   5
<212>   PRT
<213>   Artificial Sequence

<220>
<223>   cationic lipopeptide

<400>   3
Cys Lys Lys His His
1               5

<210>   4
<211>   6
<212>   PRT
<213>   Artificial Sequence

<220>
<223>   cationic lipopeptide

<400>   4

Cys Lys Lys His His His
1               5

<210>   5
<211>   7
<212>   PRT
<213>   Artificial Sequence

<220>
<223>   cationic lipopeptide

<400>   5
Cys Lys Lys His His His His
1               5

<210>   6
<211>   8
<212>   PRT
<213>   Artificial Sequence

<220>
<223>   cationic lipopeptide

<400>   6
Cys Lys Lys His His His His His
1               5

<210>   7
<211>   6
<212>   PRT
<213>   Artificial Sequence

<220>
<223>   cationic lipopeptide

<400>   7
Cys Lys Lys Lys His His
1               5

<210>   8
<211>   7
<212>   PRT
<213>   Artificial Sequence

<220>
<223>   cationic lipopeptide

<400>   8
Cys Lys Lys Lys His His His
1               5